# EUROPEAN PATENT SPECIFICATION

(11) **EP 0 973 381 B1**
(45) Date of publication and mention of the grant of the patent: **03.03.2010**
(21) Application number: 98914591.7
(22) Date of filing: 09.04.1998
(51) Int. Cl.: A01K 67/027, C07K 14/16, C12N 7/04, C12N 7/01, A61K 39/008, A61K 39/21, C12N 1/10, C12N 1/36, G06T 7/00

(54) **Animal model for evaluation of vaccines**
Tiermodell zur Berechnung von Impstoffen
Model animal aux fins d'une appréciation de vaccins

(30) Priority: 09.04.1997 US 838702; 01.05.1997 US 848760; 09.12.1997 US 69163 P
(43) Date of publication of application: 26.01.2000
(73) Proprietor: AMDL, Inc., Tustin CA 92780 (US)
(72) Inventor: Chang, Lung-Ji, Gainesville, FL 3206-6939 (US)
(74) Representative: Thomas, Simon
(86) International application number: PCT/US1998/006944
(87) International publication number: WO 1998/044788

(56) References cited:
- EP-A- 0 322 240
- WO-A-89/12823
- WO-A-91/16910
- WO-A-93/10219
- WO-A-96/11279
- WO-A-96/40238
- WO-A-97/32987
- WO-A-98/17815
- WO-A-98/39463
- AGATSUMA, T. ET AL.: "Protection of hu-PBL-SCID beige mice from HIV-1 infection by a modified oligonucleotide, RKS-1443" ANTIVIRAL RESEARCH, vol. 30, no. 1, 1996, page A35 XP002073900
- AGATSUMA, T. ET AL.: "Protection of hu-PBL-SCID Beige mice from HIV-1 infection by a 6-mer modified oligonucleotide, R-95288." ANTIVIRAL RESEARCH, vol. 34, no. 3, May 1997, pages 121-130, XP002073911
- ZHANG, C. ET AL.: "Protective immunity to HIV-1 in SCID/beige mice reconstituted with peripheral blood lymphocytes of exposed but uninfected individuals" PROCEEDINGS OF THE NATIONAL ACADEMY OF SCIENCES OF USA., vol. 93, no. 25, December 1996, pages 14720-14725, XP002073864 WASHINGTON US
- MCCUNE J M ET AL: "THE SCID-HU MOUSE: MURINE MODEL FOR THE ANALYSIS OF HUMAN HEMATOLYMPHOID DIFFERENTIATION AND FUNCTION" SCIENCE, vol. 241, 23 September 1988, pages 1632-1639, XP000600471
- IDO LUBIN ET AL: "ENGRAFTMENT AND DEVELOPMENT OF HUMAN T AND B CELLS IN MICE AFTER BONE MARROW TRANSPLANTATION" SCIENCE, vol. 252, no. 5004, 19 April 1991, pages 427-431, XP000381745
- KILCHHERR E ET AL: "REGULATION OF HUMAN IGE RESPONSE IN HU-PBL-SCID MICE" CELLULAR IMMUNOLOGY, vol. 151, 1993, pages 241-256, XP000606887
- MOSIER D E ET AL: "TRANSFER OF A FUNCTIONAL HUMAN IMMUNE SYSTEM TO MICE WITH SEVERE COMBINED IMMUNODEFICIENCY" NATURE, vol. 335, 15 September 1988, pages 256-259, XP000650830
- MOSIER D E ET AL: "CD4 T CELLS IN HIV-1 INFECTED HU-PBL-SCID MICE" INTERNATIONAL CONGRESS OF IMMUNOLOGY ABSTRACTS,23 August 1992, page 711 XP000317469
- DUCHOSAL, M.A. ET AL. : "Immunization of hu-PBL-SCID mice and the rescue of human monoclonal Fab fragments through combinatorial libraries" NATURE., vol. 355, 16 January 1992, pages 258-262, XP002086397 LONDON GB
- HUPPES W ET AL: "HUMAN TO MOUSE GVHD;A MODEL HIV STUDIES" INTERNATIONAL CONGRESS OF IMMUNOLOGY ABSTRACTS,23 August 1992, page 724 XP000283152
- SEGALL H ET AL: "GENERATION OF PRIMARY ANTIGEN-SPECIFIC HUMAN CYTOTOXIC T LYMPHOCYTES IN HUMAN/MOUSE RADIATION CHIMERA" BLOOD, vol. 88, no. 2, 15 July 1996, pages 721-730, XP000198162
- GREENWOOD J D: "XENOGENEIC PBL-SCID MICE: THEIR POTENTIAL AND CURRENT LIMITATIONS" LABORATORY ANIMAL SCIENCE, vol. 43, no. 2, April 1993, pages 151-155, XP000676036
- CHANG, L.-J. & ZHANG, C.: "Infection and replication of Tat-minus human immunodeficiency viruses: genetic analyses of LTR and tat mutants in primary and long-term human lymphoid cells" VIROLOGY, vol. 211, no. 1, August 1995, pages 157-169, XP002086398 ORLANDO US cited in the application
- MICHAEL, N.L. ET AL.: "Defective accessory genes in a Human Immunodeficiency Virus type 1-infected long term survivor lacking recoverable virus" JOURNAL OF VIROLOGY., vol. 69, no. 7, July 1995, pages 4228-4236, XP002086399 ICAN SOCIETY FOR MICROBIOLOGY US cited in the application
- ZUFFEREY R ET AL: "MULTIPLY ATTENUATED LENTIVIRAL VECTOR ACHIEVES EFFICIENT GENE DELIVERY IN VIVO" NATURE BIOTECHNOLOGY, vol. 15, September 1997, pages 871-875, XP002056816
- AMANO, M. ET AL.: "Immunofluorescence-digital image processing system for the quantitation of secreted immunoglobulin by single cells" JOURNAL OF IMMUNOLOGICAL METHODS., vol. 144, no. 1, 1991, pages 127-140, XP002086401 NEW YORK US
- AMANO, M. ET AL.: "Application of the ILISPOT-IDIP system for the enumeration of different sizes of IgA spot forming cells in the murine small and large intestine" JOURNAL OF IMMUNOLOGICAL METHODS., vol. 164, no. 1, 1993, pages 79-90', XP002086402 NEW YORK US
- CUI, Y.& CHANG, L.J.: "Computer-assisted, quantitative cytokine enzyme-linked immunospot analysis of human immune effector cell function" COMPTES RENDUS HEBDOMADAIRES DES SEANCES DE L'ACADEMIE DES SCIENCES, SERIE C: SCIENCES CHIMIQUES, vol. 22, no. 6, June 1997, pages 1146-1149, XP002086403 MONTREUIL FR
- MCCUTCHEON, M. ET AL.: "A sensitive ELISPOT assay to detect low-frequency human T lymphocytes" JOURNAL OF IMMUNOLOGICAL METHODS., vol. 210, no. 2, 29 December 1997, pages 149-166, XP002086404 NEW YORK US
- HERR, W. ET AL.: "The use of computer-assisted video image analysis for the quantification of CD8+ T lymphocytes producing tumor necrosis factor alpha spots in response to peptide antigens" JOURNAL OF IMMUNOLOGICAL METHODS., vol. 203, no. 2, 25 April 1997, pages 141-152, XP002086405 NEW YORK US
- CAYEUX S ET AL: "COEXPRESSION OF INTERLEUKIN-4 AND B7.1 IN MURINE TUMOR CELLS LOADS TO IMPROVED TUMOR REJECTION AND VACCINE EFFECT COMPARED TO SINGLE GENE TRANSFECTANTS AND A CLASSICAL ADJUVANT" HUMAN GENE THERAPY, vol. 7, no. 4, 1 March 1996, pages 525-529, XP002035091
- HODGE J W ET AL: "INDUCTION OF ANTITUMOR IMMUNITY BY RECOMBINANT VACCINIA VIRUSES EXPRESSING B7-1 OR B7-2 COSTIMULATORY MOLECULES" CANCER RESEARCH, vol. 54, 1 November 1994, pages 5552-5555, XP002035093
- BUEELER H ET AL: "INDUCTION OF ANTIGEN-SPECIFIC TUMOR IMMUNITY BY GENETIC AND CELLULAR VACCINES AGAINST MAGE: ENHANCED TUMOR PROTECTION BY COEXPRESSION OF GRANULOCYTE-MACROPHAGE COLONY-STIMULATING FACTOR AND B7-1" MOLECULAR MEDICINE, vol. 2, no. 5, September 1996, pages 545-555, XP002035094

## Description

### Cross-Reference to Related Applications

This application is a continuation-in-part of co-pending application Serial No. 08/848,760, filed May 1, 1997; which is a continuation-in-part of application Serial No. 08/838,702, filed April 9, 1997. This application also claims priority from U.S. provisional application Serial No. 60/069,163, filed December 9, 1997.

### Field of the Invention

The present invention relates to the evaluation of vaccines. In one embodiment, the present invention provides humanized animal models for the evaluation of vaccines designed to confer immunity against human pathogens, including vaccines directed against the human immunodeficiency virus. The present invention further relates to HIV vaccines. In particular, the present invention provides attenuated replication- competent HIV vaccines and replication-defective HIV vaccines. In a further embodiment, the present invention relates to immunotherapy for the treatment of tumors. In particular the present invention provides combinations of immune-modulating proteins that induce systemic immunity against tumors and provides humanized animal models for immunogene therapy.

### Background of the Invention

Conventional treatment of cancer typically involves the use of chemotherapeutic agents. The father of chemotherapy, Paul Ehrlich, imagined the perfect chemotherapeutic as a "magic bullet;" such a compound would kill invading organisms without harming the host. This target specificity is sought in all types of chemotherapeutics, including anticancer agents.

However, specificity has been the major problem with conventional anticancer agents. In the case of anticancer agents, the drug needs to distinguish between host cells that are cancerous and host cells that are not cancerous. The vast majority of anticancer drugs are indiscriminate at this level. Typically anticancer agents have negative hematological affects (*e.g*., cessation of mitosis and disintegration of formed elements in marrow and lymphoid tissues) and immunosuppressive action as well as a severe impact on epithelial tissues (*e.g*., intestinal mucosa), reproductive tissues and the nervous system [Calabresi and Chabner, In: Goodman and Gilman, The Pharmacological Basis of Therapeutics (Pergamon Press, 8th Edition) pp. 1209-1216].

Success with chemotherapeutics as anticancer agents has also been hampered by the phenomenon of multiple drug resistance, resistance to a wide range of structurally unrelated cytotoxic anticancer compounds [Gerlach et al. (1986) Cancer Surveys 5:25]. In addition, certain cancers are non-responsive to known chemotherapeutics agents and patients with these cancers invariably die within a short period following diagnosis (*e.g*., glioblastoma multiforme, recurrent metastatic melanoma, breast, lung and pancreatic cancers).

To address the drawbacks of chemotherapy for the treatment of cancer, immune system-based therapies or cancer immunotherapies have been developed. The goal of cancer immunotherapy is to harness the patient's own immune system to recognize and attack tumors. The recognition and rejection of tumor cells requires the participation of T lymphocytes (T cells).

T cells play a crucial role in a number of immune responses including the recognition of foreign antigens, destruction of virally infected cells and providing help to B cells to permit the production of antibodies that neutralize foreign antigens. In order for a T cell to recognize its target antigen, the antigen must be presented to the T cell by an antigen-presenting cell (APC) such as dendritic cells, macrophages, Langerhans cells and B cells. The APC presents the target antigen as part of a complex containing immune molecules termed major histocompatibility complex (MHC) in mice and human leukocyte antigens (HLA) in humans. Two classes of MHC molecules are known: MHC class I molecules which are expressed on all nucleated cells and MHC class II molecules which are expressed only on APCs. Class I molecules present endogenous protein fragments (not recognized as foreign) and viral antigens (recognized as foreign) while class II molecules present protein fragments derived from proteins that entered the cell by endocytosis or phagocytosis (*i.e.,* proteins which are mainly derived from infectious agents such as parasites and bacteria).

T cells recognize MHC-antigen complexes on APCs via their T cell receptor (TCR)/CD3 complex; the TCR complex together with the CD4 or CD8 coreceptors bind to MHC class II or I, respectively. Occupancy of the TCR alone is not sufficient to active the T cell to respond; activation also requires antigen-independent signals provided by the engagement of costimulatory molecules present on the surface of the T cell with their cognate ligands present on the surface of the APC. The costimulatory proteins serve to stabilize the interaction of the T cell with the APC and to transduce costimulatory signals that lead to the secretion of cytokines, proliferation of the T cell and induction of the T cell's effector function. Engagement of the TCR in the absence of costimulation results in anergy (*i.e*., nonresponsiveness) of the T cell [Schwartz (1992) Cell 71:1065; Liu and Linsley (1992) Curr. Opin. Immunol. 4:265; Allison (1994) Curr. Opin. Immunol. 6:414 and Linsley and Ledbetter (1993) Annu. Rev. Immunol. 11:191]. In addition to the requirement for costimulatory signals, T cells require growth factors (*i.e*., cytokines such as interleukin-2) in order to cause proliferation of antigen-reactive T cells.

Several cell surface proteins have been identified as potential costimulatory molecules including LFA-3, ICAM-1 and members of the CD28/CTLA-4 family. The CD28/CTLA-4 family of proteins, present on the surface of T cell, has been shown to be an important costimulator required for interleukin-2 (IL-2) driven proliferation of T cells. The ligands for the CD28/CTLA-4 proteins are members of the B7 family (*e.g*., B7-1, B7-2 and B7-3).

Cancer immunotherapy aims to induce tumor-specific T cell responses that are effective in the rejection of tumors. The notion that the immune system is naturally involved in identifying and suppressing tumors is supported by the fact that immunocompromised patients have an increased incidence of tumors [Frei et al. (1993) Transplant. Proc. 25:1394]. However, given the incidence of cancer, even in seemingly immunologically normal individuals, it is clear that the immune system fails to recognize and destroy all tumor cells. Indeed animal studies have shown that the majority of tumors fail to provoke an immune response even when these tumors express potentially recognizable tumor-specific antigens [Boon et al. (1994) Annu. Rev. Immunol. 12:337 and Houghton (1994) J. Exp. Med. 180:1]. Several reasons for the lack of immunogenicity of tumor cells have been proposed including failure to express MHC class I molecules, downregulation of transporters for antigen processing and the lack of costimulatory molecules on tumor cells [Garrido et al. (1993) Immunol. Today 14:491; Restifo et al. 91993) J. Exp. Med. 177:265; Cromme et al. (1994) J. Exp. Med. 179:335; Chong et al. (1996) Human Gene Ther. 7:1771].

In an attempt to increase the immunogenicity of tumor cells, genes encoding costimulatory proteins and/or cytokines have been introduced into tumor cells. The modified tumor cells are then examined for their ability to induce an anti-tumor response (*in vivo* or in *vitro*). As discussed below, these experiments have been carried out in animal models (*e.g*., in mice and rats) and the results have been conflicting leading some in the field to question the utility of existing animal models for the development of immunogene therapy protocols aimed at the treatment of humans.

The majority of the existing animal models for immunogene therapy involve the use of immunocompetent syngeneic animals (*e.g*., mice and rats) and established tumor cell lines. Typically, one immunogene (*e.g*., mouse IL-2) is introduced into an established mouse tumor cell line, the tumor cells are selected in culture to identify cells expressing the transferred immunogene and the modified tumor cells are injected into recipient mice and the mice are examined for the presence of tumors. In some experiments, the mice receive irradiated modified tumor cells as a vaccine followed by a challenge with unmodified tumor cells (vaccination/challenge experiment). In some cases, the expression of certain immunogenes has been shown to increase the immunogenicity of the tumor cell (*i.e.,* to reduce the tumorigenicity of the tumor cell). An experimentally induced animal tumor is said to be immunogenic if the tumor is rejected following transplantation into syngeneic animals previously immunized or vaccinated with irradiated cells of the same tumor. Nonimmunogenic tumors are not rejected under these conditions.

Overall the results of these syngeneic animal experiments have been conflicting. For example, the expression of the costimulatory molecule B7-1 in the mouse melanoma cell line B-16 was found to lead to the rejection of B7-1 expressing B-16 cells in syngeneic mice in one study [Wu et al. (1995) J. Exp. Med. 182:1415] while in another study B7-1-expressing B-16 cells were found to be tumorigenic [Chen et al. (1994) J. Exp. Med. 179:523]. In the case where B7-1 expression was found to increase the immunogenicity of B-16 cells, the authors reported that animals that rejected the modified B-16 cells did not develop an enhanced systemic immunity against unmodified or wild type B-16 cells [Wu *et al., supra*]. In another study, the expression of B7-1 or B7-2 in mouse colorectal tumor or melanoma cells was found to confer a local anti-tumor response in immunocompetent mice [Chong et al. (1996) Human Gene Ther. 7:1771]. However, no systemic immunity was conferred by vaccination of mice with B7-1 or B7-2 expressing colorectal tumor cells and the expression of B7-1 or B7-2 in the melanoma cells was found to reduce the systemic immunity conferred by the B7-expressing cells relative to that conferred by vaccination with wild type melanoma cells even when the B7-expressing cells also expressed interferon-γ (Chong *et al., supra*).

It has been reported that the rejection of B7-1 expressing tumor cell lines is limited to highly immunogenic cell lines as B7-1 expression in poorly immunogenic fibrosarcomas (*e.g*., MAC101, MCA102 and Ag104) and the B-16 melanoma cell line does not reduce the tumorigenicity of these lines. As discussed above, expression of B7-1 in the B-16 melanoma cell line was found by one group to reduce the tumorigenicity of these cells (Wu *et al., supra).* This discrepancy may be explained by differences in the level of B7-1 expression achieved by different groups. However, tumor cells expressing only B7-1 have been found to be ineffective in inducing the rejection of established tumors.

The failure of B7-1 alone to induce the rejection of established tumors was postulated to be due to the induction of a state of anergy in potentially reactive T cells. Therefore, combinations of B7-1 and cytokines were tested to see if this state of anergy could be overcome. Combinations of costimulatory molecules and various growth factors or cytokines has proven to be more effective than the use of either category of molecules alone. For example, the expression of B7-1 in the mouse NC adenocarcinoma cell line was found to have no effect on the tumorigenicity of these cells in immunocompetent syngeneic mice [Gäken et al. (1997) Human Gene Ther. 8:477]. However, the expression of both B7-1 and IL-2 in the NC adenocarcinoma cell line substantially reduced the tumorigenicity of these cells in mice.

While existing animal models have demonstrated that in general a combination of costimulatory molecules and a cytokine and/or a chemokine is preferable to the use of any one of these groups alone, the data from different groups using combinations of these molecules is conflicting. For example, Dilloo *et al.* reported that mice immunized with A20 B cell lymphoma cells mixed with IL-2 and the chemokine lymphotactin developed a potent anti-tumor response while mice immunized with B cell lymphoma cells mixed with GM-CSF developed a much reduced anti-tumor response [Dilloo et al. (1996) Nature Med. 2:1090]. On the other hand Levitsky *et al.,* reported that vaccination of mice with GM-CSF-expressing A20 B lymphoma cells lead to a complete rejection of pre-established A20 tumors while vaccination with either II-2-expressing or B7-1-expressing A20 cells did not [Levitsky et al. (1996) J. Immunol. 156:3858]. This apparent conflict lead Dilloo and Brenner to remark that "it [is] difficult to be confident that current murine models can be used to pick the 'best' cytokine for a particular human tumor" [Nature Med. (1997) 3:126]. Clearly the art needs improved models for determining which cytokines, costimulatory molecules and/or chemotactins, or which combination thereof is best suited for the treatment of particular human tumors.

In order to provide effective cancer immunotherapy, the art needs means to increase the immunogenicity of human tumors as well as animal models predictive of human anti-tumor immune responses.

The development of effective vaccines for the prevention of human immunodeficiency virus (HIV) infection has also been hampered by the lack of suitable animal models. Given the complex interaction of HIV with the immune system, there is no substitute for *in vivo* models for HIV infection and HIV vaccine evaluation. Existing animal models employed for HIV infection and vaccine development include chimpanzees infected with HIV-1, macaques infected with either SIV or HIV-2, cats infected with feline immunodeficiency virus and scid/scid mice reconstituted with human cells and infected with HIV. None of these models are ideal for the evaluation of HIV vaccines. The replication of HIV in chimpanzees is more restricted than in humans; therefore, vaccines shown to be protective in chimpanzees may fail to protect humans as chimpanzees may be more easy to protect from HIV infection. In addition, the number of chimpanzees available for research is small and therefore, it is difficult to obtain statistically significant experimental results, these animals are costly to maintain and once exposed to HIV these animals must be confined for the rest of their lives at a cost of greater than $100,000.00/chimp [Stott and Almond (1995) Nat. Med. 1:295]. While the supply of macaques is greater than that of chimpanzees, these animals are also costly to maintain. Most importantly, macaques cannot be infected with HIV-1 and thus, infection with HIV-2 or SIV must be used as a surrogate model for HIV-1 infection. Scid/scid mice reconstituted with human lymphocytes (hu-PBL-SCID mice), while permitting infection of the human cells with HIV-1, have failed to provide a model in which HIV vaccines and HIV immunity can be evaluated. This failure is due to the fact that most of the human T lymphocytes in the reconstituted scid/scid mice exhibit activated cell phenotypes soon after reconstitution, and almost all (>99%) human T cells exhibit mature memory phenotypes in a state of reversible anergy [Rizza et al. (1996) J. Virol. 70:7958; Tarry-Lehmann and Saxon (1992) J. Exp. Med. 175:503; Tany-Lehmann et al. (1995) Immunol. Today 16:529]. Therefore, the lack of sufficient numbers of immature naive T cells after reconstitution renders the hu-PBL-SCID model unsuitable for the evaluation of anti-HIV immunity following administration of a HIV vaccine.

The ultimate system for the study of HIV vaccines is, of course, the human. However, given the fact that HIV infection is almost invariably fatal in humans, ethical considerations limit the use of human volunteers in HIV vaccine evaluation. The art needs animal models in which anti-HIV immunity can be assessed to permit the evaluation of HIV vaccines.

### Brief Summary of the Invention

The present invention provides novel humanized animal models that permit the identification of immune-modulating genes and combinations thereof useful for the treatment of human tumors. In addition, the present invention provides vaccines comprising cells modified to express at least one immune-modulating gene. The present invention also provides methods of treating subjects having a tumor with a tumor cell vaccine modified to express one or more immune-modulating genes. The novel animals of the present invention provide a means of evaluating vaccines, including cancer vaccines and vaccines directed against human pathogens (*e.g*., HIV, malaria, *Leishmania, etc*.). In addition, the subject invention provides HIV vaccines including live attenuated HIV vaccines and HIV DNA vaccines.

The present invention also concerns an immunodeficient mouse comprising a sufficient number of human immune cells capable of producing a sustained immune response. In one embodiment, the present invention provides an immunodeficient mouse comprising human T lymphocytes expressing the CD45 antigen wherein at least about 10% of the human T lymphocytes expressing the CD45 antigen represent immature naive T lymphocytes. In another embodiment, the present invention provides an immunodeficient mouse comprising human T lymphocytes expressing the CD45 antigen wherein at least about 5% of these T lymphocytes represent immature naive T lymphocytes. Alternatively, at least about 1% of the human T lymphocytes expressing the CD45 antigen represent immature naive T lymphocytes. The invention is not limited by the nature of the immunodeficient mouse strain employed. In a preferred embodiment, the immunodeficient mouse is a SCID/beige mouse.

In another specific embodiment, the immunodeficient mouse comprising human T lymphocytes further comprises human tumor cells. The invention is not limited by the nature of the human tumor cells employed. The human tumor cells may be established tumor cells, primary tumors cells or tumor cells (established or primary) modified to express one or more immune-modulating genes, genes encoding cell cycle regulators and genes encoding inducers of apoptosis.

In another embodiment, the present invention provides a SCID/beige mouse comprising human cells, such as, for example, immune cells. The invention is not limited by the nature of the human immune cells, these cells may be, for example, human PBLs, splenocytes, cells isolated from lymph nodes and/or peritoneal lavage. In a preferred embodiment, the SCID/beige mouse comprising human immune cells further comprises human tumor cells. In a preferred embodiment, the tumor cells are derived from central nervous system cells, most preferably glioblastoma cells. In another preferred embodiment, the tumor cells are malignant melanoma cells.

The present invention further provides a method comprising: (a) providing: (i) a SCID/beige mouse; (ii) human tumor cells; and (iii) human peripheral blood lymphocytes; (b) introducing a first dose of the tumor cells into said mouse; (c) reconstituting the mouse containing said tumor cells with the lymphocytes; and (d) monitoring the reconstituted mouse for growth of the tumor cells.

In a preferred embodiment, the method of the subject invention further comprises identifying at least one immune modulating gene, or a gene encoding a cell cycle regulator, or a gene that encodes an inducer of apoptosis, whose expression in a tumor cell prevents the growth of the introduced tumor cells in a reconstituted mouse. In another preferred embodiment, the method comprises, following the reconstitution, the additional step of vaccinating the reconstituted mouse with a second dose of tumor cells. In a preferred embodiment, the first dose of tumor cells comprises unmodified tumor cells and the second dose of tumor cells comprises irradiated tumor cells. In a particularly preferred embodiment, the irradiated tumor cells express at least one immune-modulating gene, or a gene encoding a cell cycle regulator, or a gene that encodes an inducer of apoptosis.

In one embodiment of the methods of the present invention, the tumor cells and the lymphocytes come from the same donor. In another embodiment, the tumor cells and the lymphocytes come from different donors.

The present invention further provides a method comprising: (a) providing: (i) a SCID/beige mouse; (ii) irradiated and unirradiated human tumor cells; and (iii) human peripheral blood lymphocytes; (b) reconstituting said mouse with the lymphocytes; (c) vaccinating the mouse with the irradiated tumor cells; (d) introducing the unirradiated tumor cells into the vaccinated mouse; and (e) monitoring the vaccinated mouse for growth of the unirradiated tumor cells.

In a preferred embodiment, the method further comprises identifying at least one immune modulating gene, or a gene encoding a cell cycle regulator, or a gene that encodes an inducer of apoptosis, whose expression prevents the growth of said unirradiated tumor cells in said vaccinated mouse.

The present invention also provides vaccines comprising a cell expressing an antigen and an immune-modulating protein. The cell can be, for example, a tumor cell or microorganism such as *Leishmania, Mycobacterium, Listeria* and *Plasmodium.* Preferably, the immune-modulating protein is a cell cycle regulator, inducer of apoptosis, a viral factor, a tumor suppressor, etc. Viral factors include viral proteins and viral growth regulators. The vaccines of the present invention are not limited by the nature of the immune modulating protein employed. In a specific embodiment, the additional immune modulator is a cytokine. In a preferred embodiment, the cytokine is selected from the group consisting of interleukin 2, interleukin 4, interleukin 6, interleukin 7, interleukin 12, granulocyte-macrophage colony stimulating factor (GM-CSF), granulocyte colony stimulating factor, interferon-gamma, tumor necrosis factor-alpha. In an exemplified embodiment, a tumor cell expressing a gene encoding an immune-modulating protein B7-2 and a gene encoding the cytokine GM-CSF is provided.

The present invention further provides a method of treating a tumor comprising: (a) providing: (i) a subject having a tumor of the central nervous system; (ii) an expression vector encoding an immune-modulating protein, such as the human B7-2 protein, and at least one additional immune modulator or a cell cycle regulator or inducer of apoptosis; (b) transferring the expression vector into the tumor under conditions such that the immune-modulating protein and the immune-modulator (and/or a cell cycle regulator or inducer of apoptosis) are expressed by at least a portion of the tumor. In a preferred embodiment, the method further comprises, prior to transfer of the expression vector, the step of removing at least a portion of the tumor from the subject and following the transfer of said expression vector, irradiating the tumor cells expressing the immune-modulating protein and the immune-modulator (and/or a cell cycle regulator or inducer of apoptosis) and introducing the irradiated tumor cells back into the subject to create an immunized subject. In another embodiment, the method further comprises introducing at least one additional dose of irradiated tumor cells expressing the immune-modulating protein and an additional immune-modulator (and/or a cell cycle regulator or inducer of apoptosis) into the immunized subject.

The invention further provides a method comprising: (a) providing: (i) an immunodeficient mouse, such as a SCID/beige or SCID/nod mouse, comprising human cells; (ii) an injectable preparation comprising at least one component derived from a human pathogen; and (iii) a composition comprising an infectious human pathogen; (b) injecting the mouse with the injectable preparation to produce an injected mouse; (c) exposing the injected mouse to the composition; and (d) monitoring the exposed mouse for the presence of infection of the human cells by the infectious human pathogen. Preferably, the human cells are immune cells. In a preferred embodiment, the injecting of step (b) is repeated at least once prior to exposing the mouse to the composition. The injectable preparation comprises a candidate vaccine; a candidate vaccine may comprise proteins or peptides or nucleic acids and may be immunogenic. However, as this method offers a means to evaluate candidate vaccines, some candidate vaccines are expected to be immunogenic (*i.e*., capable of invoking an immune response directed against the pathogen from which the protein, peptide or nucleic acid was derived) and some are expected to not be immunogenic (and would therefore be rejected as a vaccine for use in a subject such as a human). In a preferred embodiment, the injectable preparation is immunogenic. The injectable preparation may comprise pharmacological carriers and excipients such as aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions. Further, the injectable preparation may comprise as adjuvant (*e.g*., alum, complete or incomplete Freund's, SAF-1).

The invention is not limited by the nature of the human pathogen. In a preferred embodiment, the human pathogen is selected from the group comprising human immunodeficiency virus, *Leishmania, Mycobacterium* (*e.g., M. tuberculosis*), and *Plasmodium* (the causative agent of malaria).

The invention is not limited by the nature of the component of the human pathogen present in the injectable composition. In a preferred embodiment, the component of the human pathogen comprises at least a portion of a protein derived from the pathogen; the component may be a complete protein molecule or a peptide derived from the complete protein (and may be produced by peptide synthesis, recombinant DNA methodologies or protease digestion of the intact protein). In another preferred embodiment, the component of the human pathogen comprises DNA. In a particularly preferred embodiment, the DNA comprises proviral DNA encoding a human immunodeficiency virus genome capable of expressing viral structural genes but incapable of being packaged into viral particles. In a preferred embodiment, the DNA comprises plasmid DNA selected from the group consisting of pHP-1, pHP-2 and pHP-3.

In another embodiment, the injectable preparation comprises an attenuated replication-competent human immunodeficiency virus (*i.e*., capable of producing only a limited infection and preferably incapable of producing the pathological changes associated with infection by a non-attenuated HIV). The invention is not limited by the nature of the change which renders the virus attenuated. In a preferred embodiment, the attenuated virus comprises a mutated *tat* gene; preferably, the genome of the attenuated virus cannot express a functional Tat protein. In another preferred embodiment, the genome of the attenuated virus genome further comprises a mutated *nef* gene; preferably, the genome of the attenuated virus cannot express either a functional Tat protein or Nef protein. In a preferred embodiment, the attenuated virus is selected from the group consisting of HIV_{CMV/tat-B}, HIV_{CMV/AD8/nef-B/tat}-_{B}, HIV_{NLA43/AD8/tat-B/nef-B} and HIV_{NL43/nef-B/tat-B.}

In another embodiment, the injectable preparation comprises attenuated *Leishmania* cells. The invention is not limited by the nature of the change which renders the *Leishmania* cell attenuated. In a preferred embodiment, the attenuated *Leishmania* cell comprises heterologous DNA encoding a cysteine protease gene. The cysteine protease gene may be a leishmanial cysteine protease gene or a cysteine protease gene from another organism. When a leishmanial cysteine protease gene is employed it is preferably placed in operable combination with promoters and, optionally, enhancers functional in a *Leishmania* cell. As the leishmanial cysteine protease gene is not present in its native configuration (*i.e*., chromosomal configuration), it comprises heterologous DNA. Most preferably, the leishmanial cysteine protease gene is present on an expression vector such as pALT-Neo or pX. As shown herein, the overexpression of cysteine protease in *Leishmania* cells attenuates the *Leishmania* cell and provides an effective vaccine for the prevention of leishmaniasis. In another preferred embodiment, the heterologous DNA further comprises DNA encoding a human immunodeficiency virus gene selected from the group consisting of the *env* gene, the *gag* gene, the *pol* gene, the *tat* gene, the *rev* gene, the *vif* gene, the *vpu* gene, the *vpr* gene and the *nef* gene*. Leishmania* cells infect macrophages and, therefore, *Leishmania* cells expressing HIV proteins provide a means to present HIV antigens to a host in the context of an APC (*i.e*., provides an HIV vaccine).

The invention is not limited by the nature of the monitoring employed to detect the presence (or absence) of infection of the human immune cells in the SCID/bg mouse comprising human immune cells (*e.g.,* a hu-PBL-SCID/bg mouse). Serum collected from the mouse following vaccination and challenge with the pathogen may be examined for the presence of human immunoglobulin directed against the pathogen (evidence of humoral immunity). Cell mediated immunity directed against the pathogen may be examined by a variety of means known to the art, including but not limited to, *in vitro* ELISPOT analysis of γ-IFN production by lymphocytes (*e.g*., splenocytes) isolated from the vaccinated and challenged mice (using pathogen-infected PBLs as stimulators). Further, the mouse may be examined for the presence of infection in the sites expected to be infected by the pathogen employed for the challenge (*e.g*., immunostaining of lymphocytes collected from mice challenged with HIV to detect the presence of HIV proteins in and/or on lymphocytes and/or macrophages).

The present invention also provides a method comprising: (a) providing: (i) an immunodeficient mouse, such as a SCID/beige mouse, comprising human immune cells; (ii) an injectable preparation comprising one or more components derived from a human immunodeficiency virus (HIV); and (iii) a composition comprising non-attenuated human immunodeficiency virus; (b) injecting the mouse with the injectable preparation to produce an injected mouse; (c) exposing the injected mouse to the composition; and (d) monitoring the exposed mouse for the presence of infection of the human immune cells by the non-attenuated human immunodeficiency virus. The invention is not limited by the non-attenuated virus employed; any virus capable of causing a non-attenuated infection may be employed. Preferably, the non-attenuated virus is a virus isolated from a patient with AIDS (*i.e*., a clinical isolate) or is a virulent laboratory strain. Particularly preferred non-attenuated HIV strains include the NL4-3 strain, the ADA strain and HIV-1 primary isolates covering the different HIV clades (*e.g*., 92RW008, 92BR003, 92HT593, etc. available from the NIH AIDS Research and Reference Reagent Program of National Institutes of Health, Bethesda, MD).

In a preferred embodiment, the injecting of step (b) is repeated at least once prior to exposing the mouse to the composition. The injectable preparation comprises a candidate vaccine; a candidate vaccine may comprise proteins or peptides or nucleic acids and may be immunogenic. However, as this method offers a means to evaluate candidate vaccines, some candidate vaccines are expected to be immunogenic (*i.e*., capable of invoking an immune response directed against the pathogen from which the protein, peptide or nucleic acid was derived) and some are expected to not be immunogenic (and would therefore be rejected as a vaccine for use in subject such as a human). In a preferred embodiment, the injectable preparation is immunogenic. The injectable preparation may comprise pharmacological carriers, excipients and adjuvants as discussed above.

The invention is not limited by the nature of the component of the HIV present in the injectable composition. In a preferred embodiment, the component of the HIV comprises at least a portion of a protein derived from a HIV; the component may be a complete protein molecule or a peptide derived from the complete protein (and may be produced by peptide synthesis, recombinant DNA methodologies or protease digestion of the intact protein). In another preferred embodiment, the component of the HIV comprises DNA derived from HIV. In a particularly preferred embodiment, the DNA comprises proviral DNA encoding a HIV genome capable of expressing viral structural genes but incapable of being packaged into viral particles. In a preferred embodiment, the DNA comprises plasmid DNA selected from the group consisting of pHP-1, pHP-2 and pHP-3.

The invention also provides an attenuated human immunodeficiency virus wherein the genome of the virus comprises a mutated tat gene and a mutated *nef* gene. The invention is not limited by the nature of the mutation in the *tat* and *nef* genes*;* insertions, deletions, substitutions may be employed to mutate the tat and *nef* genes. Preferably, the genome of the attenuated virus comprises a mutated tat gene and a mutated *nef* gene that cannot express a functional Tat protein or a functional Nef protein. In a preferred embodiment, the attenuated virus is selected from the group consisting of HIV_{CMV/tat-B}, HIV_{CMV/AD8/nef-B/tat-B}, HIV_{NL43/AD8/tat-B/nef-B} and HIV_{NL43/nef-B/tat-B}.

The invention further provides a DNA construct comprising the provirus of a replication-defective human immunodeficiency virus, wherein the DNA construct is selected from the group consisting of pHP-1, pHP-2 and pHP-3.

The invention also provides a method comprising: (a) providing: (i) a SCID/beige mouse; (ii) human fetal hematopoietic tissue comprising human immune cells (*e.g*., fetal liver, bone marrow, thymus, lymph node); (iii) an injectable preparation comprising one or more components derived from a human immunodeficiency virus vaccine; (iv) a composition comprising non-attenuated human immunodeficiency virus; (b) inserting the hematopoietic tissue under the kidney capsule of the mouse to provide a transplanted mouse; (c) injecting the transplanted mouse with the vaccine to produce an injected mouse; (d) exposing the injected mouse to the composition; and (e) monitoring the exposed mouse for the presence of infection of the human immune cells by the non-attenuated human immunodeficiency virus. The invention is not limited by the non-attenuated virus employed; any virus capable of causing an non-attenuated infection may be employed. Preferably, the non-attenuated virus is a virus isolated from a patient with AIDS (*i.e*., a clinical isolate) or is a virulent laboratory strain. Particularly preferred non-attenuated HIV strains include the NL4-3 strain, the ADA strain and HIV-1 primary isolates covering the different HIV clades (*e.g*., 92RW008, 92BR003, 92HT593, etc. available from the NIH AIDS Research and Reference Reagent Program of National Institutes of Health, Bethesda, MD).

In a preferred embodiment, the injecting of step b) is repeated at least once prior to exposing the mouse to the composition. The injectable preparation comprises a candidate vaccine; a candidate vaccine may comprise proteins or peptides or nucleic acids and may be immunogenic.

The subject invention also concerns methods for enumeration and analysis of results obtained from an immunospot assay, such as an enzyme-linked immunospot (ELISPOT) assay. The methods comprise performing an immunospot assay wherein a detectable signal produced by the assay is converted to a digital image using computer-assisted video imaging means. In an exemplified embodiment, the detectable signal is produced by means of an enzyme substrate reaction that produces a detectable colorimetric signal. Once the detectable signal has been processed into a digital image, the image can be analyzed using analysis software, such as, for example, NIH Image Version 1.55 shareware. The methods of the invention can be used with one- or two-color immunospot assays. Using the methods of the present invention eliminates manual microscopic counting of immunospot assay results and provides objective and reproducible immunospot assay analyses.

### Brief Description of the Drawings

**Figure 1** provides a schematic showing the pLSN, pLSNB70, pLSNGM1, pLSN-BG9 and pLSN-GFP retroviral constructs.

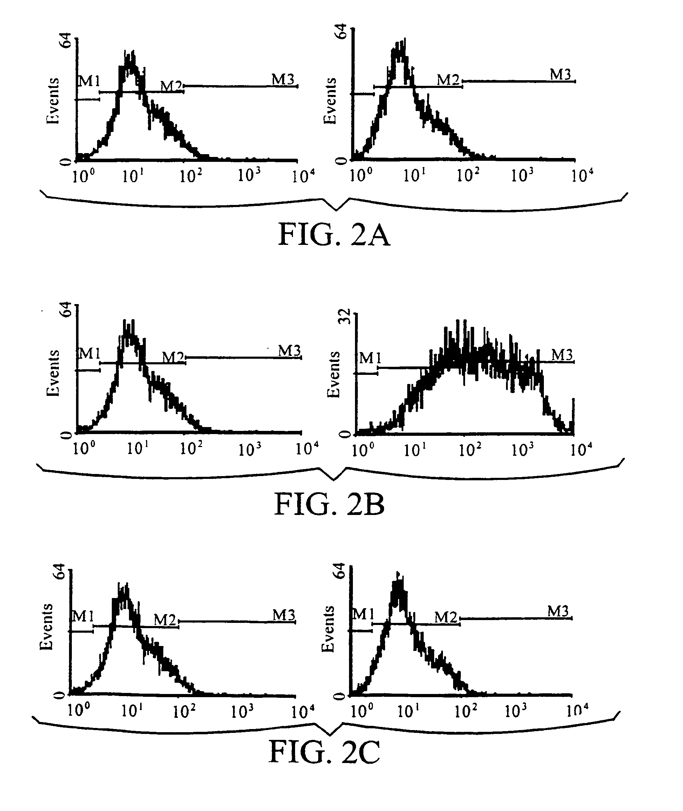

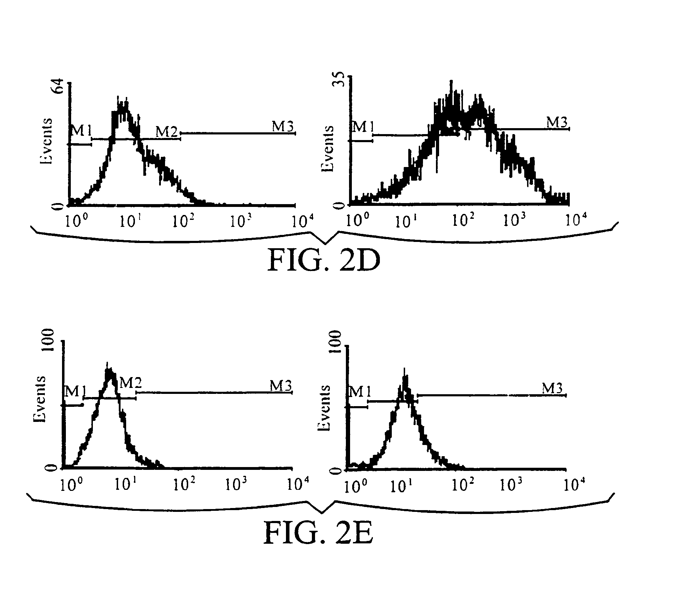

**Figures 2A-E** provide flow cytometry histograms for wild type D54MG cells (2A), B7-2-transduced D54MG cells (2B), GM-CSF-transduced D54MG cells (2C), B7-2 and GM-CSF-transduced D54MG cells (2D), and GFP-transduced D54MG cells (2E). For Figs. 2A-2D, the histograms on the left represent D54MG cells stained with isotype matched control antibodies while the histograms on the right represent staining with monoclonal anti-human B7-2 antibodies. For Fig. 2E, the **histogram** on the left represents unstained wild type D54MG cells while the histogram on the right represents unstained GFP-transduced D54MG.
**Figures 3A** **and** **3B** show the inhibition of growth of B7-2-transduced D54MG cells compared to unmodified D54MG cells in Hu-PBL-SCID/bg mice. In Fig. 3A, all mice were reconstituted with PBLs while in Fig. 3B half the mice from both groups were left unreconstituted.
**Figures 4A** **and** **4B** show the inhibition of growth of GM-CSF-transduced D54MG cells compared to unmodified D54MG cells in Hu-PBL-SCID/bg mice. Figures 4A and 4B represent data from two separate experiments.
**Figures 5A** **and** **5B** show the inhibition of unmodified D54MG cell challenges in Hu-PBL-SCID/nod mice (5A) and Hu-PBL-SCID/bg mice (5B) vaccinated with irradiated D54MG-B7-2/GM-CSF cells.
**Figures 6A and 6B** show the percentage of CD45+ lymphocytes in reconstituted SCID/bg and SCID/nod mice, respectively.
**Figures 7A and 7B** show RT activity in infected PBLs from LR and HR seronegative individuals using a macrophage tropic HIV at an moi of 0.2 (7A) or at an moi of 0.001-0.0001 (7B), respectively.
**Figures 8A-8E** demonstrate the *in vivo* infection of hu-PBL-SCID/bg mice. Figures 8A-8D show immunostaining for HIV-1 infected cells in peritoneal lavage (8A and 8C) and splenocytes (8B and 8D) of mice reconstituted with PBLs from a LR (8C and 8D) or HR individual (8A and 8B). Figure 8E shows the percentage of HIV-1 infected cells in the peripheral blood, spleen and peritoneal lavage of hu-PBL-SCID/bg mice reconstituted with PBLs from a LR or HR individual and challenged with T cell or macrophagic tropic HIV-1.
**Figure 9** shows an ELISPOT analysis of HIV-1-specific IFN-γ production in PBLs of HR and LR uninfected individuals.
**Figures 10A-10E** demonstrate the HIV-1 infection of human PBLs in hu-PBL-SCID/bg mice treated or untreated with anti-CD8 antibodies. Figs. 10A-10D show FACS analysis of splenocytes derived from the reconstituted mice (10A and 10C no anti-CD8 treatment; 10B and 10D after anti-CD8 treatment). Figure 10E shows the percentage of HIV-1 infected cells in the spleen and peritoneal lavage of hu-PBL-SCID/bg mice reconstituted with PBLs from a LR or HR individual, with or without anti-CD8 treatment, and infected with HIV_{NLAD8}.
**Figures 11A-11F** provide schematics showing the organization of the HIV-1 genome (11 A) and the organization of the HIV_{NL4-3} virus, (11B) HIV_{NL43/AD8/tat-B/nef-B} (11C), HIV_{NL43/nef-B/tat-B} (11D), HIV_{CMV/nef-B/tat-B} (11E) and HIV_{CMV/AD8/nef-B/tat-B} (11F) clones.
**Figures 12A-B** provide schematics showing a portion of the wild type HIV-1 sequence as well as the tat-B (Fig. 12A; wild-type sequence provided in SEQ ID NO:26) and nef-B mutations (Fig. 12B; wild-type sequence provided in SEQ ID NOS:27 and 28).

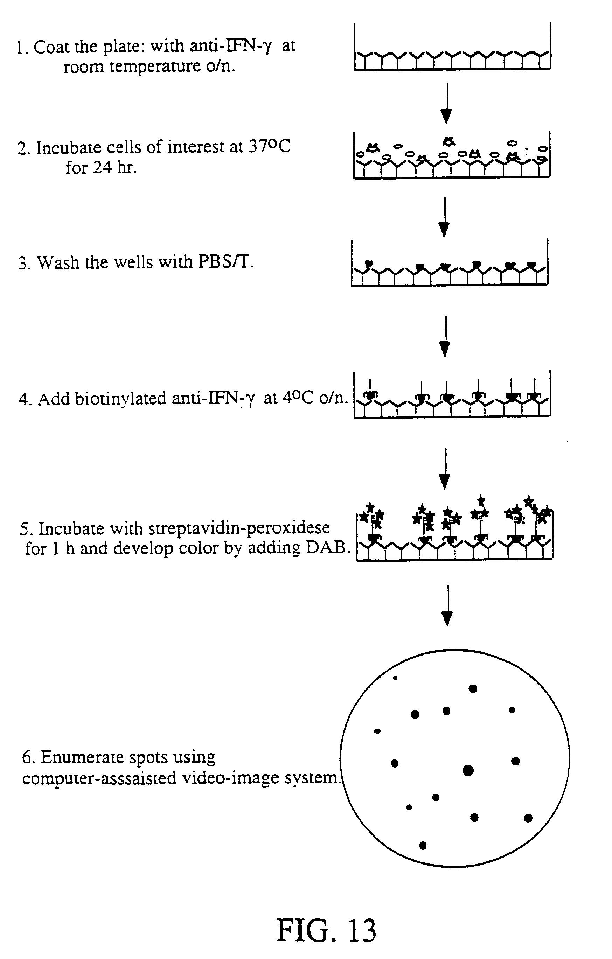

**Figure 13** shows a schematic flow chart of an ELISPOT assay. (1) 96-well nitrocellulose-bottomed plates (MULTISCREEN-HA, Millipore, Mississauga, Ontario) are coated with 70 µl/well mouse anti-human IFN-γ monoclonal Ab (10 mg/ml, Pharmingen, San Diego, California) and incubated at room temperature overnight. (2) The wells are washed 2 times with PBS and incubated at 37°C for 1 h with 100 µl/well of RPMI 1640 containing 10% FCS. The cells of interest are then added to each well to a total volume of 200 µl as described in Materials and Methods. (3) Each well is washed with PBS/T (0.05% TWEEN 20) 4 times and blocked by 100 µl of 20% FCS in PBS/T for 15 min. (4) 75 µl of biotinylated anti-human IFN-γ mAb (2.5 mg/ml, Pharmingen) is then added to each well and incubated overnight at 4°C. (5) The wells are washed four times using PBS/T and incubated with 100 µl of peroxidase-labeled streptavidin (1:800 dilution, CALTAG, South San Francisco, California) in PBS for 1 h. After being washed twice with PBS/T and twice with PBS, 100 µl DAB (Sigma Chemical) solution containing 0.3% NiCl₂ is then added, incubated for 15-20 min, and rinsed four times using ddH₂O. (6) After being air-dried overnight, the number of spots in each well is counted by computerized video-imaging as described in Materials and Methods
**Figure 14** shows computer-assisted image capturing and enumeration of ELISPOT assay. Figure 14A shows a retrieved image of ELISPOT assay using "NIH image 1.55." The well on the left is a control which contains only AA-2. The wells in the middle and on the right received 1,000 and 200 PBLs together with AA-2, respectively. Figure 14B shows a converted binary image by setting up a proper threshold. Figure 14C shows computer enumeration of ELISPOTs using the option "Particle Analysis."
**Figure 15** shows ELISPOT analyses of lymphocyte reactions to non-specific allo-antigens or to HIV-1-specific antigens. Figure 15A shows allogeneic responses to AA-2 of PBLs from a healthy donor (hatched bar) and a cancer patient under chemotherapy (solid bar). Figure 15B shows HIV-1-specific lymphocyte response in an HIV-1 unexposed (solid bar) or a multiply-exposed, seronegative donor (hatched bar).
**Figure 16** shows ELISPOT analyses of the contribution of different lymphocyte subsets to IFN-γ production upon non-specific, allogeneic or HIV-1-specific antigen stimulation. Figure 16A shows CD4-, CD8-, and/or CD56-depleted, allogeneic PBL responses to AA-2; Figure 16B shows HIV-1-specific IFN-γ ELISPOT analysis of autologous PBL responses of an HIV-1-exposed, seronegative individual.

### Definitions

To facilitate understanding of the invention, a number of terms are defined below.

As used herein, "immunodeficient mouse" refers to a mouse or mouse strain which is deficient in immune system function, including at least a deficiency in function or presence of mature B and T lymphocytes. Examples of immunodeficient mouse strains include, but are not limited to the C.B-17-SCID-nod, C.B-17scid/scid and C.B-17-SCID-beige strains. Particularly preferred immunodeficient mice have a severe combined immunodeficiency characterized by a lack of mature T, B and natural killer (NK) lymphocytes (*e.g*., the C.B-17-SCID-beige mouse strain).

As used herein, "human T lymphocytes" refers to T lymphocytes of human origin. When present in a mouse reconstituted with human blood cells, the human T lymphocytes may be obtained from a variety of sources in the reconstituted mouse including blood (*i.e*., peripheral blood lymphocytes or PBLs), lymph nodes, spleen, peritoneal lavage, etc.

Human T lymphocytes are identified by the presence of certain markers or cell surface proteins including CD3, CD4, CD8, T cell antigen receptor (TCR) and CD45. The presence of these markers on a lymphocyte may be determined by standard immunocytological means such as incubation (or staining) of a cell suspension containing lymphocytes with antibodies specific for these markers; the antibodies may be directly labeled (*e.g*. with a fluorophore such as fluorescein, phycoerythrin, Texas Red, etc.) or the presence of the antibody bound to the surface of a lymphocyte may be detected using a secondary antibody (*i.e*., an antibody directed at the first antibody or a component thereof) that is labeled. The stained lymphocytes may then be analyzed using a fluorescence microscope or a FACS (fluorescence-activated cell sorter) analysis. "Mature human T lymphocytes" express either CD4 or CD8, CD3 and a TCR. "Immature human T lymphocytes" express both CD4 and CD8 (*i.e*., they are CD4+8+); these cells are also referred to as progenitor T cells. "Immature naive human T lymphocytes" are immature T lymphocytes that have not been activated (*i.e*., they have not engaged antigen specific for their TCR or been stimulated by a nonspecific mitogen) and are said to be naive. "Immature naive T lymphocytes" includes CD4+8+ T cells as well as CD45RA+ T cells.

A mouse "comprising CD45+ T lymphocytes wherein at least about 5% of the human CD45+ T cells represent immature naive T lymphocytes" is a mouse in which about 5% or more of the CD45+ T cells are either CD4+8+ or CD45RA+ or the sum of the percentage of CD45+ T cells that are CD4+8+ and CD45RA+ is at least about 5%.

CD45 proteins are found on the surface of all hematopoietic cells, except for erythrocytes [The Leukocyte Antigen Facts Book, Barclay et al. (1993), Academic Press, London, UK, pp.202-204]. Different isoforms of CD45 are found on different lymphoid cell types; CD45RO is found on activated and memory T cells, whereas CD45RA is found on naive T cells.

The term "SCID/beige mouse" refers to the C.B-17-SCID-beige mouse strain. The terms SCID/beige, SCID-beige and SCID/bg are used interchangeably herein.

The term "human tumor cells" refers to tumor cells of human origin; a tumor cell is a neoplastic or cancerous cell. Tumor cells may be "established tumor cells," *i.e*., those which can be maintained indefinitely in tissue culture or may be primary tumor cells," *i.e*., tumor cells freshly isolated or explanted from a patient. The term "primary tumor cells" encompasses primary tumor cells maintained in tissue culture for less than or equal to 5 passages.

The term "human immune cell" refers to cells of the immune system (*e.g*., T, B and NK lymphocytes, antigen presenting cells) that are of human origin.

The term "human peripheral blood lymphocytes" refers to nucleated, non-erythroid cells derived from the blood of a human. The terms peripheral blood lymphocytes (PBLs) and peripheral blood mononuclear cells (PBMCs) are used herein interchangeably.

The term "central nervous system cells" refers to cells derived from the central nervous system (*i.e*., cells derived from the brain and spinal cord).

A mouse "reconstituted with human peripheral blood lymphocytes" is a mouse in which human PBLs have been introduced (*e.g*., by intraperitoneal injection) and persist for a period of at least 4 weeks.

A "SCID/beige mouse comprising human immune cells" is a SCID/bg mouse that has been reconstituted with human PBLs or another source of human immune cells (*e.g*., human fetal hematopoietic tissues) and thus, contains human immune cells. SCID/beige mouse comprising human immune cells whose serum contains at least 100 µg/ml of human immunoglobulin (Ig) are preferentially employed for the evaluation of vaccines.

An "injectable preparation" is a preparation suitable for injection into an animal (*e.g*., a mouse).

An "immune-modulating gene" is a gene encoding a protein that modulates an immune response. Examples of immune-modulating genes include, but are not limited to, cytokines, costimulatory molecules and chemotactins. The product of an immune-modulating gene is said to be an "immune modulator."

A "cytokine" is a hormone-like protein, typically of low molecular weight, that regulates the intensity and/or duration of an immune response and is involved in cell to cell communication. Examples of cytokines include but are not limited to the interleukins (*e.g*., interleukin 2, interleukin 4, interleukin 6, interleukin 7, interleukin 12), granulocyte-macrophage colony stimulating factor, granulocyte colony stimulating factor, interferon-gamma (IFN-γ) and tumor necrosis factor-alpha (TNF-α).

The term "cell cycle regulator" refers to any protein whose activity modulates progression of the cell cycle. Particularly preferred cell cycle regulators are those that block cell cycle progression. Examples include but are not limited to the HIV vpr gene product, p21, inhibitors of mammalian cyclins, etc.

The term "inducer of apoptosis" refers to any protein whose activity induces apoptosis in a cell. Inducers of apoptosis include but are not limited to apoptin (the product of the chicken anemia virus VP3 gene), BAX, BAD, a BCL-X derivative and the HIV vpr gene product.

The term "vaccine" as used herein refers to any preparation that gives rise to an antigen or antigens that is or can be presented to a cell of an animal immune system in order to elicit a prophylactic and/or therapeutic immune response to the antigen.

The term "expression vector" as used herein refers to a recombinant DNA molecule containing a desired coding sequence and appropriate nucleic acid sequences necessary for the expression of an operably linked coding sequence in a particular host organism. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals.

Transcriptional control signals in eucaryotes comprise "promoter" and "enhancer" elements. Promoters and enhancers consist of short arrays of DNA sequences that interact specifically with cellular proteins involved in transcription [Maniatis, et al., Science 236:1237 (1987)]. Promoter and enhancer elements have been isolated from a variety of eukaryotic sources including genes in yeast, insect and mammalian cells and viruses (analogous control elements, *i.e*., promoters, are also found in prokaryotes). The selection of a particular promoter and enhancer depends on what cell type is to be used to express the protein of interest. Some eukaryotic promoters and enhancers have a broad host range while others are functional in a limited subset of cell types [for review see Voss, et al., Trends Biochem. Sci., 11:287 (1986) and Maniatis, *et al., supra* (1987)]. For example, the SV40 early gene enhancer is very active in a wide variety of cell types from many mammalian species and has been widely used for the expression of proteins in mammalian cells [Dijkema, et al., EMBO J. 4:761 (1985)]. Two other examples of promoter/enhancer elements active in a broad range of mammalian cell types are those from the human elongation factor 1α gene [Uetsuki et al., J. Biol. Chem., 264:5791 (1989); Kim et al., Gene 91:217 (1990); and Mizushima and Nagata, Nuc. Acids. Res., 18:5322 (1990)] and the long terminal repeats of the Rous sarcoma virus [Gorman et al., Proc. Natl. Acad. Sci. USA 79:6777 (1982)] and the human cytomegalovirus [Boshart et al., Cell 41:521 (1985)].

The term "promoter/enhancer" denotes a segment of DNA which contains sequences capable of providing both promoter and enhancer functions (for example, the long terminal repeats of retroviruses contain both promoter and enhancer functions). The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e*., molecular biological techniques).

The presence of "splicing signals" on an expression vector often results in higher levels of expression of the recombinant transcript. Splicing signals mediate the removal of introns from the primary RNA transcript and consist of a splice donor and acceptor site [Sambrook et al., Molecular Cloning: A Laboratory Manual, 2nd ed., Cold Spring Harbor Laboratory Press, New York (1989) pp. 16.7-16.8]. A commonly used splice donor and acceptor site is the splice junction from the 16S RNA of SV40.

Efficient expression of recombinant DNA sequences in eukaryotic cells requires signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence which directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one which is isolated from one gene and placed 3' of another gene. A commonly used heterologous poly A signal is the SV40 poly A signal. The SV40 poly A signal is contained on a 237 bp *Bam*HI*lBcl*I restriction fragment and directs both termination and polyadenylation [Sambrook, *supra,* at 16.6-16.7]. This 237 bp fragment is contained within a 671 bp *Bam*HI/*Pst*I restriction fragment.

The term "stable transfection" or "stably transfected" refers to the introduction and integration of foreign DNA into the genome of the transfected cell. The term "stable transfectant" refers to a cell which has stably integrated foreign or exogenous DNA into the genomic DNA of the transfected cell.

The terms "selectable marker" or "selectable gene product" as used herein refer to the use of a gene which encodes an enzymatic activity that confers resistance to an antibiotic or drug upon the cell in which the selectable marker is expressed. Selectable markers may be "dominant"; a dominant selectable marker encodes an enzymatic activity which can be detected in any mammalian cell line. Examples of dominant selectable markers include the bacterial aminoglycoside 3' phosphotransferase gene (also referred to as the *neo* gene) which confers resistance to the drug G418 in mammalian cells, the bacterial hygromycin G phosphotransferase (*hyg*) gene which confers resistance to the antibiotic hygromycin and the bacterial xanthine-guanine phosphoribosyl transferase gene (also referred to as the *gpt* gene) which confers the ability to grow in the presence of mycophenolic acid. Other selectable markers are not dominant in that their use must be in conjunction with a cell line that lacks the relevant enzyme activity. Examples of non-dominant selectable markers include the thymidine kinase (*tk*) gene which is used in conjunction with TK- cell lines, the carbamoyl-phosphate synthetase-aspartate transcarbamoylase-dihydroorotase (CAD) gene which is used in conjunction with CAD-deficient cells and the mammalian hypoxanthine-guanine phosphoribosyl transferase (*hprt*) gene which is used in conjunction with HPRT cell lines. A review of the use of selectable markers in mammalian cell lines is provided in Sambrook *et al., supra* at pp.16.9-16.15. It is noted that some selectable markers can be amplified and therefore can be used as amplifiable markers (*e.g*., the CAD gene).

### Detailed Disclosure of the Invention

In one embodiment, the present invention provides humanized animal models suitable for the evaluation of anti-human tumor immunity. These animal models permit the identification of combinations of immune-modulating genes (IMGs) which when delivered to human tumor cells induce an effective anti-tumor response, including a systemic anti-tumor response. The Description of the Invention is divided into the following sections: I. Immunogene Therapy; II. The Hu-SCID/beige Model For Immunotherapy; III. Combination Immunogene Therapy; IV. Evaluation Of Candidate Vaccines Using The Hu-PBL-SCID/beige Model; V. HIV Vaccines; VI. Alternative Strategies For Cancer Therapy And Vaccine Production; and VII. Computer-Assisted Immunospot Analysis.

### I. Immunogene Therapy

Immunogene therapy involves the introduction of genes encoding proteins that regulate or modulate the immune response. Particularly useful in the present invention are those genes that encode proteins that are involved in the activation of T cells. As discussed above, the majority of tumors are not immunogenic, *i.e*., they fail to provoke an immune response in an animal.

### II. The Hu-PBL-SCID/beige Model For Immunotherapy

The C.B-17 SCID/beige (SCID/bg) mouse, an immunocompromised mouse, was employed for the humanized mouse model as this strain of mice lacks T cell, B cell and natural killer (NK) cell function [Froidevaux and Loor (1991) J. Immunol. Methods 137:275]. As shown herein, the SCID/bg mouse supports the growth of a variety of established human tumor cell lines as well as primary human tumor cells. SCID/bg mice were efficiently reconstituted with human peripheral blood lymphocytes (PBLs) with CD45+ human cells constituting up to 60% of the splenocytes and 2-7% of the peripheral blood mononuclear cells in the reconstituted mice.

Importantly, the peripheral blood of the Hu-PBL-SCID/bg mice were found to contain high numbers of immature or progenitor T cells (*i.e*., CD4+8+ cells and CD45RA+ cells). These results are in contrast to the results obtained by reconstitution of C.B-17scid/scid mice (Hu-PBL-SCID). In human PBL-reconstituted C.B-17 scid/scid mice, most human lymphocytes exhibit activated cell phenotypes (HLA-DR+ and CD25+ or CD69+) soon after reconstitution, and almost all (>99%) human T cells exhibit mature memory phenotypes (CD45RO+) in a state of reversible anergy [Rizza *et al., supra;* Tarry-Lehmann and Saxon, *supra;* Tarry-Lehmann *et al.* (1995), *supra*]*.* Therefore, the lack of sufficient numbers of immature naive T cells after reconstitution renders the Hu-PBL-SCID model unsuitable for the evaluation of anti-tumor immunity. In contrast, the Hu-PBL-SCID/bg mice show evident levels of CD45RA+ and CD4+8+ cells 4-6 weeks after reconstitution. Thus, the Hu-PBL-SCID/bg mice of the present invention provide a suitable model for the evaluation of anti-tumor immunity.

The use of the Hu-PBL-SCID/bg mice as a model for human immunogene therapy is illustrated herein for the identification of a combination of immune-modulating genes (IMGs) effective in the treatment of human glioblastoma multiforme.

Glioblastoma multiforme is the most common primary central nervous system neoplasm in humans. Despite improvements in diagnosis and treatment of glioblastoma multiforme, mean survival from time of diagnosis remains less than one year [Chang et al. (1983) Cancer 52:997; McDonald and Rosenblum (1994) In: Principles of Neurosurgery, Regachary and Wilkins, eds., Wolfe Publishing, Toronto, pp. 26.21-26.32].

Although glioblastomas *in situ* are normally infiltrated to varying degrees by lymphocytes [Kuppner et al. (1989) J. Neurosurgery 71:211; Black et al. (1992) J. Neurosurgery 77:120], evidence indicates these lymphocytes are unactivated. This may be in part due to secretion of immunosuppressive factors such as prostaglandin E₂, transforming growth factor β₂, and interleukin-10, all of which inhibit lymphocyte activation [Fontana et al. (1982) J. Immunol. 129:2413; Siepl et al. (1988) Eur. J. Immunol. 18:593; Kuppner *et al., supra;* Sawamura et al. (1990) J. Neuro-Oncol. 9:125; Nitta et al. (1994) Brain Res. 649:122; Huettner et al. (1995) Am. J. Pathol. 146:317]. Immunogene therapy strategies were designed to overcome such local immunosuppression by promoting tumor antigen presentation and/or anti-tumor lymphocyte activation.

As discussed above, most preclinical immunogene therapy studies have used rodent genes and rodent tumor models. However, the applicability of such models to human systems is unclear. This is particularly true for glioblastoma models utilizing rodent tumors (rat 9L-glioma and C6-glioma) which have sarcomatous features that are significantly different from human glioblastoma multiforme [Benda et al. (1968) Science 161:370; Barker et al. (1973) Cancer Res. 33:976; Day and Bigner (1973) Cancer Res. 33:2362]. Therefore, the present invention provides a novel humanized mouse model utilizing a human glioblastoma cell line, human immunogenes and human lymphocytes.

Using retroviral vectors, genes encoding human GM-CSF and/or B7-2 were efficiently transferred *in vitro* into a human glioblastoma cell line. Thereafter, the effect of GM-CSF and/or B7-2 expression on glioblastoma growth *in vivo* was examined in a human tumor/human PBL /severe combined immunodeficiency mouse (hu-PBL-SCID) model. Human glioblastoma cells (with or without therapeutic gene transfer) and human PBLs were grafted into SCID/bg or SCID/nod mice and the effect on tumor growth locally and at distant sites was observed. SCID/bg and SCID/nod mice accept human tumor and lymphocyte grafts without rejection as they lack mature T, B, and NK lymphocytes.

As shown herein, inhibition ofGM-CSF and B7-2-transduced tumors was seen in human lymphocyte-reconstituted SCID/bg mice demonstrating that expression of these genes by glioblastoma cells overcomes local immunosuppression and results in a significant antitumor immune response. Furthermore, inhibition of wild type challenge growth in mice vaccinated with irradiated tumor cells transduced with B7-2 and GM-CSF demonstrated that expression of these genes by glioblastoma cells induced a systemic immune response that inhibits tumor growth at distant sites. These results provide the first *in vivo* demonstration of human GM-CSF immunogene therapy in a human glioblastoma model.

The human glioblastoma-Hu-PBL-SCID/bg model employed herein represents an allogeneic system that comprises an established human glioblastoma cell line and lymphocytes from unrelated donors. This model has more similarities to autologous systems than may be immediately apparent. Unlike the classical immune-mediated rejection seen in allogeneic organ transplantation, this system is free of graft-origin "passenger lymphocytes." These lymphocytes are important in initiating allogeneic organ rejection responses by presenting antigen to host lymphocytes in the context of allogeneic Class II Major Histocompatibility Complex (MHC) [Larsen et al. (1990) Annals Surgery 212:308; Chandler and Passaro (1993) Archives Surg. 128:279; Moller (1995) Transplantation Proc. 27:24]. The D54MG glioblastoma cell line employed in this model expresses only Class I MHC and not Class II MHC *in vitro.* Therefore, the only Class II MHC molecules available for tumor antigen presentation in this model are those present on engrafted human PBLs. In other words, this model glioblastoma system is allogeneic for Class I MHC but autologous for Class II MHC.

As described herein, the human tumor/Hu-PBL-SCID/bg model can be used as an autologous model system comprising human tumor cells and PBLs from the same patient. Such an autologous model is preferred to an allogeneic model system; however, for certain rapidly progressing tumors it may be difficult to obtain PBLs from the same patient once the patient's tumor has been established in the Hu-PBL-SCID mice. In these cases, an allogeneic model, using PBLs from a donor unrelated to the tumor donor is employed.

The human tumor/Hu-PBL-SCID/bg model of the present invention provides a simple and powerful method to analyze human lymphocyte responses to human tumors *in vivo* and thus provides a means to determine which combination of IMGs are best suited for the treatment of specific tumors.

### III Combination Immunogene Therapy

The human tumor/Hu-PBL-SCID/bg model of the present invention provides a simple and powerful method to determine which combination of IMGs or IMGs and/or cell cycle regulators, inducers of apoptosis and tumor suppressor genes (*e.g*., the wild type p53 gene) are best suited for the treatment of specific tumors.

Many immunomodulatory genes are potentially useful and more than one may be necessary for overcoming tumor immunosuppression. These include genes encoding cytokines, major histocompatibility complex molecules, and T cell costimulatory molecules [Dranoff et al. (1993) Proc. Natl. Acad. Sci. USA 90:3539; Gajewski et al. (1995) J. Immunol. 154:5637]. An ideal tumor vaccine should coexpress a combination of immunostimulatory genes from distinct immunomodulatory pathways (*e.g*., costimulators, cytokines, and chemoattractive adjuvants) and may also express cell cycle regulators, inducers of apoptosis and tumor suppressor genes.

In the illustrative example provided herein, a combination of the T cell costimulatory molecule B7-2 and the therapeutic cytokine granulocyte-macrophage colony stimulating factor (GM-CSF) is employed to increase the immunogenicity of a human glioblastoma cell line. Genes encoding the B7-2 and GM-CSF proteins were introduced into and co-expressed in the tumor cells. Tumor cells modified to express at least one immune-modulating gene and another immune-modulating gene, a cell cycle regulator, an inducer of apoptosis, and/or a tumor suppressor gene can be used as a tumor vaccine according to the methods of the present invention.

GM-CSF stimulates growth and differentiation of granulocytes, monocytes/macrophages, microglia, and other antigen presenting cells. It has recently come into widespread clinical use as a treatment of neutropenia due to its hematopoietic effects [Lieshcke and Burgess (1992) N. Engl. J. Med. 327:28; Aglietta et al. (1994) Seminars Oncol. 21:5; Engelhard and Brittinger (1994) Seminars Oncol. 21:1]. The importance of this cytokine in tumor immunogene therapy was recently demonstrated by Dranoff*, et al.* who showed that vaccination with irradiated GM-CSF-transduced tumor cells produced specific and marked growth inhibition of wild type tumor challenges in mouse models of adenocarcinoma and melanoma [Dranoff *et al., supra*]*.* Of 10 cytokine genes tested, GM-CSF resulted in the greatest tumor growth inhibition in this mouse model.

B7-2 is one of a family (B7-1, B7-2, and B7-3) of lymphocyte cell surface molecules that have recently been identified as costimulatory molecules necessary for T-cell activation in conjunction with antigen presentation in the context of a major histocompatibility complex molecule. The absence of costimulatory molecules on tumor cells may contribute to their failure to be detected and eliminated by the immune system [Galea-Lauri et al. (1996) Cancer Gene Ther. 3:202]. The specific roles of the various costimulatory molecules are yet to be clearly defined. It has been suggested that B7-1 expression promotes differentiation of intermediate T_{H} cell precursors into T_{H}1 effector cells (cellular immune responses) while B7-2 expression leads to T_{H}2 differentiation (humoral immune responses) [Kawamura and Furue (1995) Eur. J. Immunol. 25:1913; Thompson (1995) Cell 71:979]. However, both B7-1 and B7-2 have been shown to promote cell mediated immune responses in animal models [Hodge et al. (1994) Cancer Res. 54:5552; Lanier et al. (1995) J. Immunol. 154:97; Plumas et al. (1995) Eur. J. Immunol. 25:3332]. More recently, the effectiveness of B7-1 expression in promoting tumor rejection has been questioned (Wu *et al., supra*). Furthermore, T cells must receive costimulatory signals from APCs within the first 12 hours of T cell receptor stimulation for maximal interleukin-2 production [Mondino and Jenkins (1994) J. Leuckocyte Biol. 55:805]. Therefore, the rapid induction of B7-2 (not B7-1) on APC's after antigen stimulation suggests that B7-2 is the preferable costimulatory molecule to promote antitumor cell-mediated immune responses (Galea-Lauri *et al., supra*).

Other IMGs to be examined for their effectiveness in treating human tumors in the novel animal models of the present invention include, but are not limited to, APO-1 (Fas), APO-1 ligand (FasL) [Hahne et al. (1996) Science 274:1363; Seino et al. (1997) Nature Med. 3:165; Strand et al. (1996) Nature Med. 2:1361], IL-12A and IL-12B, IL-2, IL-4, IL-6, IL-7, IL-10, GM-CSF, G-CSF, IFN-γ, CD40 and TNF-α. In addition, genes encoding cell cycle regulators or inducers of apoptosis can be employed in combination with IMGs for the modification of tumor cells. Expression vectors, including retroviral vectors, containing one or more IMG (and/or cell cycle regulators or inducers of apoptosis) are constructed as described herein. When more than one IMG (or genes encoding cell cycle regulators or inducers of apoptosis) is to be contained on the same construct, each IMG is preferably separated from the other(s) using an IRES as described herein. A particularly preferred IRES is the poliovirus IRES.

Once an effective combination of IMGs has been identified for a particular human tumor, those IMGs are delivered to a patient's tumor cells *in vivo* or *in vitro* followed by a return of the modified tumor cells (typically the modified cells will be irradiated prior to introduction) to the patient. As described more fully in the examples below, a variety of means may be employed for the delivery of IMGs to human tumor cells (*e.g*., biolistic transformation of tumor cells *in situ,* cationic liposomes, retroviral infection, etc.).

### (a) Combination Immunogene Therapy For Glioblastoma Using B7-2 and GM-CSF

As shown in the examples below, the human tumor/Hu-PBL-SCID/bg model of the present invention was employed to determine that tumor growth was markedly inhibited when these animals were vaccinated with glioblastoma cells transduced with genes encoding the T cell costimulatory molecule B7-2 and the proinflammatory cytokine GM-CSF.

Treatment of patients having glioblastoma multiforme tumors can be conducted as follows. One to three grams of tumor are harvested when patients originally present and undergo surgery. The tissue is harvested and treated as described in Ex. 11. Tumor cells are grown until sufficient numbers are present to allow retroviral gene transfer and selection.

The primary tumor cells are transfected with pLSNBG9 (encodes both B7-2 and GM-CSF) and selected by growth in the presence of G418 as described below. Briefly, virus stock is thawed from -80°C at 37°C. Polybrene is added to the thawed virus solution at a final concentration of 4 µg/ml. Culture medium is added to the virus solution to bring the final volume to 1.5 ml. Logarithmic growth phase tumor cells in T25 flasks are incubated in the virus supernatant at 37°C, 5% CO₂ for 3 hours. The same volume of medium containing polybrene but lacking virus is used as a control. After 3 hours, a further 3 ml of culture medium is added to each flask and the cells are incubated overnight. Medium is changed the next morning. Twenty-four hours after retroviral transduction, 200 µg/ml (final concentration) of the neomycin analog G418 is added to the culture medium. Medium is changed every 2 to 3 days until complete selection has taken place (*i.e*., all cells in the control flask are dead). After selection, cells are cultured in growth medium containing reduced concentrations of G418 (100 µg/ml) and are allowed to grow to confluence.

Transduced tumor cells are split into a new T25 culture flask at a density of 1 x 10⁶ cells. the amount of GM-CSF secreted into the culture medium is determined by ELISA (Quantikine, R&D Systems) 24 hr later. B7-2 expression is evaluated by flow cytometry using a monoclonal antibody specific for the human B70 antigen (*i.e*., B7-2). Aliquots of transduced cells to be used as vaccines are tested for the presence of bacteria, fungi, mycoplasma, HIV, Hepatitis B and Hepatitis C and replication-competent retrovirus (using the standard S+L- assay; Bassin et al. (1971) Nature 229:564).

Tumor cells that have been transduced with GM-CSF and B7-2 genes, selected and expanded are cyropreserved for future use. Briefly, cells are aliquoted in small volumes into cryopreservation tubes at 1 x 10⁶ cells/tube. Total volume is made up to 0.5 ml with a mixture of DMEM/F12, FCS and DMSO to make a final concentration of 10% FCS and 20% DMSO. Cells are placed in an insulated styrofoam rack and placed at -80°C for 24 hr, then placed in liquid nitrogen for long term storage. When cells are needed, they are thawed from liquid nitrogen at 37°C, washed twice in fresh medium and plated.

Transduced tumor cells are irradiated prior to their reinjection into patients to render the cells replication incompetent. Cells are irradiated with 20,000 Rad in a ⁶⁰Cobalt machine.

Patients are given three subcutaneous injections in total. Each vaccination comprises 2 x 10⁶ irradiated autologous tumor cells modified to express B7-2 and GM-CSF. Injections are given on alternating lumbar flank regions which are marked immediately above the injection site with India ink to allow accurate localization later in the event that a local reaction is not apparent. Patients receive 0.1 ml of vaccine injected SC at each site using a 1 ml syringe fitted with a 23 gauge needle. All vaccinations are prepared by resuspension of cells in sterile Ringer's lactate solution. Patients having recurrent glioblastomas, the first injection is given on the first or second post-operative day. For patients with treatment resistant melanomas (discussed below), the timing of the first injection is not as critical as these patients are not undergoing any further surgical resection of their tumors. All patients receive a second and third vaccination 14 and 28 days, respectively after the first vaccination.

With the exception of the first vaccination in patients with recurrent glioblastomas (who are likely post-operative inpatients at the time), all subjects are treated as outpatients. Vital signs are monitored prior to immunization and every half hour for 3 hours after the SC injections. Patients are examined every hour for 3 hours for inflammation at the injection site and for evidence of rash, wheezing or edema. Provided there are no contraindications, subjects are discharged 3 hours after treatment. Should significant reactions occur, the patient is hospitalized for constant monitoring.

Patients are assessed in the clinic 3 days after vaccination and are evaluated weekly for 8 weeks and thereafter monthly for 4 months, every 3 months for 1 year and yearly thereafter. Blood samples are obtained for standard blood chemistries and histology at each visit. In addition, blood id drawn one week after each vaccination for replication-competent retrovirus assays.

Patients are observed for any toxicities. Patients' immunologic reaction to immunogene therapy is monitored locally and systemically. Local immune response is monitored by symptoms and signs of delayed type hypersensitivity (DTH) responses at the vaccination sites. In addition, punch biopsies are performed at injection sites 2 weeks after each vaccination (*i.e*., days 14, 28 and 42). These biopsies are compared to a biopsy taken from normal lumbar flank skin on day 1 (prior to initiation of therapy). Biopsies undergo standard pathologic examination for evidence of tumor cells and inflammation. In addition, immunohistochemical staining for CD45, CD4, CD8 and NK cell markers is performed.

Systemic immune responses are measured using two separate assays. Blood samples (20 ml each) are obtained on days 0, 7, 21, 35 and 49. These samples are used to isolate peripheral blood mononuclear cells (PBMC or PBLs) by centrifugation on a density (Hystopaque) gradient. The PBMC are then stimulated *in vitro* by co-incubation for 5 days with irradiated (20,000 Rad) autologous tumor cells. The stimulated PBMC are then used in the following two assays. First, a standard ⁵¹Chromium release cytotoxic T lymphocyte (CTL) assay is performed vs. autologous tumor cells. Second, an ELISPOT assay for interferon-γ production after exposure to autologous tumor cells is performed [Zhang et al. (1996) Proc. Natl. Acad. Sci. USA 93:14720]. The ⁵¹Chromium release CTL assay is a standard assay for cell mediated immunity. This assay gives direct information concerning the ability of stimulated PBMC to kill tumor cells; however it has a relatively low sensitivity. To overcome this, the much more sensitive ELISPOT assay for interferon-γ production is also used. The ELISPOT assay determines the concentration of PBMC present that produce interferon-γ in response to exposure to autologous tumor cells. Since interferon-γ production is closely associated with T_{H}1 (cell mediated) immune responses, the number of PBMC producing interferon-γ in response to exposure to autologous tumor provides a measure of cell mediated immunity.

The clinical status of patients is followed by history, physical and laboratory parameters. In addition, appropriate diagnostic imaging tests (*e.g*., MRI scans with and without gadolinium enhancement) are obtained at 8 and 24 weeks, every 3 months for the following year, and yearly thereafter.

### (b) Combination Immunogene Therapy For Malignant Melanoma Using B7-2 and GM-CSF

Malignant melanoma is rapidly rising in North America. In contrast to other forms of skin cancers which are usually curable with surgery, malignant melanoma is often fatal due to its aggressive nature and tendency of early spread to distant organs.

Treatment of patients having malignant melanoma can be conducted using tumor cells modified to express B7-2 and GM-CSF as described above. Tumor cells are harvested from patients when patients originally present and undergo surgery and the cells are treated as described in Example 11.

Combinations of other IMGs (and/or genes encoding cell cycle regulators or inducers of apoptosis) shown to be effective at reducing tumorigenicity and or at inducing local or systemic immunity in the human tumor/Hu-PBL-SCID/bg model of the present invention are employed to treat human tumors, including glioblastoma and malignant melanoma.

In addition to the method of treatment described above wherein the patient's tumor cells are transduced with retroviruses encoding immune-modulators (*e.g*., B7-2 and GM-CSF) (and/or cell cycle regulators or inducers of apoptosis) and the tumor cells are selected in culture prior to reintroduction into the patient, the patient's tumor cells may be modified by introduction of DNA encoding the desired gene(s) (*e.g*., plasmid DNA transferred by biolistics or other physical means, recombinant adenoviruses, liposomes, direct injection of naked DNA). The cells are then allowed to express the transduced genes for a few days (or less), irradiated and used to immunize the patient. Subsequent boost immunizations may employ retrovirally transduced and selected tumor cells. The use of plasmid DNA (delivered by liposomes, biolistics, adenovirus vectors or as naked DNA) to modify tumor cells is preferred to the use of recombinant retroviruses in those cases where the patient's tumor cells take a lengthy period to grow in cell culture as retroviral transduction and selection takes a period of several weeks and for certain tumors (*e.g*., glioblastomas) it is desirable to vaccinate the patient with modified tumor cells within a few days (*e.g., ∼* 3) of the initial surgery. Vaccination within ∼3 days of tumor debulking may permit the capture of the "alarm signal" required for costimulator activation on APCs and enhancement of activated macrophage and T cell traffic across the blood-brain barrier [Fuchs and Matzinger (1992) Science 258:1156 and Matzinger (1994) Annu. Rev. Immunol. 12:991].

### IV. Evaluation Of Candidate Vaccines Using The Hu-PBL-SCD/beige Model

As discussed above, the present invention provides humanized animal models that are useful for the evaluation of tumor cell vaccines. The hu-PBL-SCID/beige mice of the present invention also provide animal models for the evaluation of vaccines designed to confer immunity against a variety of human pathogens. The examples below provide detailed protocols for the establishment of hu-PBL-SCID/bg mice and humanized SCID/bg mice (*i.e*., SCID/bg mice comprising transplanted human fetal hematopoietic tissues). These animals contain human immune cells and are used to evaluate candidate vaccines (*e.g*., HIV vaccines, malaria vaccines, Leishmania vaccines, *M. tuberculosis* vaccines, etc.). Both cellular anti-pathogen immunity and humoral anti-pathogen immunity is assessed in the reconstituted and vaccinated animals.

The immunization efficacies of heterologous vaccines of human pathogens can be evaluated in SCID/beige mice intraperitoneally injected with human peripheral blood mononuclear cells (PBMCs) or receiving human fetal thymus/liver tissue transplants. *In vivo* human immune cell reconstitution in these animals has been demonstrated by analyzing the expression of different human leukocyte markers, including markers for naive T cells (CD45RA), memory T cells (CD45RO), activation (HLA-DR) and CD4 and CD8 cells. The *in vivo* immune functions of the human cells in the reconstituted animals have been demonstrated using an HIV-1 protective immunity model (*see e.g.,* Ex. 16), and an attenuated *Leishmania* cell vaccine model (*see* Ex. 19). Other examples including immunization against malaria and tuberculosis with DNA vaccines encoding malaria multistage antigens or antigens of Bacille Calmette-Guerin (BCG) strains. SCID/beige mice are injected with freshly isolated PBMCs as described and one week later, human Ig levels are determined by ELISA. Hu-PBL-SCID/bg mice whose serum contains greater than 100 microgram/ml of human Igs are immunized via intramuscular injection with naked DNA vaccines or with dendritic cells (DCs) transfected with naked DNA vaccines which express appropriate pathogen antigens. The immunized mice are re-immunized 1-2 weeks later. The immunized mice are challenged with wild type pathogens such as HIV-1, malaria, *Mycobacterium tuberculosis* or Leishmania major one week after the last dose of vaccine. The control mice receive DNA vaccines lacking genes encoding the pathogen antigens. After wild type pathogen challenge, infection and/or disease development is examined according to standard methodology. The human immune cell-originated anti-pathogen immunity is analyzed, for cell-mediated immune (CMI) responses using reconstituted mouse splenocytes, and for humoral immune responses using antisera.

### V. HIV Vaccines

The examples below provide details for the production of HIV vaccines, including live attenuated HIV vaccines, HIV proviral DNA vaccines and modified *Leishmania* cells expressing HIV proteins. The ability of these HIV vaccines to induce cellular anti-HIV immunity and humoral anti-HIV immunity is assessed in hu-PBL-SCID/bg mice (reconstituted with PBLs or transplanted with fetal hematopoietic tissue) as described above and in the examples below.

### VI. Alternative Strategies For Cancer Therapy and Vaccine Production

In addition to the approaches outlined above for the induction of immune responses to tumor cells and human pathogens, the following alternative approaches are provided.

### (a) Use Of Replication-Competent Viruses Comprising Suicide Genes

As discussed above replication-incompetent MoMLV-based vectors can be employed to deliver therapeutic genes to tumor cells (section IV). Another approach to delivering genes to tumor cells for the purpose of cancer therapy is the use of replication-competent retroviral vectors (*e.g*., MoMLV-based vectors) carrying "suicide" genes [*e.g*., the Herpes simplex virus thymidine kinase (HSV *tk*) gene]. A suicide gene is a negative selectable marker whose expression can be selected against. That is under certain conditions, cells expressing the suicide gene are selectively killed. For example, cells expressing the HSV *tk* gene are killed when they are grown in the presence of gancyclovir. The HSV *tk* gene has been employed for cancer gene therapy. Expression vectors containing the HSV *tk* gene were injected into the brains of patients having brain tumors and the patients were given gancyclovir. These experiments have met with little success as expression of the HSV *tk* gene was limited to cells along the injection track only; that is the HSV *tk* gene could not be delivered to enough of the tumor cells using this approach to kill enough tumor cells to make a difference in the outcome of the disease.

To deliver a suicide gene to a large percentage of the tumor cells in a patient, a replication-competent retroviral vector is employed. Retroviruses, such as MoMLV, preferentially infect rapidly dividing cells; as tumor cells are generally the most rapidly dividing cells in a patient, retroviruses may be employed to deliver genes to the tumor cells. The use of a replication competent retroviral vector permits the spread of the therapeutic gene to tumor cells outside of cells located at the site of injection of the vector. Replication-competent MoMLV vectors suitable for the delivery of HSV *tk* include, but are not limited to, Moloney leukemia virus (MLV) vectors [RNA Tumor Viruses (1995) Molecular Biology of Tumor Viruses, Cold Spring Harbor Laboratory Press, Cold Spring Harbor, NY]. The HSV *tk* gene is inserted into the MoMLV vector using standard recombinant DNA techniques to create a suicide vector and DNA comprising the suicide vector is prepared. The suicide vector DNA is injected into the patient's tumor (intratumor injection) and the patient is then given gancyclovir (i.v. injection) to permit the killing of tumor cells expressing the HSV *tk* gene.

### (b) In Situ Production Of Producer Cells

In addition to the use of replication-defective retroviral vectors, attenuated viruses as a means of delivering vaccines for cancer therapy or the prevention of pathogenic infections, the present invention contemplates the injection of DNA constructs encoding the components necessary for the packaging of a retroviral vector along with a therapeutic vector into the tumor cells of a patient. Three separate DNA constructs are injected: 1) a DNA expression vector encoding the *gag-pol* genes of a retrovirus (*e.g.,* MoMLV); 2) a DNA expression vector encoding the envelope gene of a retrovirus or a vesicular stomatitis virus G protein; and 3) a replication-defective retroviral vector (*e.g*., a vector lacking packaging signals and gag, pol and/or env genes) comprising a therapeutic gene(s) (*e.g*., GM-CSF, B7-2, etc.). Cells receiving all three DNA constructs will package and shed pseudotyped viral particles comprising RNA derived from the retroviral vector containing the therapeutic gene(s). In this manner, packaging cell lines are created *in situ* in the patient's tumor. Such an approach is superior to the injection of packaging cell lines into a patient. When packaging cell lines (typically mouse fibroblast cell lines shedding a replication defective virus) are injected into a patient, the patient's immune response is often directed against the foreign cellular proteins rather than against the desired target (the tumor cell). In contrast, the injection of DNA comprising the components of the packaging cell line, rather than the cell line itself, permits the immune response to be directed at the desired target.

### VII. Computer-Assisted Immunospot Analysis

The present invention also concerns immunospot assay methods, such as, for example, an enzyme-linked immunospot (ELISPOT) assay [Cui and Chang (1997) BioTechniques 22:1146-1149], that utilize computer-assisted, video-imaging analysis. Originally developed for detecting antibody production from B lymphocytes, the immunospot assay has also been used to assess cytokine production from various immune effector cells. Although the immunospot assay can detect antibody or cytokine production at the single cell level, the visual counting of spots in a 96-well plate under microscope makes this method unsuitable for handling large sample sizes. The computer-assisted image analysis system and methods used with the immunospot assay according to the subject invention overcomes this problem. This system makes the data analysis step of the immunospot assay convenient, objective, reproducible and suitable for handling large sample pools. Studies requiring lymphocyte proliferation assay, cytotoxic lymphocyte assay, and precursor frequency assay can be conducted using the immunospot assay of the invention. This is demonstrated using examples such as mixed lymphocyte allogeneic reactions and the human immunodeficiency virus antigen-specific, cell-mediated immune responses.

In one embodiment, the methods of the subject invention comprise performing an immunospot assay wherein a detectable signal produced by the assay is converted to a digital image or signal using computer-assisted video imaging means. In an exemplified embodiment, the detectable signal is produced by means of an enzyme substrate reaction that produces a detectable colorimetric signal. Once the detectable signal has been processed into a digital image, the image can be analyzed using analysis software, such as, for example, NIH Image Version 1.55 shareware. The methods of the invention can be used with one- or two-color immunospot assays. Using the methods of the present invention eliminates manual microscopic counting of immunospot assay results and provides objective and reproducible immunospot assay analyses.

The following examples serve to illustrate certain preferred embodiments and aspects of the present invention and are not to be construed as limiting the scope thereof.

In the experimental disclosure which follows, the following abbreviations apply: M (molar); mM (millimolar); µM (micromolar); mol (moles); mmol (millimoles); µmol (micromoles); nmol (nanomoles); g (gravity); gm (grams); mg (milligrams); µg (micrograms); pg (picograms); L (liters); ml (milliliters); µl (microliters); cm (centimeters); mm (millimeters); µm (micrometers); nm (nanometers); hr (hour); min (minute); msec (millisecond); °C (degrees Centigrade); AMP (adenosine 5'-monophosphate); cDNA (copy or complimentary DNA); DTT (dithiotheritol); ddH₂O (double distilled water); dNTP (deoxyribonucleotide triphosphate); rNTP (ribonucleotide triphosphate); ddNTP (dideoxyribonucleotide triphosphate); bp (base pair); kb (kilo base pair); TLC (thin layer chromatography); tRNA (transfer RNA); nt (nucleotide); VRC (vanadyl ribonucleoside complex); RNase (ribonuclease); DNase (deoxyribonuclease); poly A (polyriboadenylic acid); PBS (phosphate buffered saline); OD (optical density); HEPES (N-[2-Hydroxyethyl]piperazine-N-[2-ethanesulfonic acid]); HBS (HEPES buffered saline); SDS (sodium dodecyl sulfate); Tris-HCl (tris[Hydroxymethyl]aminomethane-hydrochloride); rpm (revolutions per minute); ligation buffer (50 mM Tris-HCl, 10 mM MgCl₂, 10 mM dithiothreitol, 25 µg/ml bovine serum albumin, and 26 µM NAD+, and pH 7.8); EGTA (ethylene glycol-bis(β-aminoethyl ether) N, N, N', N'-tetraacetic acid); EDTA (ethylenediaminetetracetic acid); ELISA (enzyme linked immunosorbant assay); LB (Luria-Bertani broth: 10 g tryptone, 5 g yeast extract, and 10 g NaCl per liter, pH adjusted to 7.5 with 1N NaOH); superbroth (12 g tryptone, 24 g yeast extract, 5 g glycerol, 3.8 g KH₂PO₄ and 12.5 g, K₂HPO₄ per liter); DME or DMEM (Dulbecco's modified Eagle's medium); ABI (Applied Biosystems Inc., Foster City, CA); ATCC (American Type Culture Collection, Rockville, MY); Beckman (Beckman Instruments Inc., Fullerton CA); Becton Dickinson (Becton Dickinson, San Jose, CA); BM (Boehringer Mannheim Biochemicals, Indianapolis, IN); Bio-101 (Bio-101, Vista, CA); BioRad (BioRad, Richmond, CA); Brinkmann (Brinkmann Instruments Inc. Wesbury, NY); BRL, Gibco BRL and Life Technologies (Bethesda Research Laboratories, Life Technologies Inc., Gaithersburg, MD); Caltag (Caltag Laboratories Inc., South San Francisco, CA); CRI (Collaborative Research Inc. Bedford, MA); Eppendorf (Eppendorf, Eppendorf North America, Inc., Madison, WI); Falcon (Becton Dickenson Labware, Lincoln Park, NJ); Invitrogen (Invitrogen, San Diego, CA); New Brunswick (New Brunswick Scientific Co. Inc., Edison, NJ); NEB or New England Biolabs (New England BioLabs Inc., Beverly, MA); Novagen (Novagen, Inc., Madision, WI); Pharmingen (PharMingen, San Deigo, CA); Pharmacia (Pharmacia LKB Gaithersburg, MD); Promega (Promega Corporation, Madison, WI); R & D Systems (R & D Systems Inc., Minneapolis, MN); Sigma (Sigma Chemical Co., St. Louis, MO); and Stratagene (Stratagene Cloning Systems, La Jolla, CA).

Unless otherwise indicated, all restriction enzymes were obtained from New England Biolabs and used according to the manufacturers directions. Unless otherwise indicated, synthetic oligonucleotides were synthesized using an ABI DNA synthesizer, Model No. 391.

### Example 1 - Construction Of Mono- And Bi-Cistronic Retroviral Vectors And Packaging Of Recombinant Virus

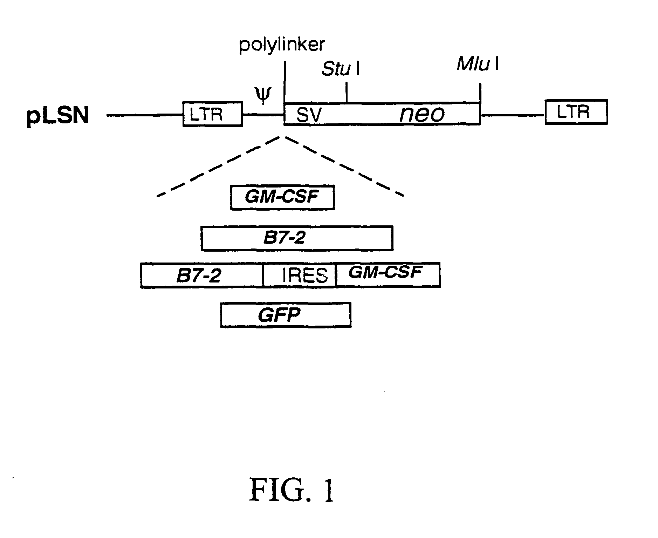

Retroviral gene therapy vectors were constructed that contained the genes for GM-CSF, B7-2, GM-CSF and B7-2, or GFP gene as shown schematically in Figure 1. Figure 1 provides a schematic showing the map of the parental vector (pLSN), pLSNB70 (encodes human B7-2), pLSNGM1 (encodes human GM-CSF), pLSN-BG9 (encodes both B7-2 and GM-CSF) and pLSN-GFP (encodes GFP).

All genes were cloned into the polylinker region of the MLV-based pLSN plasmid [Robinson et al. (1995) Gene Therapy 2:269 and co-pending Application Serial No. 08/336,132]. Therapeutic genes were inserted into pLSN such that their expression was under the control of the retroviral LTR (long terminal repeat) whereas a neomycin-resistance gene was under the control of an internal SV40 promotor. The vectors lacked the gag, pol, or env genes necessary for retroviral packaging in order to render them replication incompetent. These structural proteins were provided in *trans* by the retroviral packaging cell line PA317 [ATCC CRL 9078; Markowitz et al. (1988) J. Virol. 62:1120] or PG13 [ATCC CRL 10686; Miller et al. (1991) J. Virol. 65:2220]. For the bi-cistronic vector containing GM-CSF and B7-2 genes, the two genes were interposed with an internal ribosome entry site (IRES) derived from the Encephalomyocarditis Virus (EMCV) genome (pCITE-1, Novagen).

### (a) Construction Of pLSN

pLSN is a derivation of pLNL6, a retroviral vector approved for clinical use in the United States of America. To construct pLSN an intermediate vector, pLLL, was first constructed.

pLLL was constructed using pLNL6 (SEQ ID NO: 1) as a starting point. pLNL6 contains the MoMuLV promoter in the 3'LTR and the murine sarcoma virus (MSV) promoter in the 5' LTR. For ease in subsequent cloning steps, the few cloning sites and the internal SV-neo gene present in pLNL6 were removed and replaced with a synthetic polylinker to generate pLLL.

To construct pLLL, pLNL6 was digested with ClaI and *Bcl*I and the vector fragment was gel purified using GeneClean (Bio-101) according to the manufacturer's instructions. A doublestranded insert containing the polylinker site was constructed using the following two oligonucleotides: 5'-GATCTAAGCTTGCGGCCGCAGATCT CGAGCCATGGATCCTAGGCCTGATCACGCGTCGACTCGCGAT-3' (SEQ ID NO:2) and 5'-CGATCGCGAGTCGACGCGTGATCAGGCCTAGGATCCATGGCTCG AGATCTGCGGCCGCAAGCTTA-3' (SEQ ID NO:3). These oligonucleotides were annealed, kinased and ligated to the gel purified pLNL6 vector fragment and the ligation mixture was used to transform competent DH5α cells (BRL). Proper construction of pLLL was confirmed by restriction enzyme digestion of plasmid DNA prepared from ampicillin-resistance bacterial colonies as well as by DNA sequencing near the site of insertion of the polylinker.

To generate a vector containing a selectable marker which allows for the isolation of cells which have incorporated the vector DNA, pLSN was created. pLSN contains the *neo* gene under the transcriptional control of the SV40 enhancer/promoter. To create pLSN, a *Bam*HI/*Stu*I fragment containing SV40 enhancer/promoter was isolated from pLNSX [Miller, A.D.and Rosman, G.J. (1989) BioTechniques 7:980]. pLNSX and pLLL were digested with *Bam*HI and *Stu*I and the digestion products were gel purified. A small fragment of approximately 350 bp which contained the SV40 promoter from pLNSX was cloned into the pLLL vector. The final product, designated pLLL/SV40, was confirmed by restriction enzyme digestion using *Bam*HI and *Cla*I*.*

In order to insert a better translation initiation codon at the beginning of the *neo* gene, the *neo* gene was isolated from pLNSX using PCR. *Pfu* polymerase (Stratagene) was used to amplify the gene. This amplification was conducted in 5 µl of lOx *Pfu* reaction buffer, 0.5 µl of dNTP (15 mM), 0.5 mM of each of the following primers: 5'-AAGCTTGATCACCACCATGATTGAACAAGATGG-3' (SEQ ID NO:4) and 5'-CCGGATCCGTCGACCCCAGAGTCCCGCTCAGAAG-3' (SEQ ID NO:5), 0.5 µl of pLNSX (0.01 µg) and 38 µl of ddH₂O. These primers contain the modified translation initiation control sequence (-CCACCATG-), as this modification was found to greatly increase the strength of the *neo* gene in tissue culture cells [Kozak, M. (1986) Cell 44:283].

The mixture was heated at 95 °C for 5 min and 1 µl of *Pfu* polymerase was added. This reaction mixture was cycled through 30 cycles at 94°C for 1 min, 55°C for 1 min, and 72°C for 3 min. After amplification, the DNA comprising the *neo* gene was gel purified and ligated with the Bcl*I*-digested pLLL/SV40 vector to create pLSN. Confirmation of proper construction was made by restriction enzyme digestion as well as DNA sequencing.

### (b) Cloning Of The Human B7-2 And GM-CSF cDNAs

GM-CSF and B7-2 cDNAs were amplified from normal human lymphocyte RNA by RT-PCR as follows. In each case, the oligonucleotides used for PCR amplification comprised an optimized eukaryotic initiation sequence. For amplification of the GM-CSF cDNA, the following primer pair was used: 5' primer: 5'-CCCGGG AAGCTTCCACCATGTGGCTGCAGAGCCTG-3' (SEQ ID NO:6) and 3' primer: 5'-AATGGATCCTATCACTCCTGGACTGGCTC-3' (SEQ ID NO:7). The sequence of the human GM-CSF cDNA is available in GenBank accession no. M11220 and in SEQ ID NO:8. For amplification of the B7-2 cDNA, the following primer pair was used: 5' primer: 5'-TGTGGATCCACCATGGGACTGAGTAACATT-3' (SEQ ID NO:9) and 3' primer: 5'-TTTGGATCCTTAAAAACATGTATCACTTTTGTCGC-3' (SEQ ID NO:10). The sequence of the human B7-2 cDNA is available in GenBank accession no. U04343 and in SEQ ID NO:11. RT-PCR was carried out using an RT-PCR kit (BRL and Promega) according to the manufacturer's instructions.

The PCR amplified B7-2 gene was cloned directly (*i.e*., blunt-end ligation) into HincII digested pT7T318U (Pharmacia) to generate pT7T318U-B7-2. The PCR amplified GM-CSF gene was digested with *Hind*III and *Bam*HI and cloned into *Hind*III and *Bam*HI digested pBluescript KS (-) (Stratagene) to generate pBS-GM-CSF. Proper amplification and cloning of the B7-2 and GM-CSF open reading frames was confirmed by partial DNA sequencing and by restriction enzyme digestion.

### (c) Construction Of Retroviral Vectors Containing B7-2 GM-CSF And GFP Genes

The B7-2 and GM-CSF cDNAs were subcloned into the retroviral vector pLSN to generate pLSNB70 and pLSNGM1, respectively. To generate pLSNB70, pT7T318U-B7-2 was digested with *Bam*HI and the B7-2 fragment was gel purified and cloned into *Bgl*II-digested pLSN. To generate pLSNGM1, pBS-GM-CSF was digested with *Hind*III and *Bam*HI and the GM-CSF fragment was gel purified and cloned into *Hind*III- and *Bam*HI-digested pLSN.

In addition, cDNA for the reporter gene green fluorescent protein (GFP) was also inserted into the retroviral vector pLSN to generate pLSN-GFP. The GFP gene was PCR amplified using *Pfu* polymerase (Stratagene) from pGFP (Clontech) using the following primer pair: 5' primer: 5'-AAAAGCTTGGATCCACCATGAGTAAA GGA-3' (SEQ ID NO: 12) and 3' primer: 5'-AATCTAGATTACTATTTGTATAGTT CATCC-3' (SEQ ID NO: 13). The PCR amplified GFP gene was cloned directly into EcoRV-digested pBluescript KS(-) to generate pBS-GFP#1. pBS-GFP#1 was digested with *N*o*t*I and *XhoI* and the GFP fragment was gel purified and inserted into *Not*I*-* and XhoI-digested pLSN to generate pLSN-GFP.

Each of the resulting vectors contained a neomycin resistance gene driven by an internal SV40 promoter.

### (d) Construction Of A Bi-Cistronic Retroviral Vector Encoding B7-2 And GM-CSF

A bi-cistronic retroviral vector, pLSN-BG9, containing the B7-2 and GM-CSF genes separated by an IRES was constructed as follows. Three DNA fragments were gel purified: the EMCV IRES from XhoI- and *Hind*III-digested pGEM-IRES8 (described below); the GM-CSF gene from *Hind*III- and *Bam*HI-digested PCR product (section b); and the B7-2 gene from *Not*I-and XhoI-digested pLSNB70. The three purified fragments were mixed together and ligated into *Not*I*-* and *Bam*HI-digested pLSN to generate pLSN-BG9.

pGEM-IRES8 was constructed by isolating the EMCV IRES fragment from *Eco*RI*-* and MscI-digested pCITE-1 (Novagen) and inserting this fragment into *Eco*RI- and *Sma*I-digested pGEM-7Zf+ (Promega).

### (e) Generation Of Recombinant Retrovirus

Retroviral plasmid DNA was transfected into the packaging cell line PA317 [Miller, A.D. and Buttimore, C. (1986) Mol. Cell. Biol. 6:2895 and Miller, A.D. (1990) Hum. Gene Ther. 1:5] by lipofection as described (Robinson *et al., supra).* Briefly, PA317 cells were transfected with pLSNGM1, pLSNB70, pLSN-BG9, or pLSN-GFP using lipofectamine (Gibco/BRL). Lipofection was carried out according to the manufacturer's protocol.

PA317 cells were grown in DMEM containing 10% FBS and penicillin and streptomycin in an atmosphere containing 10% CO₂ at 37°C. Twenty hours prior to lipofection, PA317 cells were placed into a T25 flask (Falcon) at 50% confluency (approximately 1 x 10⁶ cells/flask). To transfect the cells, DNA (4 µg) was added to 300 µl serum-free DMEM lacking antibiotics in a microcentrifuge tube (Eppendorf), and mixed gently. In a 15 ml polycarbonate tube (Falcon), 300 µl serum-free DMEM and 12 µl of lipofectamine were mixed gently. The two solutions were combined by adding the DNA-containing solution dropwise into the lipofectamine tube, and the mixture was incubated at RT for 45 min. Following this incubation, 2 ml of serum-free DMEM was added and mixed gently. The cells were washed with serum-free DMEM and the DNA/lipofectamine mixture was gently added to the cells. The cells were incubated at 37°C in a 10% CO₂ incubator for 5 hr. After the 5 hr incubation, 2.5 ml of DMEM containing 20% FBS and antibiotics was added to the T25 flask and the cells were incubated overnight. Twenty hours after the 5 hr incubation, the medium was replaced with fresh DMEM containing 20% FBS and antibiotics. For vector titration, the medium was changed at 24 hr after the medium was replaced with fresh DMEM and virus was harvested 24 hr later. When cells were to be cloned (*i.e*., for the production of stable producer cell lines), the transfected PA317 cells were split at a 1:10 ratio into selective medium (*i.e*., DMEM containing 500 µg/ml G418 and 10% FBS).

Replication incompetent virus was harvested from supernatant of the transfected PA317 cultures 48 hrs after transfection of the PA317 cells and immediately frozen at -80°C for later use.

### Example 2 - Expression Of B7-2 And GM-CSF In The Human Glioblastoma Cell Line D54MG

Viral particles containing recombinant retroviral genomes encoding either GM-CSF, B7-2, B7-2/GM-CSF or GFP were used to transduce the human glioblastoma cell line D54MG.

### (a) Growth Of D54MG In Tissue Culture

The human glioblastoma cell line D54MG [obtained from Dr. D. Bigner, Duke University, Durham, NC; Bigner *et al.* (1981) J. Neuropathol. Exp. Neurol. 40:201] was cultured in Dulbucco's Modified Eagle's Media (DMEM) with 10% fetal bovine serum (Gibco), 0.2 units/ml penicillin-streptomycin solution (Sigma), and 0.2 mM glutamate at 37°C in a humidified atmosphere containing 5% CO₂.

### (b) Retroviral Transduction

Frozen virus stock was thawed at 37°C. Polybrene was added to the thawed virus solution at a final concentration of 4 µg/ml. Culture media was added to the virus solution to bring the final volume to 1.5 ml. D54MG cells in logarithmic growth phase in T25 flasks (approximately 1 x 10⁶ cells) were incubated in the virus supernatant at 37°C, 5% CO₂ for 3 hours. The same volume of media and polybrene without virus was used as control. After 3 hours, a further 3 ml of culture media was added to each flask and they were incubated overnight. Media was changed the next morning.

### (c) Selection Of Transduced Cells

Twenty-four hours after retroviral transduction, 500 µg/ml of the neomycin analog G418 was added to the culture media. Media was changed every two to three days until complete selection had taken place (*i.e*., all cells in the control flasks were dead). After selection, the cells were cultured in growth media containing reduced concentrations of G418 (250 µg/ml) and allowed to grow to confluence.

### (d) Analysis Of B7-2 Expression By Flow Cytometry

Transduced D54MG cells at approximately 70% confluence were harvested by scraping after room temperature incubation for 15 minutes in 0.02% EDTA in Phosphate-Buffered Saline (PBS). In aliquots of 10⁶ cells per 200 µl of immunofluorescence (IF) buffer (2% fetal calf serum, 0.02% sodium azide in PBS), samples were incubated on ice for 1 hour with 1 µg of RPE-conjugated monoclonal anti-human B7-2 antibody (Ancell) or 1 µg of RPE-conjugated isotype-control murine IgG₁ antibody (Pharmingen). Cells were washed four times in IF buffer, and fixed in 1% formalin in PBS. Samples were then read on a cytometer (B-D Flow Cytometer) using standard techniques.

### (e) Analysis Of GM-CSF Expression By ELISA

Transduced D54MG cells at approximately 70% confluence in T75 flasks were incubated in fresh media for 24 hours. After this period of incubation, media was harvested and centrifuged briefly to remove cells and debris. The cell number per flask was determined. GM-CSF levels in the harvested media was tested using a commercially available kit (Quantikine, R & D Systems) as per the manufacturer's instructions. The level of GM-CSF was converted to µg/10⁶ cells/24 hours based on the cell number in the flasks from which the media originated.

### (f) Expression Of B7-2 And GM-CSF In D54MG Cells

Flow cytometry studies showed that B7-2 was expressed on the surface of B7-2- and B7-2/GM-CSF-transduced D54MG cells but not on wild type or GM-CSF-transduced cells (1-2 orders of magnitude fluorescence shift compared to isotype controls). Flow cytometry of GFP-transduced D54MG cells without staining revealed mildly increased autofluorescence compared to wild type D54MG. Representative flow cytometry histograms are shown in Figs. 2A-E.

Figs 2A-E provide flow cytometry histograms for: wild type D54MG (2A), B7-2-transduced D54MG (2B), GM-CSF-transduced D54MG (2C), B7-2 and GM-CSF-transduced D54MG (2D), and GFP-transduced D54MG (2E). For Figs. 2A-2D, the histograms on the left represent D54MG cells stained with isotype matched control antibodies while the histograms on the right represent staining with monoclonal anti-human B7-2 antibodies. For Fig. 2E, the histogram on the left represents unstained wild type D54MG cells while the histogram on the right represents unstained GFP-transduced D54MG.

GM-CSF production was significant for GM-CSF-transduced (30 ng/10⁶ cells/day) and B7-2/GM-CSF-transduced (5 ng/10⁶ cells/day) cells but not for wild type or B7-2-transduced cells. Gene expression in the transduced D54MG cells is summarized in Table 1.

**TABLE 1**

| Therapeutic Gene Expression *In Vitro* In Wild Type And Transduced D54MG By ELISA (GM-CSF) Or Flow Cytometry (B7-2) | | | |
|---|---|---|---|
| Cell Line | Vector (Genes Transferred) | GM-CSF Production (ng/10⁶Cells/Day) | B7-2 Expression (Orders Of Magnitude Fluorescence Shift) |
| D54MG | None | 0.0 | 0 |
| D54MG | pLSNB70 (B7-2) | 0.0 | 2 |
| D54MG | PLSNGM1 (GM-CSF) | 30.0 | 0 |
| D54MG | pLSNBG9 (B7-2 and GM-CSF) | 5.0 | 1 |

The above results demonstrate that therapeutic gene expression in the D54MG human glioblastoma cell line was high after *in vitro* transduction with the pLSN-based retroviral vectors. Levels of GM-CSF production were comparable to those reported in other retrovirally transduced tumor cell lines [Dranoff et al. (1993) Proc. Natl. Acad. Sci. USA 90:3539 and Jaffee et al. (1993) Cancer Res. 53:2221]. Quantification of B7-2 expression is difficult using flow cytometry as expression is essentially a binary system (either present or absent on the cell surface). However, B7-2 molecules were clearly present on the surface of D54MG cells transduced with retroviral vectors containing the B7-2 gene and absent on wild type or GM-CSF-transduced cells. These results demonstrate that the above-described retroviral vectors provide simple, reliable tools for transferring GM-CSF and/or B7-2 genes into human glioblastoma cells *in vitro.*

### Examples 3- Tumor Growth Efficiency And Human PBL Reconstitution In SCID/beige And SCID/nod Mice

This example describes the reconstitution of SCID/nod and SCID/beige mice with human PBL and the engraftment of human tumor cells in these mouse strains.

### (a) Animals And Human PBL Reconstitution

For most experiments, four to five week old female C.B-17-SCID-beige mice were purchased from Taconic (Germantown, New York). For the first vaccination/challenge experiment, four to five week old female SCID/nod mice were obtained from Dr. L. Pilarski (Cross Cancer Institute, University of Alberta, Edmonton, Alberta). The mice were maintained in filtered cages in a virus free environment and received cotrimoxazole in their drinking water twice per week.

Hu-PBL-SCID mouse reconstitution was carried out as previously described [Zhang et al. (1996) Proc. Natl. Acad. Sci. USA 93:14720]. Briefly, each mouse was intraperitoneally (IP) injected with 2-3 x 10⁷ PBLs resuspended in 0.5 ml of Hanks' balanced salt solution. A near 100% success rate in reconstitution of SCID/bg mice was obtained when fresh PBLs were used. Five days to three weeks after reconstitution, mice were bled from the tail and the human Ig level was assessed by enzyme-linked immunosorbent assay (ELISA) using a monoclonal rabbit anti-human IgG/IgM antibody (Jackson Labs) and control human IgG (Sigma).

Preliminary results indicated that SCID/nod mice were not as reliably reconstituted with human peripheral blood lymphocytes as were SCID/bg mice. Unlike SCID/bg mice, reconstitution of SCID/nod mice with human PBLs appeared to vary both from PBL donor to PBL donor and from mouse to mouse. Furthermore, many hu-PBL-SCID/nod mice developed a disease characterized by cachexia, alopecia, and facial edema 3 to 6 weeks after reconstitution. This was not seen in unreconstituted SCID/nod mice or in SCID/bg mice with or without reconstitution. The etiology was unclear, though the possibilities of Graft vs. Host Disease and diabetes melitis were considered. Because of these difficulties, reconstituted SCID/bg mice are preferred for tumor growth and vaccination/challenge experiments.

### (b) Subcutaneous Tumor Growth Experiments

Five to seven week old SCID/bg mice were injected subcutaneously (SC) on the right flank with 2 x 10⁶ retrovirally-transduced and selected D54MG cells. Control mice were injected SC on the right flank with wild type D54MG cells. Six days post injection, mice were reconstituted via IP injection as described above with 2 x 10⁷ human peripheral blood lymphocytes isolated on a Histopaque gradient (Sigma) from the buffy coat layer of whole blood from healthy donors. Reconstitution was monitored by examination of sera (tail bleeds) for the presence of human Ig by ELISA. In all but one experiment, half of the mice from each group (transduced and untransduced) were left unreconstituted as controls. Tumor size was measured in three directions by calipers every 3 to 5 days. Comparison was made between transduced and untransduced tumor cells and between reconstituted and unreconstituted mice.

### (c) Statistical Analysis

Comparison between tumor sizes in different groups was performed using standard one-way analysis of variance (ANOVA).

### (d) Tumor Growth And Human PBL Reconstitution In SCID/beige And SCID/nod Mice

As described below, SCID/bg mice receiving human PBL via IP injection supported the growth of a human melanoma cell line with nearly 100% success and these animals demonstrated significant levels of human lymphocytes by flow cytometric analysis in spleen and peripheral blood 38 days post reconstitution. This example shows that both SCID/nod and SCID/bg mice supported the growth of wild type D54MG cells (human glioblastoma) subcutaneously with 100% efficiency (35/35) regardless of human lymphocyte reconstitution. D54MG tumors transduced with B7-2, GM-CSF, or both B7-2 and GM-CSF also grew with 100% efficiency in unreconstituted SCID/bg mice (4/4, 8/8, and 4/4 respectively). Growth of D54MG tumors transduced with B7-2 and GM-CSF was inhibited in human lymphocyte-reconstituted mice as detailed below.

Reconstitution with human PBLs was monitored by ELISA for the presence of human Ig using sera collected from the tail of the reconstituted mice. All SCID/bg mice receiving human PBLs (46/46) had significant levels (>100 µg/ml) of human Ig in serum within 14 days of reconstitution. However, 3 of 12 SCID/nod mice that received human PBLs failed to demonstrate human Ig on serial testing and were excluded from the study. Subsequent studies with SCID/nod mice revealed that the success of human PBL reconstitution also varied significantly from PBL donor to PBL donor. For these reasons, SCID/bg mice (not SCID/nod) were preferred for tumor growth and vaccination/challenge experiments.

### Example 4 - Growth Suppression Of B7-2 And GM-CSF-Transduced D54MG But Not Wild Type D54MG In Hu-PBL-SCID/bg Mice

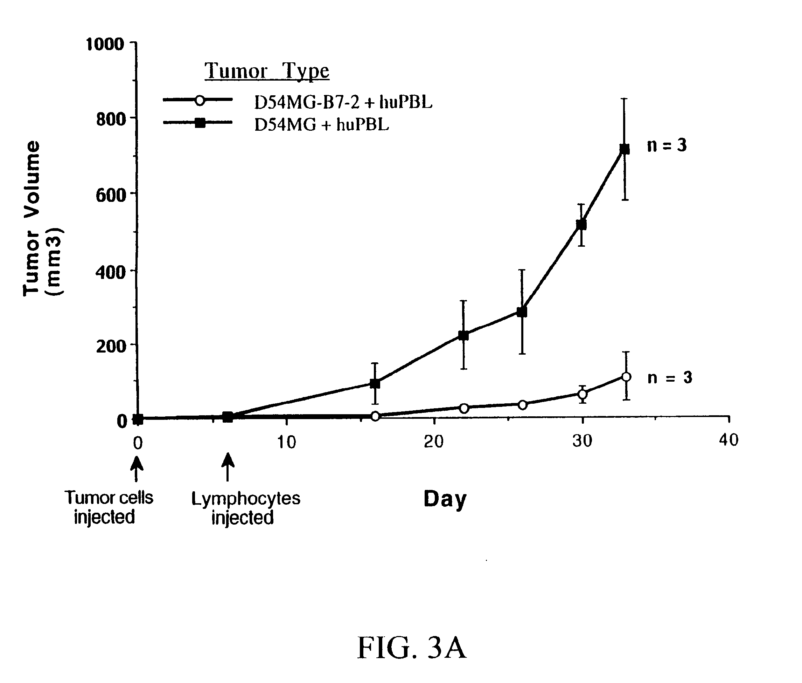

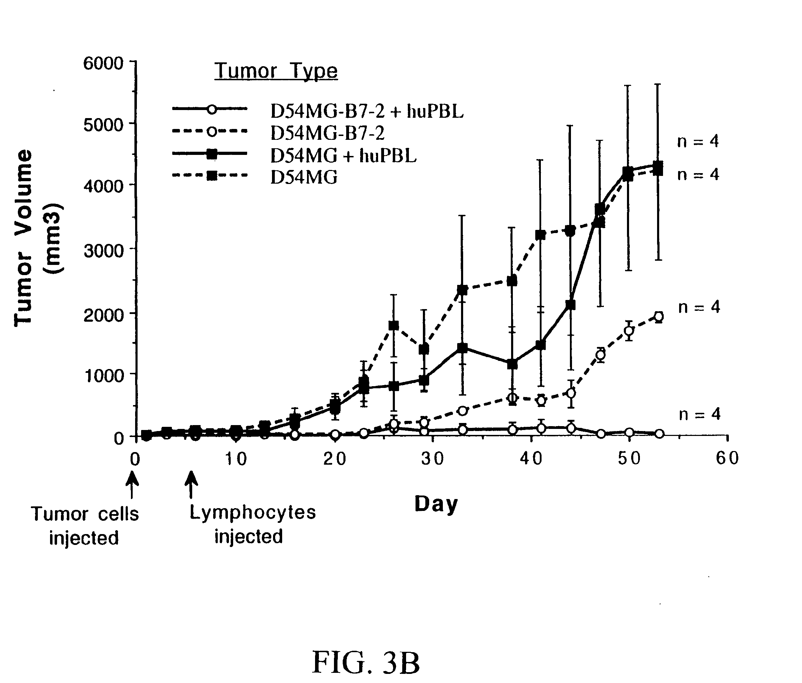

In two separate experiments, growth of D54MG transduced with B7-2 in human PBL reconstituted SCID/bg mice was markedly inhibited compared to untransduced and/or unreconstituted controls. These results are summarized in Figs. 3A and 3B.

For the results shown in Fig. 3, all mice received 2 x 10⁶ tumor cells (either D54MG or D54MG-B7-2) subcutaneously on the right flank on Day 0. Mice were injected IP with 2 x 10⁷ human PBL six days after tumor cell injection. Reconstitution was confirmed by detection of serum human Ig levels >100 µg/ml. In the first experiment (3A), all mice were reconstituted with human PBLs. In Fig. 3A, tumor volume (mm³) is plotted over time (days) for mice receiving untransduced D54MG cells (open circles; "D54MG") and D54MG cells transduced with pLSNB70 (solid squares; "D54MG-B7-2"). In the second experiment (3B), half the mice from both groups (D54MG and D54MG-B7-2) were left unreconstituted (hatched lines). In Fig. 3B, tumor volume (mm³) is plotted over time (days) for mice receiving untransduced D54MG cells (solid squares) and D54MG cells transduced with pLSNB70 (open triangles). In Fig. 3, arrows are used to indicate the times at which the tumor cells and lymphocytes were injected.

A standard test for statistical significance in subcutaneous tumor growth models is comparison of tumor volumes by ANOVA at a point approximately two-thirds along the growth curve [Gallagher et al. (1993) Tumor Immunology: A Practical Approach, IRL Press, Oxford, UK]. By this criteria, the mean tumor volume for B7-2-transduced tumors in human lymphocyte-reconstituted mice was significantly less than the mean tumor volume for wild type (*i.e.*, non-transduced) tumors by 22 days in the first experiment and by 35 days in the second experiment (p<0.05 in both). Interestingly, growth of D54MG-B7-2 tumors in unreconstituted mice was also mildly inhibited compared to wild type tumors (Fig. 3B). However, growth inhibition was much more marked in the reconstituted mice. These results suggests that, although a small portion of the growth inhibition seen for D54MG-B7-2 may be human lymphocyte-independent, the predominant effect is dependent on human lymphocytes.

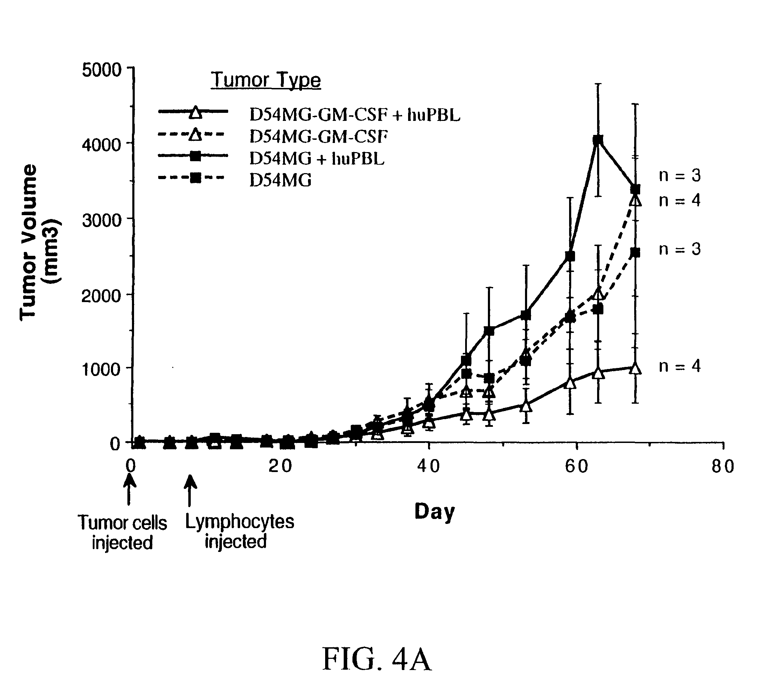

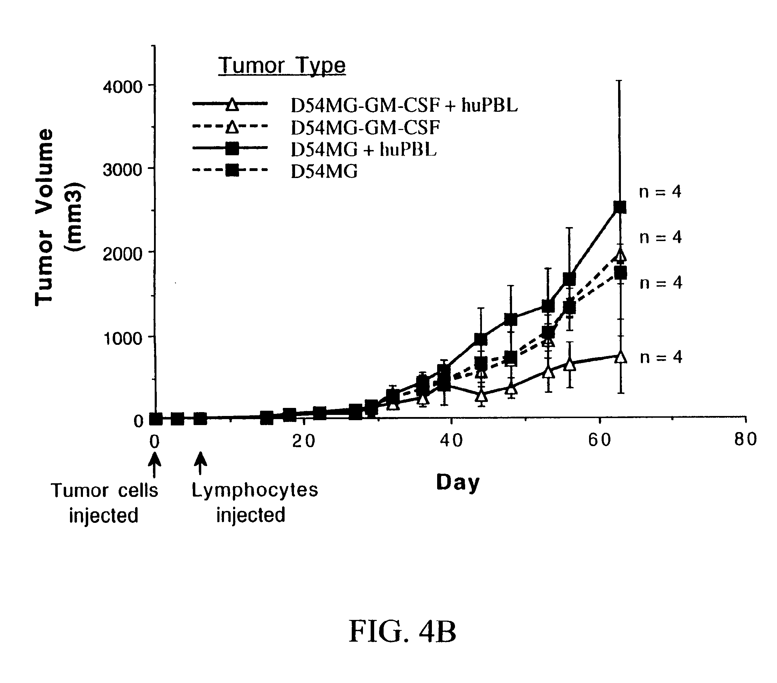

Growth of GM-CSF-transduced D54MG in human lymphocyte reconstituted mice was moderately inhibited compared to untransduced and/or unreconstituted controls in two separate experiments. These results are summarized in Figs. 4A and 4B.

For the results shown in Fig. 4, all mice received 2 x 10⁶ tumor cells (either D54MG or D54MG-GM-CSF) subcutaneously on the right flank on Day 0. Mice were injected IP with 2 x 10⁷ human PBL six days after tumor cell injection. Reconstitution was confirmed by detection of serum human Ig levels >100 µg/ml in ELISAs. In both experiments shown in Figs. 4A and 4B, half the mice from both groups (D54MG and D54MG-GM-CSF) were left unreconstituted (hatched lines). In Fig. 4A, tumor volume (mm³) is plotted over time (days) for mice receiving untransduced D54MG cells (solid squares; "D54MG") and DM54MG cells transduced with pLSNGM1 (open triangles; "D54MG-GM-CSF"). In Fig. 4B, tumor volume (mm³) is plotted over time (days) for mice receiving untransduced D54MG cells (solid squares) and D54MG cells transduced with pLSNGM1 (open triangles). In Fig. 4, arrows are used to indicate the times at which the tumor cells and lymphocytes were injected.

Neither of the experiments shown in Figs. 4A and 4B achieved statistical significance by ANOVA on their own. However, when the results from the two experiments were pooled, GM-CSF-transduced tumors in human PBL-reconstituted mice were significantly smaller than wild type by 55 days (p<0.001). No inhibition of D54MG-GM-CSF tumor growth was observed in unreconstituted mice.

These results demonstrate that transduction of D54MG tumor cells with recombinant retroviral vectors encoding either B7-2 or GM-CSF resulted in an inhibition of tumor growth in human PBL-reconstituted SCID/bg mice (Figs. 3 and 4). This demonstrates that the expression of either the human B7-2 gene or the human GM-CSF gene in glioblastoma cells overcomes local immunosuppression and results in a significant antitumor response. As demonstrated in the example below, the expression of both human B7-2 and GM-CSF genes in glioblastoma cells induces a systemic immune response that inhibits tumor growth at distant sites.

### Example 5 - Efficacy Of Immunization Of Hu-PBL-SCID/nod And Hu-PBL-SCID/bg Mice With Therapeutic Gene-Modified D54MG Tumor Cells

The ability to inhibit tumor growth in human PBL-reconstituted SCID mice by vaccination of the reconstituted mice prior to challenge with tumor cells was examined.

### (a) Vaccination/Challenge Experiments

In the first vaccination/challenge experiment, five to six week old female SCID/nod mice were reconstituted with 2 x 10⁷ PBL from healthy donors via IP injection. Reconstitution was monitored by examination of sera (tail bleeds) for the presence of human Ig by ELISA. Five days after reconstitution, mice were vaccinated via IP injection of 1 x 10⁵ irradiated (20,000 rad) D54MG cells (either wild type, GFP-transduced, or B7-2 and GM-CSF-transduced). Five days post-vaccination, all mice received SC injections of 1 x 10⁶ unirradiated wild type D54MG cells on the right flank. Tumor growth was measured as described above.

The second vaccination experiment was performed similarly, but in this instance, SCID/bg mice (Taconic) were used. In addition, the order of injections was slightly different. These mice first received SC injections of 1 x 10⁶ wild type D54MG cells on their right flanks. Ten days later, they were reconstituted with 2 x 10⁷ human PBL. Ten days after reconstitution, the mice were vaccinated with 1 x 10⁵ irradiated tumor cells (either wild type, GFP-transduced, or B7-2/GM-CSF-transduced).

### (b) Vaccination Of Hu-PBL-SCID/bg Mice With D54MG Cells Expressing Therapeutic Genes Induces A Systemic Immune Response

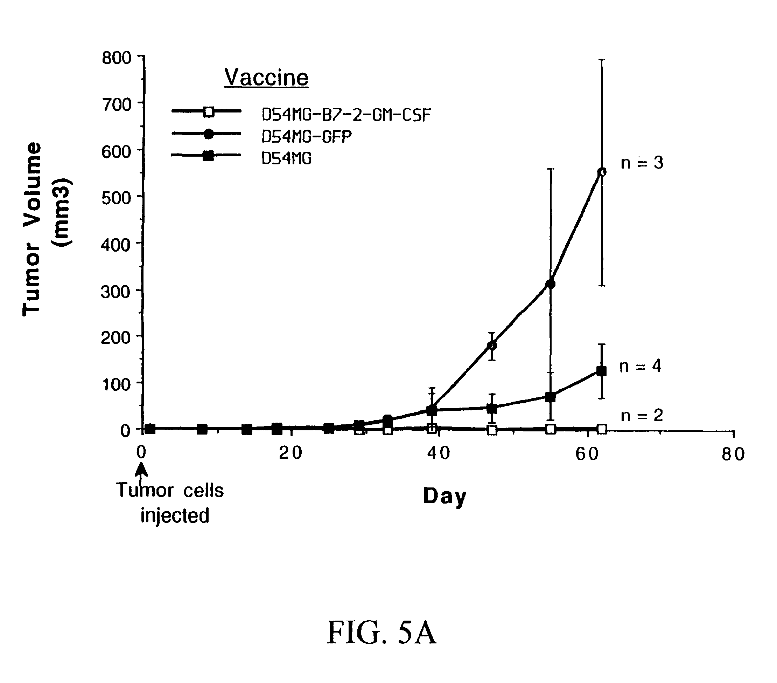

SCID/nod and SCID/bg mice were reconstituted, vaccinated and challenged with wild type D54MG cells as described above. As shown in Fig. 5A, growth of wild type D54MG tumors in human PBL-reconstituted SCID/nod mice which had been vaccinated with irradiated B7-2/GM-CSF-transduced D54MG was markedly inhibited compared to mice vaccinated with wild type or GFP-transduced D54MG. In Fig. 5A, tumor volume (mm³) is plotted over time (days) for mice vaccinated with either D54 cells transduced with pLSN-BG9 (open squares; "D54MG-B7-2-GM-CSF"), D54MG cells transduced with pLSN-GFP (solid circles; "D54MG-GFP") or untransduced D54MG cells (solid squares; "DM54MG"). In Fig. 5A, an arrow indicates the time at which the tumor cells were injected.

While the results shown in Fig. 5A demonstrate that the growth of wild type D54MG tumors was inhibited in Hu PBL-SCID/nod mice vaccinated with irradiated B7-2/GM-CSF-transduced D54MG cells. However, small sample sizes due to the exclusion of several unsuccessfully reconstituted mice (3/12) prevented this effect from reaching statistical significance. This problem was subsequently overcome by performing all remaining studies with reconstituted SCID/bg mice.

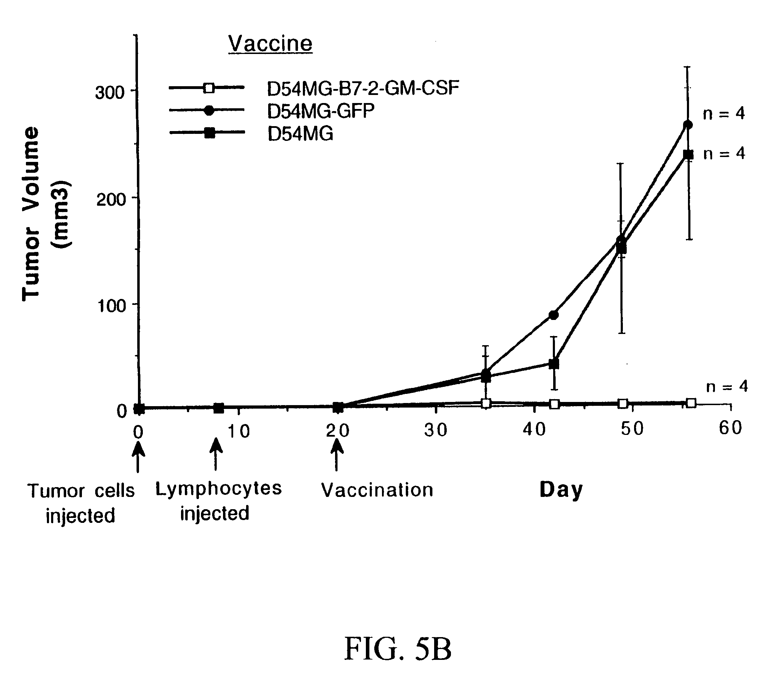

In a second vaccination experiment, wild type growth inhibition was seen in human PBL-reconstituted SCID/bg mice vaccinated with irradiated D54MG-B7-2/GM-CSF cells but not in mice vaccinated with either wild type D54MG or D54MG-GFP cells. In this second vaccination experiment all mice were successfully reconstituted with human PBLs as judged by ELISA (human Ig >100 µg/ml). The results are summarized in Fig. 5B. In Fig. 5B, tumor volume (mm³) is plotted over time (days) for mice vaccinated with either D54 cells transduced with pLSN-BG9 (open squares; "D54MG-B7-2-GM-CSF"), D54MG cells transduced with pLSN-GFP (solid circles; "D54MG-GFP") or untransduced D54MG cells (solid squares; "DM54MG"). In Fig. 5B, arrows are used to indicate the times at which the tumor cells and lymphocytes were injected as well as the time at which the vaccination was given.

Unlike the first vaccination study (Fig. 5A), in the second vaccination study wild type D54MG cells were injected subcutaneously into the flank 20 days prior to vaccination. At the time of vaccination, tumors were palpable (1-2 mm³) on all mice. After vaccination, tumor growth continued exponentially in mice vaccinated with wild type or GFP-transduced D54MG. However, in mice vaccinated with GM-CSFB7-2 transduced D54MG, tumor size increased slightly after vaccination (peaking at a mean of 8 mm³) and then regressed. Differences between the experimental and control groups achieved statistical significance by ANOVA (p<0.001) by day 42.

The above results demonstrate the inhibition of wild type tumor challenge growth in mice vaccinated with irradiated tumor cells transduced with retroviral vectors containing the B7-2 and GM-CSF genes and demonstrates that expression of these genes by glioblastoma cells induces a systemic immune response that inhibits tumor growth at distant sites.

It should also be noted that the present model, while an allogeneic system, has more similarities to autologous systems than may be immediately apparent. Unlike the classical immune-mediated rejection seen in allogeneic organ transplantation, this system is free of graft-origin "passenger lymphocytes." These lymphocytes are important in initiating allogeneic organ rejection responses by presenting antigen to host lymphocytes in the context of allogeneic Class II major histocompatibility complex (MHC) [Larsen et al. (1990) Annals of Surgery 212:308; Chandler and Passaro (1993) Archives of Surgery 128:279; Moller (1995) Transplantation Proc. 27:24]. The D54MG cell line expresses only Class I MHC and not Class II MHC *in vitro.* Although allogeneic Class I MHC molecules are expressed by the D54MG cells in this model, the absence of graft-origin Class II MHC-positive cells in the tumor renders this model more like an autologous system.

The use of the hu-PBL-SCID mouse model and a human glioblastoma tumor cell line has demonstrated the therapeutic utility and feasibility of *in vivo* immunogene therapy using GM-CSF or B7-2 genes (alone or in combination). The hu-PBL-SCID model provides a simple and powerful method to analyze human lymphocyte responses to human tumors *in vivo.* Early cancer immunotherapy studies using hu-PBL-SCID mice were limited by the influence of residual murine NK lymphocyte activity [Reddy et al. (1987) Cancer Res. 47:2456; Hill et al. (1991) FASEB J. 5:A965; Mueller et al. (1991) Cancer Res. 51:2193; Zhai et al. (1992) Cancer Immunol. Immunother. 35:237]. This problem has been overcome by the use of the SCID/bg and the SCID/nod mouse strains that are deficient in NK cells [Croy and Chapeau (1990) J. Reprod. Fert. 88:231; MacDougal et al. (1990) Cell. Immunol. 130:106; Prochazka et al. (1992) Proc. Natl. Acad. Sci. USA 89:3290; Mosier et al. (1993) J. Exp. Med. 177:191; Malkovska et al. (1994) Clin. Exp. Immunol. 96:158].

In conclusion, the retroviral gene therapy vectors provided herein demonstrated efficient bi-cistronic gene transfer (B7-2 and GM-CSF) to human glioblastoma cells *in vitro.* In an *in vivo* allogeneic human tumor/lymphocyte system, glioblastoma cells that express these genes result in lymphocyte-mediated responses that inhibit tumor growth locally and at distant sites. These results demonstrate the feasibility of immunogene therapy for glioblastoma multiforme using B7-2 and GM-CSF genes.

### Example 6 - Transduction And Expression Of The B7-2 Gene In Established Tumor Cell Lines

To determine whether most of the human tumor cells are susceptible to retroviral transduction, and whether the T-cell costimulator gene B7-2 can be expressed in different tumors, a series of different types of human tumors were transduced with the B7-2 vector. Conditions for transduction were as described in Ex. 2. Twenty-four hrs after transduction, the cells were cultured in media containing different concentrations of G418. Results of this study are shown in Table 2.

**TABLE 2**

| Transduction Of Tumor Cell Lines With pLSNB70 | | |
|---|---|---|
| Cell Type | Medium | Transduction Rate* / µg/ml (G418) |
| HeLa cervical epithelial carcinoma | DMEM | +++ / 500-1000 |
| SW480 colon adenocarcinoma | DMEM or L15 | +++++ / 750-1000 |
| HT-29 colon adenocarcinoma | McCoy's | +++++ / 500 |
| U87 MG/Glioblastoma-Astrocytoma | MEM+NaPy+NEa.a. or DMEM | +++++ / 1000 |
| SK-N-MC neuroblastoma | MEM++NaPy+NEa.a | +++ / 600 |
| SK-N-SH / neuroblastoma | <MEM+NaPy+NEa.a. | ++ |
| A-431 epidermoid carcinoma | DMEM | ++ / 250 |
| RD embryonal rhabdomyosarcoma | MEM | +++ / 250 |
| HepG2 hepatoma | DMEM | +++++ / 750-1000 |
| Huh7 hepatoma | DMEM | ++ / 500-750 |
| PC3 prostate tumor | F12/DMEM | - / 250 no survivors |
| DU 145 prostate tumor | MEM | ++++/600 |
| A375 melanoma | DMEM | ++++ / 1000 |
| SK-Me-1 melanoma | MEM (suspension cells) | /300 |

| | | |
|---|---|---|
| 1+: <10 scc; 2+: >30 scc; 3+: >50 scc; 4+: >80 scc; 5+: >100 scc; and scc: single cell clone. MEM (Minimal Esstential Medium); NaPy (sodium pyruvate); NE a.a. (non-essential amino acids). | | |

The results shown in Table 2 demonstrate that most of the established human tumor cells were efficiently transduced by the pLSNB70 virus. Some of the tumor cells are more resistant to G418 than others. The transduction rate was determined by counting the number of G418-resistant cell colonies. Expression of the B7-2 gene was demonstrated by immunohistochemical staining using an anti-B7-2 monoclonal antibody. Except for the few cell lines that were sensitive to low levels of G418 (*e.g*., PC3), most of the tumor cell lines tested were efficiently transduced with the B7-2 gene, and selected by G418 within two weeks.

### Example 7 - Transduction And Expression Of B7-2 In Primary Tumor Cells

Retroviral transduction of established cell lines is generally more efficient than transduction of primary tumor cells. For *in vivo* or *ex vivo* gene transduction, however, the target cells are fresh (*i.e*., primary) tissues or tumors. It is thus important to demonstrate B7-2 transduction in freshly isolated primary tumor cells. To this end, several tumor specimens from surgery or biopsy were propagated in culture for less than 5 passages and transduced with the pLSNB70 virus as described in Ex. 2. After transduction, the cultures were selected with G418. The results are summarized in Table 3.

**TABLE 3**

| Retroviral Transduction Of Primary Tumor Cultures | | |
|---|---|---|
| Cell type | Medium | Transduction / Rate*/µg/ml |
| E81 (p11) | F12/DME | ++/250 |
| E82 (p3) | F12/DME | ++++ / 250 |
| E81 (p11) | F12/DME | + / 250 |
| Hepatoma (PC) | RPMI; F12/DME | - / 500 |
| Melanoma (DD) | RPMI | ++ / 250 |
| Melanoma (DJ) | RPMI | ++ / 250 |
| Adenocarcinoma (BP) | RPMI | +++ / 250 |
| Adenocarcinoma (LJ) | RPMI | +++ / 250 |

| | | |
|---|---|---|
| • (1+: <10 scc; 2+: >30 scc; 3+: >50 scc; 4+: >80 scc; 5+: > 100 scc;scc, single cell clone). | | |

As shown in Table 3, primary tumor cultures were found to be sensitive to low concentrations of G418 (approximately 250 µg/ml G418). Nevertheless, most of the transduced cells were selected within 10 days. The results shown in Table 3 illustrate the successful transduction of three primary gliomas, two melanomas and two adenocarcinomas with pLSNB70. B7-2 expression was also confirmed by immunohistochemical staining using a mouse anti-B7-2 antibody as the primary antibody and a FITC-labeled goat anti-mouse antibody as the secondary antibody. The majority of the selected cells (*i.e*, the transduced tumor cells that survived growth in the presence of G418) showed positive surface staining for B7-2.

### Example 8 - Retroviral Transduction Of GM-CSF In Established And Primary Tumor Cells

In animal studies, a high level of GM-CSF expression has been shown to be related to its therapeutic efficacy [Jaffee et al. (1993) Cancer Res. 53:2221 and Dranoff et al. (1993) Proc. Natl. Acad. Sci. USA 90:3539]. Two different retroviral vectors were constructed that contained the human GM-CSF cDNA to examine the level of GM-CSF expressed in transduced tumor cells. The first vector pLSN, which contains the wild type MoMLV LTR, was described above in Ex. 1 as was the construction of pLSNGM1. The second vector, pLGCTSN (ATCC 97803; Robinson *et al., supra),* contains a modified LTR in which the MoMLV U3 region is replaced by the CMV-IE enhancer/promoter and the HIV TATA and TAR elements which leads to an increased level of expression in human cells; in addition, pLGCTSN contains an extended packaging signal and a 3' splice acceptor sequence from MoMLV. The human GM-CSF cDNA was inserted into pLGCTSN to create pLGCTSN-GM1 as follows. The GM-CSF gene was released from pBS-GM-CSF (Ex. 1) by digestion with *Hin*dIII and *Bam*HI and this fragment was inserted into *Hin*dIII- and *Bam*HI-digested pLGCTSN to generate pLGCTSN-GM1.

A human melanoma cell line (SK-MEL-1; ATCC HTB 67) and the primary adenocarcinoma (LJ) culture ["AD 1 (LJ)"] were transduced with retroviruses derived from either pLSNGM1, pLGCTSN-GM, pLSNBG9 or pLSNB70, and selected with G418. The expression of GM-CSF was determined by ELISA using culture supernatants collected from the G418-selected cultures. Table 4 summarizes the amount of GM-CSF (ng/10⁶ cells/24 hr) present in the culture supernatants.

**TABLE 4**

| ELISA Of GM-CSF Expression In Established And Fresh Tumor Cell Cultures | | |
|---|---|---|
| Vector | SK-MEL-1 | AD1(LJ) |
| Control (pLSNB70) | 0.1 | 0.03 |
| pLSNGM1 | 9 | 4 |
| pLGCTSN-GM | 9 | 8 |
| pLSNBG9 | 9 | 0.65 |

The virus derived from pLSNB70 (encodes B7-2) was used as a negative control for the expression of GM-CSF in the transduced cells; pLSNB70-transduced melanoma cells expressed GM-CSF at <0.1 ng/ml/10⁶ cells in 24 h which was close to the background level of the ELISA. The pLSNGM1- and the pLGCTSN-GM-transduced cells expressed 9 ng/ml/10⁶cells of GM-CSF in 24 hr. This study used an established human melanoma cell line (SK-MEL-1) and the level of GM-CSF expression was determined 3 months after cells were selected by G418.

A similar study was performed using a primary human adenocarcinoma culture AD1(LJ). The primary culture was prepared as follows. The tumor tissue was surgically removed, placed in HBSS on ice immediately after surgery and transferred to a biosafety hood. Ten milliliters of HBSS was placed in a sterile petri dish and the tumor tissue was added. Using forceps and a scalpel, normal tissue surrounding the tumor was removed and the tumor was transferred to a second petri dish containing 5-10 ml HBSS and the tumor was minced. Ten ml of a suspension containing the minced tumor was transferred to a 15 ml tube and the cells were collected by centrifugation at 100xg for 8 min. at room temp. The cells were resuspended in 2 ml of DMEM containing 20% FCS. The resuspended cells (2-3 ml) were transferred to a 50 ml tube and the following enzymes were added: 1 mg/ml collagenase type V, 10 µg/ml hyaluronidase type V, 300 U/ml DNase type IV and 10 µg/ml gentamycin sulfate. The cells suspension was then incubated at 37°C with shaking for 60 min. The cells were then plated in tissue culture flasks containing DMEM containing 10% FCS, 10 µg/ml gentamycin sulfate, penicillin/streptomycin/glutamine solution (Gibco/BRL) and 1.25 µg/ml amphotericin (growth medium) and grown overnight in an incubator containing 5%CO2 at 37°C. On the second day, non-adherent cells were removed by washing the flasks with 3 ml growth medium. The medium was changed every 3-7 days depending upon the growth rate of the cells. The cells were transduced with the pLSNB70 virus and grown in the presence of G418 as described above.

As shown in Table 4, the transduced and selected primary human tumor cells [AD1(LJ)] produced GM-CSF in the range of 1-8 ng/ml/10⁶ cells/24 hr (GM-CSF expression was determined 2-3 weeks after the cells were selected using G418). Thus, the un-modified retroviral vector pLSN is capable of producing high levels of GM-CSF in primary human tumor cells. The level of GM-CSF expression may be cell-type dependent, and further modification of the vector (*e.g*., use of cell-type- or tissue-specific enhancers and/or promoters) may further increase the level of expression in specific tumor cells.

### Example 9 - Bi-Cistronic Expression Of B7-2 And GM-CSF In Established And Primary Tumor Cells

To express both the B7-2 and GM-CSF therapeutic genes simultaneously in tumor cells, the bi-cistronic retroviral vector pLSNBG9 (Ex. 1) was employed. The plasmid DNA was transfected into PA317 cells and virus was harvested and used for transduction as described in Ex. 1. Both the established melanoma cell line SK-MEL-1 and the primary adenocarcinoma cell culture AD1(LJ) were transduced and selected by G418. After selection (typically 2 weeks after applying G418), the expression of GM-CSF and B7-2 in these two cell types was determined by ELISA (Table 4) and flow cytometry, respectively. Table 4 shows that the level of GM-CSF expression for the bi-cistronic vector was similar to the mono-cistronic vector pLSN-GM in SK-MEL-1 cells but was ten times less than the mono-cistronic vector in the primary AD1(LJ) culture. The FACS analysis indicated that the mono-cistronic (pLSNB70) and bi-cistronic (pLSNBG9) vectors expressed B7-2 in both cell types. These results demonstrate that both B7-2 and GM-CSF can be expressed simultaneously following transduction of established and primary tumor cultures with virus derived from the bi-cistronic pLSNBG9 vector.

### Example 10 - Construction Of Expression Vectors For The High Level Expression Of Therapeutic Genes

The promoter strength of the retroviral vector determines the expression level of the transduced gene in eukaryotic cells. To increase the level of gene expression, we have modified the MLV vector and generated a series of vectors with enhanced promoter strength (Robinson *et al., supra*)*.* To further improve the promoter strength, the strength of several promoters was examined in the human tumor cell line, HeLa (ATCC CCL 2), using a reporter CAT assay. Plasmids in which the CAT gene was placed under the transcriptional control of either the CMV-IE promoter/enhancer, the EF1α promoter/enhancer with or without intron 1, the MCT promoter (the modified MoMLV LTR present in pLGCTSN), and the HIV LTR (a number of these CAT constructs are described in Robinson *et al., supra*)*.* The plasmids were transfected into HeLa cells and 48 hr later cell lysates were prepared and assayed for CAT activity as described (Robinson *et al., supra).* Constructs containing plasmids comprising the HIV-1 TAR element *(i.e.,* the HIV LTR and the pMCT promoters) were also co-transfected along with a *tat* expression construct. The results are summarized in Table 5. In Table 5, the promoter activity is expressed relative to the activity of the CMV-IE promoter. In Table 5, the following abbreviations are used: EF1α+intron (the 1.442 kb EF1α enhancer/promoter element comprising intron 1); EF1α-intron (a 475 bp fragment containing the EF1α enhancer/promoter corresponding to map units 125 to 600 of the human EF1α gene) HIV+Tat (the pHIV-1/LTR-cat construct co-transfected with the *tat* expression construct); MCT (pMCT-cat); and MCT+Tat (pMCT-cat co-transfected with the *tat* expression construct).

**TABLE 5**

| Promoter | Relative Activity |
|---|---|
| CMV-IE | 1.0 |
| EF1α+Intron | 2.16 |
| EF1α-Intron | 0.02 |
| HIV+Tat | 0.13 |
| MCT | 0.32 |
| MCT+Tat | 1.2 |

The results shown in Table 5 indicate that the human EFIα promoter plus its intron has the strongest activity compared with all other promoters tested. Thus, the EF1α promoter may be used to drive the expression of therapeutic genes in tumor cells. Retroviral and plasmid constructs in which the EF1α+intron promoter/enhancer is used to drive the expression of therapeutic genes is described below.

### (a) Construction Of A Plasmid Expression Vector Containing The EF1α Enhancer/Promoter

The human EF1α enhancer/promoter is abundantly transcribed in a very broad range of cell types including L929, HeLa, CHU-2 and COS cells [Uetsuki, T. et al., J. Biol. Chem., 264:5791 (1989) and Mizushima, S. and Nagata, S., Nuc. Acids. Res., 18:5322 (1990)]. A 1.442 kb fragment containing the human EF1α enhancer/promoter and a splice donor and acceptor from the human EF1α gene was isolated from human genomic DNA as follows. The 1.442 kb fragment corresponds to map units 125 to 1567 in the human elongation factor 1α gene (SEQ ID NO:14).

Genomic DNA was isolated from the MOU cell line (GM 08605, NIGMS Human Genetic Mutant Cell Repository, Camden, NJ) using standard techniques [Sambrook, J. et al., Molecular Cloning: A Laboratory Manual, 2nd. ed., Cold Spring Harbor Laboratory Press, (1989) pp. 9.16-9.23]. Two synthetic oligonucleotide primers were used to prime the polymerase chain reaction (PCR) for the isolation of the 1.442 kb fragment containing the human EF1α enhancer/promoter. U.S. Patent Nos. 4,683,195, 4,683,202 and 4,965,188 cover PCR methodology and are incorporated by reference.

The 5' primer, designated HEF1αL5, contains the following sequence: 5'-AAGCTTTGGAGCTAAGCCAGCAAT-3' (SEQ ID NO:15). The HEF1αL5 primer generates a *Hin*dIII site at the 5' end of the 1.442 kb fragment. The 3' primer, designated HEF1αL3B, contains the following sequence: 5'-TCTAGAGTTTTCACGA CACCTGA-3' (SEQ ID NO:16). The HEF1αL3B primer generates *a Xba* I site at the 3' end of the 1.442 kb fragment. PCR conditions were as reported in Saiki, R.K. et al., Science 239:487 (1988). Briefly, 10 µg MOU genomic DNA and 1 µM final concentration of each primer were used in a 400 µl PCR reaction. Reaction conditions were 94°C for 1 minute, 60°C for 1 minute, 72°C for 1.5 minutes, 30 cycles. *Taq* DNA polymerase was obtained from Perkin-Elmer Cetus; the reaction buffer used was that recommended by the manufacturer. The PCR reaction products were electrophoresed on a low melting agarose the 1.442 kb fragment was gel purified and digested with *Hin*dIII and *Xba*I. The *Hin*dIIII/*Xba*I fragment was then inserted into pSSD5 (described below) to generate pHEF1αBSD5.

pSSD5 was constructed by digestion of the plasmid pLI [Okayama, H. and Berg, P., Mol. Cell. Biol., 3:280 (1983)] with *Pst*I and *Eco*RI. Synthetic oligonucleotides (Operon) were ligated onto the *Pst*I and *Ec*oRI ends of pLI to generate the polylinker of pSSD5 (the SD5 polylinker). The sequences of the oligonucleotide pair used to create the polylinker are: SD5A 5'-TCTAGAGCGGCCG CGGAGGCCGAATTCG-3' (SEQ ID NO:17) and SD5B 5'- GATCCGAATTCGGCC TCCGCGGCCGCTCTAGATGCA-3' (SEQ ID NO:18). The ligation of this oligonucleotide pair into pLI destroyed the *Pst*I site. Following the addition of the polylinker, the plasmid was digested with *Hin*dIII and partially digested with *Bam*HI. The 572 bp *Hin*dIII*lBam*HI fragment containing the SV40 enhancer/promoter, the 16S splice junction and the SD5 polylinker was isolated by electrophoresis of the digestion products on a low melting temperature agarose gel (SeaPlaque, FMC BioProducts, Rockland, ME). The 572 bp fragment was cut out of the gel and the agarose was removed by digestion with β-Agarase I (New England Biolabs) followed by isopropanol precipitation according to the manufacturer's directions.

The 572 bp fragment was inserted into the plasmid pcDV1 [Okayama and Berg, *supra*] as follows: pcDV1 was digested with *Hin*dIII and *Bam*HI and the 2.57 kb fragment containing the SV40 poly A sequences and the pBR322 backbone was ligated to the 572 bp fragment containing the SV40 enhancer/promoter, 16S splice junction and polylinker. The resulting plasmid was named pSSD.

The 671 bp *Bam*HI*lPst*I fragment containing the SV40 poly A sequences (SV40 map units 2533 to 3204) was removed from SV40 DNA and cloned into pUC19 digested with *Bam*HI and *Pst*I. The resulting plasmid was then digested with *Bc*/I (corresponds to SV40 map unit 2770). The ends were treated with the Klenow enzyme and dNTPs to create blunt ends. Unphosphorylated *Pvu*II linkers (New England Biolabs) were ligated to the blunted ends and the plasmid was circularized to create pUCSSD. The SV40 poly A sequences can be removed from pUCSSD as a *Bam*HI*lPvu*II fragment.

pSSD5 was constructed by ligating together the following three fragments: 1) the 1873 bp *Ssp*I/*Pvu*II fragment from pUC19; this provides the plasmid backbone; 2) the 796 bp fragment containing the SV40 enhancer/promoter and 16S splice junction and the polylinker from pSSD; this fragment was obtained by digestion of pSSD with *Ssp*I and partial digestion with *Bam*HI followed by isolation on low melting agarose and recovery as described above; and 3) the 245 bp *Bam*HI*lPvu*II fragment from pUCSSD (this fragment contains the SV40 poly A sequences). The three fragments were mixed together and ligated using T4 DNA ligase to create pSSD5. The polylinker of pSSD5 contains the following restriction sites: *Xba*I, *Not*I, *Sfi*I, *Sac*II and *Eco*RI*.*

### (b) Construction Of pHEF-B70

A plasmid, pHEF-B70, in which the EF1α enhancer/promoter is used to drive the expression of the human B7-2 gene was constructed as follows. The B7-2 gene was removed from pLCTSNB70 (described below) by digestion with *Bam*HI and the isolated B7-2 fragment was ligated into *Bam*HI-digested pHEF1αBSD5 to generate pHEF-B70.

pLCTSNB70 was constructed by ligating the B7-2 gene released from pT7T318U-B7-2 (Ex. 1) by *Bam*HI digestion into *Bam*HI-digested pLCTSN (ATCC No. 97802; Robinson *et al., supra).*

### (c) Construction Of pHEF-IL-12

In the plasmid pHEF-IL-12, the IL-12A and IL-12B genes are under the transcriptional control of the EF1α enhancer/promoter.

### (i) Cloning Of The Human IL-12 cDNA

RNA was isolated from activated NC37 B cells (ATCC CCL 214) and the IL-12A and IL-12B cDNAs were isolated by RT-PCR as described in Ex. 1. The following primers were employed for the amplification of the IL-12A cDNA: 5'-GAA GATCTGCGGCCGCCACCATGTGGCCCCCTGGGTCAGC-3' (SEQ ID NO:19; optimized initiation sequence underlined) and 5'-CCTCTCGAGTTAGGAAGCATTCA GATAGC-3' (SEQ ID NO:20). The PCR product was cloned directly into *Eco*RV-digested pBluescript KS(-) to generate pKS-IL-12A. The IL-12A coding region is provided in SEQ ID NO:21.

The following primers were employed for the amplification of the IL-12B cDNA: 5'-AAAGAGCTCCACCATGTGTCACCAGCAGTTGGTC-3' (SEQ ID NO:22; optimized initiation sequence underlined) and 5'-AAGGATCCTAACTGCAGG GCACAGATGC-3' (SEQ ID NO:23). The PCR product was cloned directly into EcoRV-digested pBluescript KS(-) to generate pKS-IL-12B; the IL-12B coding region is provided in SEQ ID NO:24. Proper construction of both IL-12 constructs was verified by restriction enzyme digestion.

### (ii) Construction Of pLSN-IL-12

pLSN-12 is a retroviral vector containing a IL-12A-IRES-IL-12B bi-cistron. To construct pLSN-IL-12, the following four fragments were gel purified: the IL-12A gene released from pKS-IL-12A by digestion with *Cla*I and *Psp*AI; the IL-12B gene released from pKS-IL-12B by digestion with *Not*I and *Xho*I*;* the EMCV IRES was released from pGEM-IRES8 by digestion with *Xho*I and *Cla*I; and a pLSN vector backbone containing a *Psp*AI site generated by removing the HIV vpr gene from pLSNvpr (described below) by digestion with *Mlu*I and *Psp*AI*.* These four fragments were combined and ligated with *Not*I-and *Mlu*I-digested pLSNvpr to generate pLSN-IL-12.

pLSNvpr was constructed by digestion of pBS-vpr with *Not*I and *Xho*I and the vpr gene fragment was ligated with *Not*I- and *Xho*I-digested pLSN. The vpr gene was PCR amplified from pNL4-3 [Adachi et al. (1986) J. Virol. 59:284] and inserted into pBluescript KS(-) to generate pBS-vpr.

### (iii) Construction Of pHEF-IL-12

To construct pHEF-IL-12, the following three fragments were isolated: the IL-12A gene was released from pKS-12A by digestion with *Cla*I and *Psp*AI; the IL-12B gene released from pKS-IL-12B by digestion with *Not*I and *Xho*I*;* the EMCV IRES was released from pGEM-IRES8 by digestion with *Xho*I and *Cla*I. These fragments were cloned into *Not*I*-* and *Psp*AI-digested pHEFvpr. pHEFvpr was constructed by ligation of a 360 bp *Bam*HI fragment isolated from pLSNvpr with pHEF1αBSD5 that had been digested with *Bam*HI and treated with alkaline phosphatase.

### (d) Construction Of pHEF-IL-12-GM-CSF

pHEF-IL-12-GM-CSF contains a IL-12A-EMCV IRES-IL-12B- poliovirus IRES-GM-CSF tri-cistron under the transcriptional control of the EF1α enhancer/promoter. To construct pHEF-IL-12-GM-CSF, the following four fragments were gel purified: a 2373 bp *Nde*I*-Psp*AI fragment containing the IL-12B-EMCV IRES-IL-12A cistron isolated from pHEF-IL12; a 1320 bp *Nde*I*-Sph*I fragment containing the EF1α intron isolated from pHEF-IL-12; a 735 bp *Psp*AI*-Xho*I fragment containing the poliovirus IRES from pKS-P2 (an equivalent DNA fragment is generated by PCR amplification of the IRES located within the first ~730 bp of the poliovirus genome [LaMonica et al. (1986) J. Virol. 57:515] using primers that will generate *Psp*AI and *Xho*I sites).

### (e) Construction_Of pHEF-GM-CSF

pHEF-GM-CSF was derived from pHEF-IL-12-GM-CSF by complete digestion with *Sma*I and partial digestion with *Hinc*II. The resulting approximately 4409 bp fragment was gel purified and self-ligated to produce pHEF-GM-CSF.

### (f) Construction Of pHEF-BG

A plasmid, pHEF-BG, in which the 1.442 kb EF1α enhancer/promoter is used to drive the expression of both the human B7-2 and GM-CSF cDNAs (a bi-cistronic construct) is constructed as follows. The B7-2-IRES-GM-CSF bi-cistronic fragment was removed from pLSN-BG9 (Ex. 1) by digestion with *Not*I and *Bam*HI and the gel purified fragment was inserted into *Not*I*-* and *Bam*HI-digested pHEF1αBSD5 to generate pHEF-BG.

### (g) Construction Of The pLSN-IL-12-GM-CSF Retroviral Vector

pLSN-IL-12-GM-CSF provides a retroviral vector in which the MoMLV LTR is used to drive the expression of both the human IL-12 gene and GM-CSF cDNAs. pLSN-IL-12-GM-CSF was constructed by ligation of the following four fragments into *Bam*HI-digested pLSN-GFP: the 1489 bp MoMLV vector fragment derived from *Sac*II*-Xba*I-digested pLSN-GFP; the 1680 bp IL-12B-IRES containing fragment from *Xba*I*-Not*I-digested pHEF-IL-12; the 760 bp IL-12A containing fragment from *Not*I*-PspA*I digested pHEF-IL-12; and the 1252 bp GM-CSF containing fragment from *Psp*AI*-Bam*HI digested pHEF-IL-12-GM-CSF.

### (h) Construction Of The pLCTSN_pA-BG-EF Retroviral Vector

pLCTSN-pA-BG-EF contains a cistron comprising the EF1α enhancer/promoter, B7-2 and GM-CSF cDNAs and SV40 polyadenylation signal inserted in an inverted orientation (relative to transcription from the 5' LTR). This vector was constructed by ligation of the following three fragments with *Hin*dIII and *Sal*I digested pLCTSN vector: EFIα enhancer/promoter from *Sal*I*-Not*I digested pHEF, B7-2/IRES/GM-CSF cistron from *Sal*I*-Not*I digested pLCTSN-BG, and SV40 polyA signal from *Bgl*II-*Hin*dIII digested pSP72SVpA.

pLCTSN-BG was constructed by gel-purifying the following three DNA fragments: the B7-2 cDNA from pLSNB70 cut with *Not*I*-Xho*I*,* the EMCV IRES from pGEM-IRES8 cut with *Xho*I*-Hin*dIII (*Hin*dIII partial), and the GM-CSF cDNA from PCR amplified and *Hin*dIII*-Bam*HI digested DNA (Ex. 1). The purified fragment were then ligated with *Not*I*-Bam*HI digested pLCTSN to create pLCTSN-BG.

### (i) Construction Of The pLCT-pA-BG-EF Retroviral Vector

pLCT-pA-BG-EF contains a cistron comprising the EF1α enhancer/promoter, B7-2 and GM-CSF cDNAs and SV40 polyadenylation signal inserted in an inverted orientation (relative to transcription from the 5' LTR) but lacks the the neo gene found in pLCTSN-pA-BG-EF vector. pLCT-pA-BG-EF was constructed by ligation of the following three fragments with *Hin*dIII*-Sal*I digested pLCTSN: EF1α enhancer/promoter from *Sal*I*-Not*I digested pHEF, the B7-2/IRES/GM-CSF cistron from *Not*I*-Bam*HI digested pLCTSN-BG, and the SV40 polyA signal from *Not*I*-Hin*dIII digested pSP72SVpA.

pSP72SVpA was made by PCR amplification of the SV40 polyA signal from pBlueBac-His B (Invitrogen) with primers containing *Hin*dIII and *Eco*RI sites and cloned into *Hin*dIII*-Eco*RI digested pSP72 (Promega).

The plasmids described above are introduced into tumor cells, including freshly explanted tumor cells as well as tumor cells in a patient, using injection of naked plasmid DNA, liposome mediated gene transfer or through the use of a gene gun (biolistics). The retroviral vectors described above are used to generate recombinant virus which is used to transduce the encoded IMGs into tumor cells (primary and established) in culture as described in Exs. 1 and 2.

### (i) Demonstration of Biologically Active GM-CSF From the GM-CSF Cistron

To demonstrate that the GM-CSF cDNA sequences isolated by PCR amplification and used to generate mono- and multi-cistronic constructs are capable of expressing active GM-CSF, the following GM-CSF bioactivity assay was performed using the GM-CSF-dependent Mo7e cell line [Genetics Institute, Cambridge, MA; Avanzi et al. (1990) Cellular Phyisol. 145:458].

Mo7e cells were maintained in DMEM supplemented with 10% FBS, 2 mM L-glutamine, 100 U/ml penicillin-streptomycin, and 100 U/ml GM-CSF at 37°C, 10% CO₂. When the cells were at exponential growth phase (viability > 90%), they were washed twice with DMEM, and cultured for 48 hours in the absence of GM-CSF. After two days of GM-CSF starvation, the Mo7e cells, with around 20% viability, were washed once with DMEM and seeded into V-bottomed 96-well plates at 1 x 10⁴ cells/well in a total volume of 200 µl with serial dilutions of a standard GM-CSF solution of known concentration or supernatant harvested from HeLa cells transfected with either pHEF-GM-CSF or pHEF-IL-12-GM-CSF plasmid DNA (a 1:2000 dilution of the culture supernatant was employed). After two days of culturing at 37°C, 10% CO₂, to each well, 20 µl of MTT (3-[4,5-dimethylthiazol-2-yl]-2,5-diphenyltetrazolium bromide) (5 mg/ml in PBS) was added and the plates were incubated for another 6 hours. The purple crystals were then collected at the bottom the well by brief centrifugation and the supernatant was aspirated. The pellets were dissolved in 100 µl of acidified isopropanol. The optical density at 570 nm (OD₅₇₀) of each well was obtained using a microplate reader. A standard curve was constructed by plotting the known GM-CSF concentrations against the OD₅₇₀ reading. GM-CSF concentration in the harvested supernatant was obtained by comparing its OD with the standard curve. The results are summarized in Table 6.

**TABLE 6**

| Determination Of GM-CSF Concentration In Cell Culture Supernatants Of Transfected HeLa Cells | | | |
|---|---|---|---|
| Samples | GM-CSF Bioactivity (U/ml) | GM-CSF Protein Content (pg/ml) | OD₅₇₀ |
| Standard 1 | 0 | 0 | 0.2 |
| Standard 2 | 0.01 | 1 | 0.203 |
| Standard 3 | 0.05 | 5 | 0.233 |
| Standard 4 | 0.2 | 20 | 0.343 |
| Standard 5 | 0.5 | 50 | 0.60 |
| Standard 6 | 1 | 100 | 0.65 |
| pHEF-GM-CSF (1:2000) | 0.29 | 29 | 0.365 |
| pHEF-IL-12-GM-CSF (1:2000) | 0.03 | 3 | 0.235 |

The results shown in Table 6 demonstrate that the PCR amplified GM-CSF sequences encode biologically active GM-CSF. In addition these results demonstrate that placing the GM-CSF cistron downstream of another cistron (*i.e*., pHEF-IL-12-GM-CSF) reduces the amount of GM-CSF expressed (relative to a mono-cistronic expression vector). While the level of GM-CSF is reduced when G-CSF is the second or downstream cistron, the amount of GM-CSF produced in multi-cistronic GM-CSF construct is sufficient to provoke a biological response as demonstrated by the ability of tumor cells transduced with a bi-cistronic retrovirus (*i.e*., pLSNBG9) to induce systemic immunity against unmodified tumor cells (Ex. 5).

### Example 11 - Human Tumor And PBL Engraftment In SCID/beige Mice

An *in vivo* hu-PBL-SCID mouse/human tumor model was established to study the combined effects of B7-2 and GM-CSF expression in human tumor cells. C.B-17 scid/scid mice [Boussiotis et al. (1993) Proc. Natl. Acad. Sci. USA 90:11059] lack both T- and B-cell function but do maintain normal myeloid cell, natural killer (NK) cell, macrophage and dendritic cell functions *[*Shpitz et al. (1994) J. Immunol. Meth. 169:1]. To also avoid the NK activity of the SCID mouse, a new SCID mouse strain, SCID/bg, which has the NK cell function deleted was employed (C.B-17-scid-beige Inbred, Taconic).

To investigate the ability of SCID/bg mice to support the growth of human tumor cells, several established human tumor cell lines and 5 primary tumors were transplanted into the SCID/bg mice and the mice were monitored for tumor growth for up to 5 months. Primary tumors were treated as follows. Tumor samples were obtained in the operating room at the time of resection under sterile conditions. One to three grams of tumor were harvested for immediate processing. Fresh tumor biopsies were dissected to remove necrotic debris and connective tissue and were minced into 1-2 mm pieces. The pieces were then treated with the following enzymatic solutions in HBSS to provide cell suspensions:
for glioblastomas: 0.025% collagenase, 0.04% DNase and 0.05% Pronase with shaking for 30 min at 37°C and a further 30 min at 4°C. DMEM/F12 was used as the basis of the growth medium for primary glioblastoma cultures;
for hepatomas: 1 mg/ml collagenase type IV, 300 U/ml DNase type IV and 10 µg/ml gentamycin sulfate at 37°C with shaking for 30 min. RMPI 1640 or DMEM/F12 was used as the basis of the growth medium for primary hepatoma cultures;
for colon adenocarcinomas: tumors were processed by physical dissociation (cutting) or by enzymatic treatment using 1 mg/ml collagenase type V, 10 µg/ml hyaluronidase type V, 300 U/ml DNase type IV and 10 µg/ml gentamycin sulfate. The cells suspension was then incubated at 37°C with shaking for 60 min. DMEM was used as the basis of the growth medium for primary adenocarcinoma cultures.
for melanomas: tumor cells were treated using the enzyme solution described above for adenocarcinomas. RPMI 1640 was used as the basis of the growth medium for primary melanoma cultures.

The resulting cell suspensions were then filtered through a fine mesh and layered onto a Ficoll-hypaque density gradient medium (Sigma) and centrifuged at 400xg for 30 min at room temp. The cells at the interface were removed and washed twice with HBSS (centrifuged at 100xg for 10 min) and counted. The cells were then seeded into the appropriate medium [*e.g*., RPMI 1640, DMEM, DMEM/F12 (all available from Sigma), etc. containing 10% FCS and antibiotics if desired] for culturing, resuspended into freezing medium for storage or used directly for injection into mice or for gene transfection applications.

Three to four million primary tumor cells and 2 to 6 million cells from established tumor cell lines were injected SC per flank of SCID/bg mice (both flanks were injected if a large enough cell sample was available). The mice were monitored for the presence of palpable tumors. The results are summarized in Table 7.

**TABLE 7**

| Establishment Of Solid Human Tumors In SCID/bg Mice | | | | | |
|---|---|---|---|---|---|
| Tumor Inoculation | | No. Of Cells Injected | No. Of Mice | Palpable Tumor Established (Days Post- Inoculation) | Success Rate (%) |
| Cell Lines | | | | | |
| Melanoma | | | | | |
| | A375 | 2-5 x 10⁶ | 30 | 5-8 | 100 |
| | SK-MEL-1 | 4 x 10⁶ | 12 | 20-30 | 100 |

| Hepatoma | | | | | |
|---|---|---|---|---|---|
| | HepG2 | 3 x 10⁶ | 5 | 10-15 | 100 |

| Breast Cancer | | | | | |
|---|---|---|---|---|---|
| | MDA468 | 2-5 x 10⁶ | 35 | 5-7 | 100 |
| | MCF7 | 4-6 x 10⁶ | 7 | 8-14 | 100 |

| Glioblastoma | | | | | |
|---|---|---|---|---|---|
| | D54MG | 2 x 10⁶ | 5 | 5-6 | 100 |

| Primary Tumors | | | | | |
|---|---|---|---|---|---|
| Melanoma | | | | | |
| | Mel DD | 4 x 10⁶ | 8 | 30-40 | 100 |
| | Mel DJ | 3 x 10⁶ | 4 | 30-40 | 100 |
| | Mel TM | 3 x 10⁶ | 5 | 30-40 | 100 |

The results shown in Table 7 demonstrate that the SCID/bg mice can support growth of the established human tumor lines very efficiently and they support the growth of some primary tumor cells (*e.g*., melanoma cells), albeit with a lower efficiency relative to the use of established tumor cell lines. Injection of 3 x 10⁶ primary glioblastoma cells (Ed 86 and Ed 120) as well as injection of primary heptatoma cells (single donor) did not give rise to palpable tumors within 3 months in SCID/bg mice.

T-cell mediated allograft rejection has been demonstrated in SCID mice (scid/scid) engrafted with human PBLs [Malkovska et al. (1994) Clin. Exp. Immunol. 96:158] or human splenocytes [Alegre et al. (1994) J. Immunol. 153:2738]. However, the SCID/bg mouse strain has not previously been used in hu-PBL reconstitution studies. To determine whether the SCID/bg mouse could be reconstituted with human PBLs to provide an animal model for *in vivo* cancer gene therapy, SICD/bg mice were reconstituted with human PBLs by injecting 2 x 10⁷ freshly isolated human PBLs into the peritoneal cavity. After 4-8 weeks, the PBL-injected mice were sacrificed and cells harvested from the peritoneal cavity, spleen, and peripheral blood were analyzed by FACS using antibodies against mouse or human cell markers. Isotype-matched mouse Igs were used for control staining. The results are summarized in Table 8. The following antibodies were used in these experiments: human markers: anti-CD45 (anti-HLe-1, Becton Dickinson), anti-CD3 (Leu-4, Becton Dickinson), anti-CD4 (Becton Dickinson), anti-CD8 (Becton Dickinson) and mouse marker: anti-mouse-H-2K° (Pharmingen).

**TABLE 8**

| | % Human CD45 |
|---|---|
| Spleen | 1-59% |
| PBL | 0.1-17% |
| Peritoneal Cavity | 5-69% |

The results shown in Table 8 demonstrate that SCID/bg mice were efficiently reconstituted with human lymphocytes, with CD45+ human cells constituting up to ~60 % of splenocytes, up to 17% of PBLs and up to ~70% of cells obtained by peritoneal lavage in the reconstituted mice.

The percentage of different human lymphocyte subsets in the CD45+ population present in the reconstituted SCID/bg mice was examined by FACS as follows. SCID/bg mice were reconstituted with PBLs isolated from three healthy human donors as described in Ex. 3. After 4-8 weeks, the PBL-injected mice were sacrificed and lymphoid organs, PBLs and peritoneal exudates were harvested and were analyzed by FACS. The percentage ofT lymphocyte subsets was also determined within the CD45+ population in the peripheral blood of human donors A and B. The following markers were examined: CD45RO (anti-CD45RO, Becton Dickinson), CD45RA (anti-CD45RA, Becton Dickinson), HLA-DR (MG2600, Caltag), CD4 and CD8. The results are summarized in Table 9. In Table 9, the following abbreviations are used: RA+ (CD45RA positive); RO+ (CD45RO positive); RO+DR+ (CD45RO and HLA-DR positive).

**TABLE 9**

| Percentage Of T Lymphocyte Subsets In The CD45+ Population In Hu-PBL-SCID/bg Mice | | | | | | |
|---|---|---|---|---|---|---|
| Donor/Mouse | CD4+ | CD8+ | CD4+8+ | RO+ | RA⁺ | RO+DR+ |
| Donor A | 53 | 23 | 1.5 | 58 | 37 | 9.9 |
| SCID/bg-A | 72 | 42 | 17 | 99 | 1 | 67 |
| Donor B | 63 | 25 | 1.6 | 58 | 37 | 9.4 |
| SCID/bg-B | 41 | 48 | 12 | 92 | 8 | 67 |
| SCID/bg-C | 51 | 44 | 21 | 76 | 24 | 35 |

The results shown in Table 9 demonstrate that the peripheral blood of the Hu-PBL-SCID/bg mice contain high numbers of immature or progenitor T cells (*i.e*., CD4+8+ cells and CD45RA+ cells). These results are in contrast to the results obtained by reconstitution of C.B-17scid/scid mice (Hu-PBL-SCID). In human PBL-reconstituted C.B-17 scid/scid mice most human lymphocytes exhibit activated cell phenotypes (HLA-DR+ and CD25+ or CD69+) soon after reconstitution, and almost all (>99%) human T cells exhibit mature memory phenotypes (CD45RO+) in a state of reversible anergy [Rizza *et al.* (1996), *supra;* Tarry-Lehmann and Saxon, *supra;* Tarry-Lehmann *et al., supra*]*.* Therefore, the lack of sufficient numbers of immature naive T cells after reconstitution renders the Hu-PBL-SCID model unsuitable for the evaluation of anti-tumor immunity. In contrast, the Hu-PBL-SCID/bg mice show evident levels of CD45RA+ and CD4+8+ cells 4-6 weeks after reconstitution. Thus, the Hu-PBL-SCID/bg mice of the present invention provide a suitable model for the evaluation of anti-tumor immunity.

### Example 12 - Autologous Hu-PBL-SC1D/bg/Human Tumor Model

To provide an autologous Hu-PBL-SCID/bg/human tumor model, primary human tumor cells are prepared and injected SC into the flanks of SCID/bg mice as described in Ex. 11. The primary tumor cells are first transduced with vectors encoding one or more therapeutic proteins (*e.g.*, B7-2, GM-CSF, IL-12A, IL-12B, etc.) and mice are injected with the transduced tumor cells (after selection) as well as non-transduced tumor cells. Once palpable tumors are established (1-2 months), the mice are reconstituted with autologous PBLs (*i.e*., PBLs isolated from the same patient that provided the tumor cells). For each tumor, a minimum of 10 SCID/bg mice are injected with tumor cells and the rate of establishment of palpable tumors is determined. Tumors that grow with a success rate of >40% in the injected mice will receive autologous PBLs and 1 week later will receive IMG-transduced tumor cells. The tumor size (mm³) is measured every 3-4 days and the mice are monitored for survival. These mice are used to determine which combinations of therapeutic or immune-modulating genes (IMGs) result in the regression of tumor size and prolonged mouse survival.

Mice from each group are sacrificed at various intervals and spleen cells and draining lymph node cells are assayed for immune reactivity (*i.e*., anti-tumor cellular immunity) (*e.g*., ⁵¹Cr release assays, proliferation and cytokine production) to determine whether any of the assays of immune reactivity correlate with observed tumor rejection.

### Proliferation Assays And In Vitro Priming

Splenocytes and lymph node cells isolated from the reconstituted mice are co-cultured with lethally irradiated tumor cells transduced with IMGs for various lengths of time (minimum of 5 days) and assayed for proliferation by pulsing the cultures with ³H-thymidine, *e.g*., on day 4 of culture and determining ³H-thymidine incorporation into cellular DNA 18-24 hr later.

Responding lymphocytes may be restimulated repeatedly (weekly) with untransduced or transduced tumor cells to increase the frequency of T cell precursors specific to the tumor. The tumor-responsive immune cells are then transferred into mice carrying the parental tumor to determine if *in vitro* priming generates cells which can cause tumor regression.

### Cytokine Production

Interferon γ (ITNγ), tumor necrosis factor α (TNFα), and IL-2 are cytokines associated with cell-mediated immune responses that direct the expansion and activation of NK cells and lymphokine activated killer (LAK) cells and the expansion of CD8+ CTL. Such effector cells are likely to have particular relevance to the elimination of tumors since animals lacking these effector cell types do not efficiently eliminate tumors or metastases [Whiteside et al. (1994) Clin. Immunother. 1:56].

Lymphocytes isolated from the PBL and tumor reconstituted mice are co-cultured with autologous untransduced or mock-transduced and IMG expressing tumor cells are assayed for the production of cytokines associated with cell-mediated immunity. Secretion of IFNγ, TNFα and IL-2 into the culture supernatants at various times during co-culture are assayed by sandwich ELISA [Sad et al. (1995) Immunity 2:271].

### In Vitro CTL Assay

Viable cells are harvested and enumerated from co-cultures of lymphocytes with autologous tumor cells (mock transduced or expressing IMGs) after 5 days of culture or after multiple weekly stimulations (as described above). Untransduced autologous tumor cells are labelled with ⁵¹NaChromate and standard 5 hour ⁵¹Cr-release assays are performed. Lysis of autologous and allogenic tumor targets are compared to the original stimulating cells. Lysis of tumor cells matching those used as the original stimulus, but not of other tumor cells, indicates the lysis is likely antigen specific. Proof of antigen specific lysis is made by the ability to block lysis using antibodies to class I MHC molecules and the ability to eliminate the effector cell responsible for lysis through the use of anti-CD8 and complement. non-MHC restricted lysis may indicate the induction of LAK cells.

### In Vitro NK/LAK Assay

Some tumor cells escape immune T cell recognition and elimination *in vivo* by down-regulating the expression of MHC molecules, particularly class I MHC proteins [Rivoltini et al. (1995) Cancer Res. 55:3149]. However, unlike CD8+ CTL, fresh NK cells and IL-2 expanded NK cells (LAK) do not require MHC molecules for their recognition and lysis of target cells [Lanier and Phillips (1988) ISI Atlas of Science, pp. 15-29]. NK/LAK cells may therefore serve to eliminate cells expressing altered or reduced levels of class I molecules that normally escape CD8+ T cell surveillance.

Five hour ⁵¹Cr release assays are used to determine which primary (or established) tumors are NK sensitive. Co-culture of autologous PBLs or immune cells isolated from PBL and tumor reconstituted SCID/bg mice are conducted using mock transduced or IMG expressing tumors to determine whether this results in augmented NK/LAK (non MHC) restricted cytolytic activity that includes the stimulating tumor target if initially observed, or the induction of substantial NK/LAK activity when none was present (against the tumor target) in the initial assay.

### Example 13 - Delivery Of Immune-Modulating Genes To Human Tumors

Immune-modulating genes may be delivered to human tumor cells *in vivo* and *ex vivo* by a variety of means.

### (a) Retroviral Transduction

Retroviral vectors encoding immune-modulating proteins may be used to introduce IMGs into established or primary tumor cells as described in Exs. 2, 6 and 7. The transfer of IMGs using retroviruses may be made more efficient by increasing the titer of the virus encoding the IMG(s) and increasing the transduction efficiency. To increase the virus titer, single cell clones from the producer PA317 cells are be selected by growth in the presence of G418 (or selective medium suitable for the selectable marker carried on the retroviral construct) and clones producing the highest titers of virus are be expanded. To increase the titer of the producer cell line further, the PA317 cells can be reinfected with ecotropic virus [*e.g*., virus produced in GPE-86 packaging cells] and the best producer cell clones can be selected. To improve the transduction efficiency, retrovirus are used in combination with liposomes or poly-L-omithine or polylysine to enhance virus uptake.

Another way to improve gene transfer efficiency using retroviruses is to increase the targeting efficiency. Many tumor cells including glioblastomas and melanomas express excess levels of the transferrin receptor. Transferrin has been used to increase the transduction efficiency of adenovirus in combination with polylysine [Lozier et al. (1994) Human Gene Ther. 5:313]. Several recent reports demonstrated that replacing the SU (surface) domain of the env gene of a retrovirus can increase receptor-mediated transduction efficiency [Kasahara et al. (1994) Science 266:1373; Cosset et al. (1995) J. Virol. 69:6314; Dong et al. (1992) J. Virol. 66:7374; and Chu and Domburg (1995) J. Virol. 69:2659]. The human transferrin gene is 2097 bp long (coding region provided in SEQ ID NO:25). Insertion of such a long sequence into the SU domain of the env gene of MLV vector may not produce a stable Env product. However, earlier studies have suggested that the modified Env fusion protein requires the native Env for stable assembly and efficient entry. Thus, the transferrin-env fusion gene is co-transfected with the native env gene to produce retrovirus particle bearing a mixture of wild type and recombinant Env. The gene transfer efficiency of the new vector is examined by transducing glioblastomas or melanomas expressing high levels of transferrin receptor.

### (b) Recombinant Adenoviral Vectors

Recombinant adenoviruses can accommodate relatively large segments of foreign DNA (~7 kb), and have the advantage of a broad host cell range and high titer virus production [Graham and Prevec (1991) Meth. Mol. Biol. 7:109-128]. Adenoviruses have been used *in vivo* in rats to efficiently deliver genes to the liver and the pancreatic islets [reviewed in Becker et al. (1994) In Protein Expression in Animal Cells, Roth et al. eds.] and to the central nervous system [Davidson et al. (1993) Nature Genet. 3:219]. Rat livers have also been efficiently transduced *ex vivo* and then re-implanted [Shaked et al. (1994) Transplantation 57:1508].

The replication defective recombinant adenoviruses are employed; these viruses contain a deletion of the key immediate early genes E1a and E1b (Graham and Prevec, *supra*)*.* To generate and propagate recombinant viruses, a packaging cell line such as 293 cells which supply the E1a and E2a proteins *in trans* is employed. Recombinant adenoviruses are created by making use of intracellular recombination between a much larger plasmid encoding most of the viral genome and a small plasmid containing the gene of interest (*e.g*., a cytokine) bracketed by regions of homology with the viral integration site. Recombinant adenoviruses expressing mouse IL-4 and IL-10, both under the control of a CMV immediate early enhancer promoter, have been constructed. Recombinant adenoviruses expressing human B7-2 and GM-CSF as well as a second virus expressing both the IL-12A and IL-12B subunits are constructed. The DNA constructs are cloned as bi-cistronic units with IRES as described in Ex. 1.

Standard methods are used to construct the recombinant adenoviruses (Graham and Prevec, *supra* and Becker *et al., supra*). Briefly, each plasmid is co-transfected together with pJM 17 (Microbix Systems, Toronto) into sub-confluent monolayers of 293 cells (ATCC CRL 1573) using calcium phosphate precipitation and a glycerol shock. Initial recombinant viral stocks are titered on monolayers of 293 cells, and isolated single plaques are obtained and tested for cytokine expression using an ELISA. Viral stocks are amplified and titered on 293 cells, and stored in aliquots at -70°C; if necessary, stocks are concentrated by centrifugation on density gradients. To infect tumor cells with recombinant adenoviruses, freshly isolated tumor cells are mixed with adenoviral stocks in a minimal volume. Titers of stocks are typically 10⁵-10⁸/ml. Medium is replaced after several hours and the cells are followed for expression of the recombinant adenoviral-encoded IMGs and/or reporter genes.

A potential drawback of using a adenoviral delivery system is that the transduced cells may retain or express small quantities of adenoviral antigens on their surface [Yang et al. (1994) Nature Genet. 7:362]. "Second generation" adenoviral vectors which contain deletions in the E2a gene are available and are associated with less inflammation in the recipient and a longer period of expression of the gene of interest [Yang *et al.,* supra and Engelhardt et al. (1994) Proc. Natl. Acad. Sci. USA 91:6196]. If necessary, IMGs are inserted into second generation adenoviral vectors. However, since the transduced tumor cells are lethally irradiated before injection into the recipient and since other manipulations are undertaken to induce the tumor cells to secrete immune-stimulating cytokines and to express surface signalling molecules, the expression of small quantities of adenoviral proteins (when first generation vectors are employed) may provide a desirable adjuvant effect. Furthermore, the recipient is subsequently boosted with retrovirally transduced tumor cells (which express the cytokines and tumor antigens, but not the adenoviral antigens). The recipient is monitored for tumor-specific immune responses at a secondary distant site where non-transduced tumor is implanted; the generation of such a response indicates that the desired tumor-specific immunity has been achieved.

### (c) Targeted Cationic Liposomes

Cationic liposomes have proven to be a safe and effective means for inducing the transient expression of DNA in target cells [Ledley (1995) Human Gene Ther. 6:1129; Feigner (1990) Adv. Drug Delivery Rev. 5:167; Felgner et al. (1987) Proc. Natl. Acad. Sci. USA 84:7413; and Smith et al. (1993) Biochim. Biophys. Acta 1154:327]. Clinical trails are underway using cationic liposomes to introduce the CFTR gene into the lungs of cystic fibrosis patients [Caplen et al. (1994) Gene Ther. 1:139 and Alton et al. (1993) Nature Genet. 5:135] or to introduce, by direct intra-tumor injection, the T cell costimulator B7-1 into malignant melanoma lesions in order to induce a cell-mediated immune response [Nabel et al. (1993) Proc. Natl. Acad. Sci. USA 90:11307].

Cationic liposomes (*e.g*., DOTAP/DOPE) and ligand-targeted cationic liposomes are employed for the delivery of IMGs to tumor cells. Ligand-targeted liposomes are made by covalently attaching ligands or antibodies to the surface of the cationic liposome. When glioblastoma cells are to be targeted, transferrin is used as the ligand as glioblastoma cells express high levels of the transferrin receptor on their surface. When melanoma cells are to be targeted, internalizing receptors, monoclonal antibodies directed against melanoma-specific surface antigens (*e.g*., mAb HMSA5) are employed as the ligand.

Plasmid DNA encoding IMGs (*e.g*., B7-2, GM-CSF and IL-12) is formed into a complex with preformed cationic liposomes using standard methodology or alternatively the DNA is encapsulated into the liposome interior. The DNA-containing liposomes are then used to transfer the DNA to tumor cells *in vivo* by direct intra-tumor injection or *in vitro* (using freshly explanted tumor cells) followed by return of the transduced cells to the recipient (*e.g*., a patient).

### (d) Gene Transfer Using Biolistics

Biolistics (microballistics) is a method of delivery DNA into cells by projection of DNA-coated particles into cells or tissues. DNA is coated onto the surface of tiny (~1-3 µm diameter) gold or tungsten microparticles and these particles are accelerated to high velocity and are impacted onto the target cells. The particles burst through the cell membrane and lodge within the target cell. The cell membrane quickly reseals and the passenger DNA elutes off of the particle and is expressed. The biolistic method has been used to transfect mammalian cells [Williams et al. (1991) Proc. Natl. Acad. Sci. USA 88:2726; Tang et al. (1992) Nature 356:152; Sanford et al. (1993) Methods Enzymol. 217:483].

A hand-held biolistic apparatus (BioRad) is used to transfer DNA into tumor cells or isolated tumor fragments. This device uses compressed helium to drive a disc-shaped macroprojectile which carries on its surface microparticles of gold (1-5 µm) of gold which have been coated with purified plasmid DNA (coprecipitated with spermine) (Williams *et al., supra*). This apparatus has been used to successfully transfect primary tissues.

Plasmid DNA encoding the IMGs is coated onto the surface of gold microparticles according to the manufacturer's instructions (BioRad) and the biolistic apparatus is used to transfer the DNA into freshly explanted tumor cells or directly into exposed tumors (*e.g*., metastatic nodules on the surface of the liver, melanoma lesions on the skin).

### Example 14 - Further Characterization of Human PBL Reconstituted SCID/beige And SCID/nod Mice

This example provides additional characterization of the reconstituted SCID/nod and SCID/beige mice described in Examples 3 and 11 above.

To study the efficiency of human immune cell reconstitution in scid mice, SCID/nod and SCID/beige mice were reconstituted with PBLs of different donors as described in Ex. 3. Analyses of human Ig levels at 2, or 4-7 weeks after reconstitution showed that both of these strains of mice were capable of producing human Igs at levels averaging 500-600 µg/ml; Ig levels were assessed by ELISA (Ex. 3). Approximately half of the SCID/nod mice used in the reconstitution study did not produce detectable amount of human Igs.

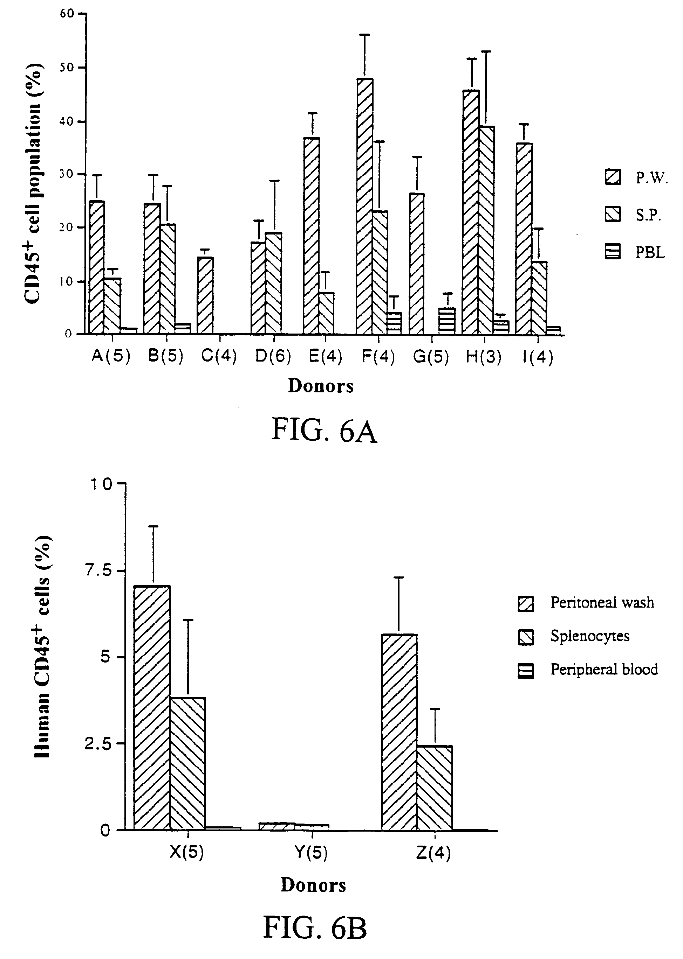

The percentages of human lymphocytes present in different organ compartments of the reconstituted mice were determined two months after reconstitution. As described in Ex. 11, cells were harvested from peritoneal cavity (P.W. or peritoneal wash), spleen (Splenocytes or S.P.), and peripheral blood (PBL), and analyzed by flow cytometry using antibodies against the human leukocyte surface marker CD45. The results are summarized in Figures 6A and 6B.

The results shown in Figs. 6A indicated that SCID/beige mice were consistently reconstituted with human PBLs in all three organ compartments with little variation among donors (donors A-I: the numbers in the brackets by the donor designation indicates the number of animals reconstituted with donor PBLs). In contrast, the overall reconstitution efficiency in the SCId/nod mice was low, *i.e.,* <10% in the peritoneal cavity, <5% in the spleen, and almost undetectable in the peripheral blood (Fig. 6B; donors X-Z). Donor-to-donor variation was also more pronounced in the SCID/nod mice. For this reason, as well as the reasons discussed in Ex. 3, reconstituted SCID/bg mice are preferred for vaccination/challenge experiments (*i.e*., evaluation of candidate vaccines).

### Example 15 - Anti-HIV Immunity Cannot Be Assessed By In Vitro Assays

The time course of disease progression after HIV infection varies depending on a number of factors including viral strains and host immune responses. While the majority of HIV-infected subjects develop AIDS at a median of ≈ 10 years, up to 5-10% of infected individuals do not appear to have clinical symptoms even after 10 years [Haynes et al. (1996) Science 271:324 and Baltimore (1995) N. Engl. J. Med. 332:259]. Indeed, epidemiological evidence suggests that some individuals may be resistant to infection by HIV; despite multiple high-risk exposures, these individuals remain HIV negative [Detels et al. (1994) J. Acquired Immune Defic. Syndr. 7:1263 and Taylor (1994) J. NIH Res. 6:29].

Both host genetic factors and the infection-immunization process following HIV exposure may play roles in the development of protective immunity. The mechanism of resistance to HIV infection in the HIV-exposed but uninfected individuals may be associated with HIV-specific cytotoxic T cell activity, a switch from Th2 to Th1 cytokine response, genetic loci linked to HLA, transporter associated with antigen processing (TAP), and HIV coreceptors, or development of anti-HLA antibodies. While strong indirect evidence supports the hypothesis that these exposed but uninfected individuals have protective immunity against HIV infection, direct *in vivo* experiments attesting to this hypothesis are still lacking. Understanding the mechanism of such protective immunity is necessary for to the design and testing of effective prophylactic vaccines and immunotherapeutic regimens.

### (a) The PBLs from High Risk Individuals Are Susceptible to HIV Infection In Tissue Culture

To determine if lymphocytes from high risk (HR) individuals were susceptible to HIV-1 infection, their PBLs were collected, activated with PHA in culture and then incubated with T cell or macrophage tropic HIV-1. Infection was scored on the basis of HIV-1 RT activity and immunohistochemical staining with AIDS patients' sera.

### (i) Study Subjects

Two individuals were identified who have engaged in regular unprotected heterosexual or homosexual intercourse with HIV-positive partners over a period of up to 10 years, but have remained HIV negative as determined by repeated serological assay, polymerase chain reaction (PCR) and PBL cocultivation. High-risk (HR) donor 1 is the heterosexual partner of a bisexual individual who had a CD4 count of 400-500/µl at the time of positive IDV diagnosis s in July 1992. An estimated 144 episodes of vaginal intercourse between the HR donor 1 and the partner took place before his diagnosis, and the HIV-positive partner's CD4 count has decreased to 30/µl at the time of study. HR donor 2 is the homosexual partner of an individual known to be HIV-1 positive since 1986 and whose CD4 count remained in the 300-400/µl range. It was estimated that unprotected anal sex took place ≈ 225 times before this study, and unprotected sexual contact has "continued" since diagnosis. Infectious virus had been isolated from phytohemagglutinin (PRA)-blasted PBLs of both HIV-positive partners. These two HR subjects who participated in the hu-PBL-SCID mouse challenge study described below do not share common HLA haplotypes. Additional multiply exposed, uninfected individuals who fit into the above criteria were also included in the *in vitro* studies. Blood provided by Canadian Red Cross and volunteers in the laboratory with no prior HR exposures to HIV were the source of control PBLs.

### (ii) Virus Preparation

HIV-1 strains used in this study include laboratory-established T cell tropic (HIV_{NL4-3}) and macrophage tropic HN-1 (HIV _{NLAD8} also referred to as HIV_{ADA}) [Adachi et al. (1986) J. Virol. 59:284]. HIV_{NL4-3} and HIV_{NLAD8} are available as catalog no. 78 and no. 416, respectively from the NIH AIDS Research and Reference Reagent Program of National Institutes of Health, Bethesda, MD and the sequence of pNL4-3, a plasmid containing a proviral form of HIV_{NL4-3} is available in the GenBank database under accession number M19921. Virus stocks were prepared from infected peripheral blood mononuclear cells or macrophages as described [Chang and Zhang (1995) Virol. 211:157]. Briefly, macrophage cultures were prepared from HIV-seronegative donors by adherence of PBLs to plastic flasks as described previously with minor modifications [Hassan et al. (1986) J. Immunol. Methods 95:273]. PBLs were prepared by density gradient separation using lymphocyte separation medium (Organon Teknika Corp., Durham, NC). The PBLs were resuspended in RPMI 1640 medium supplemented with 20% heat-inactivated human serum. Approximately 5x10⁷ PBLs were attached to a T-75 flask and incubated overnight at 37°C. The next day cells were washed three times with PBS and the attached cells were incubated with 0.02% EDTA in PBS for 5-10 min. The cells were collected with a cell scraper and plated onto a 48-well plate at 5 x 10⁴ cells per well. The viability approached 100% as determined by trypan blue staining. The initial monocytes were characterized by Wright's staining and the mature macrophages by both Wright's staining and microscopic examination.

HIV virus stocks were prepared by transfecting HeLa cells or by infecting human PBLs, CD4+ lymphocytes (T cell or macrophage tropic viruses) or macrophages (macrophage tropic viruses); infections may be performed using an moi of about 0.2. After transfection, culture media was collected 48 hr later and frozen in aliquots at -80°C. After infection, HIV RT kinetics were followed and virus was harvested at the peak ofRT production. Virus aliquots were examined by RT assay and TCID50 assay.

The same virus stocks were used for both *in vitro* and *in vivo* experiments. The multiplicity of infection (moi) or tissue culture infectious dose (TCID₅₀) was determined by serial dilution of virus stock on HIV-1 negative, PHA-stimulated PBLs as described previously [Chang and Zhang, *supra* and Ho et al. (1989) N. Engl. J. Med. 321:1621]. Briefly, PBLs from HIV-seronegative donors were stimulated with 0.25 µg/ml PHA for 3 days prior to infection and then maintained in RPMI 1640 supplemented with 10% FBS and 10% interleukin-2 (Pharmacia). PBLs were plated in 48 well plates at 0.5 million per well and infected with serially diluted virus supernatants in duplicate. Culture supernatants were harvested every 3-4 days. At approximately 3-4 weeks of time, infection kinetics was determined. The dilution point that gave 50% of positive infection (one out of two wells) was considered to be the TCID50 point.

### (iii) In Vitro HIV-1 Infection

Heparinized peripheral blood was collected from HR and low-risk (LR) HIV-negative donors. PBLs were separated by gradient centrifugation with Histopaque (Sigma). Infection was performed as described previously (Chang and Zhang, *supra*). Briefly, PBLs were treated with PHA (5 µg/ml, Sigma) and infected with T cell or macrophage tropic HIV-1 at 0.2 moi. The culture was split at a 1:3 ratio every 3-4 days. The supernatant was harvested for reverse transcriptase (RT) assay, and infections were also confirmed by immunohistochemical staining as described (Chang and Zhang, *supra*). Briefly, adherent cells were washed with PBS three times, fixed in cold acetone and methanol (1:1) for 2 min, washed three times in PBS, and incubated in blocking solution (20% FBS, 0.1 % TritonX100 in PBS) for 30 min. Non-adherent cells were attached to the surface of a 24-well plate which had been pretreated with poly-D-lysine (1 mg/ml, Sigma) at room temperature for 10 min. An HIV patient serum which was diluted at 1:2000 in a blocking solution containing 20% FBS, 0.1% TritonX100 and 2% dry milk in PBS was used as the first antibody and the incubation was done at room temperature for 1 hr or at 4°C overnight with constant shaking. After being washed in PBS for 5 min 4 times, the cells were incubated with a 1:200 dilution of normal sheep antisera at room temperature for 30 min to block non-specific signals. The secondary antibody was a biotinylated sheep anti-human antibody (Amersham) which was used at 1:2000 dilution and incubated at room temperature for 1 hr. The cells were washed four times in PBS-Tween 20 (0.3%) and incubated in the ultra-sensitive ABC staining solution (containing avidin and biotinylated horseradish peroxidase, Pierce Chemical Co.) at room temperature for 30 min. After four more washes in PBS-Tween 20, the cells were incubated in 3, 3'-Diaminobenzidine tetrahydrochloride (DAB) solution (Sigma) containing 0.3% NiCl₂ for 2-3 min. The reaction was stopped by washing cells with tap water for 1-2 min. Cell staining was scored under an inverted microscope and photographed. To reduce background staining, both the primary and the secondary antisera were preabsorbed with fixed human PBLs. Pretreatment of fixed cells with 0.01% H₂O₂ at room temperature for 5 min essentially eliminated all nonspecific background signals. The percentages of positive cells were determined by taking the average of more than three representative counts of 1,000 or 10,000 cells.

### (iv) RT Assay

Reverse transcriptase levels were measured as follows. A 10 µl sample of culture medium (supernatant) was incubated with 50 µl of a reaction cocktail containing 50 mM Tris-HCl, pH 8.3, 20 mM DTT, 0.6 mM MnCl₂, 60 mM NaCl, 0.05% NP40, 5 µg/ml of oligodeoxythymidilic acid, 10 µg/ml of polyriboadenylic acid and 10 µM of [α-³²P]dTTP (DuPont NEN, specific activity 800 Ci/mmol). The reaction was incubated at 37°C for 1 hr. A 3 µl aliquot was then spotted onto DE-81 paper (Whatman), and air dried. The DE-81 paper was washed 3 times in 2X SSC (20X SSC comprises: 3 M NaCl, 0.3 M sodium citrate, pH 7.0) and autoradiographed.

### (v) Immunohistochemical Staining and Fluorescence-Activated Cell Sorter (FACS) Analysis

Immunohistochemical staining was performed as described in section iii above. For FACS analysis, red blood cells were lysed with ammonium chloride solution [containing 9 ml of 0.16 M NH₄Cl and 1 ml of 0.17 M Tris (pH 7.62 to final pH 7.2)]. The cells were stained with PE-anti-mouse-H-2K^{d} (Pharmingen), fluorescein isothiocyanate (FITC)-anti-human-CD45 (anti-HLe-1, Becton Dickinson), phycoerythrin-anti-human-CD4 or FITC-anti-human CD8 (Becton Dickinson) for 30 min on ice followed by three washes with immunostaining buffer (PBS containing 0.1% FBS, 0.02% NaN₃)- Isotype-matched PE- and FITC-mouse Igs were used for control staining. The samples were analyzed using the LYSIS II program on a FACScan (Becton Dickinson).

### (vi) PBLs From High Risk Donors Are Susceptible To HIV Infection In Vitro

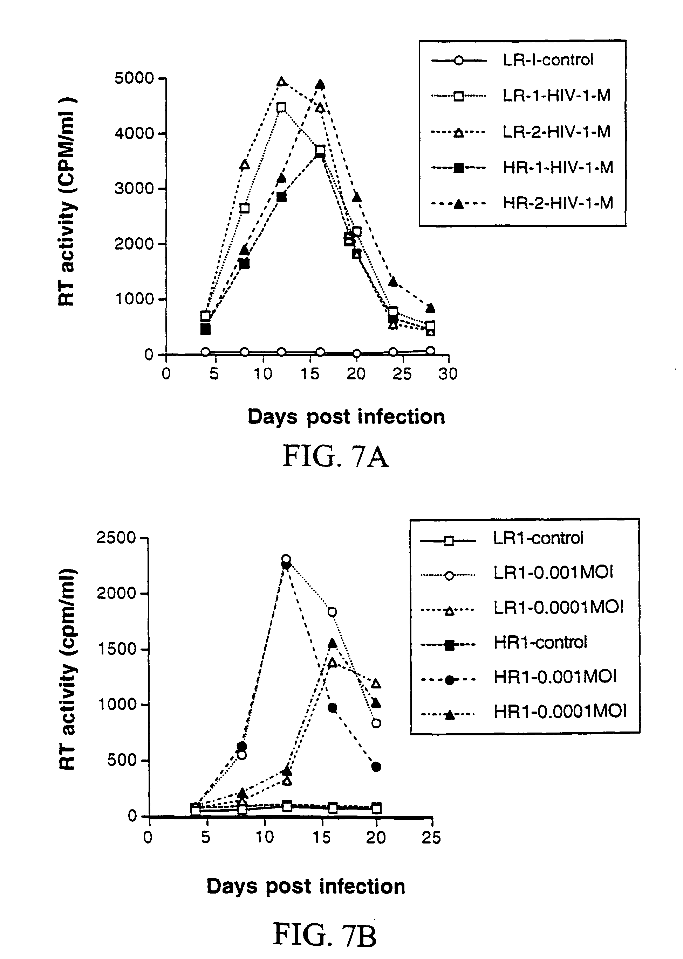

As shown in Fig. 7a, the RT assay demonstrated that PBLs from these two HR, HN-1-seronegative individuals were as susceptible as PBLs from normal LR donors to HIV-1 infection in tissue culture (Fig. 7a). In Fig. 7a, the RT activity (CPM/µl) is plotted against days post infection. The RT activity in PBLs from two LR individuals (LR-1 and LR-2) infected with the macrophage tropic HIV_{NLAD8} ("HIV-1-M") at 0.2 moi is shown using open squares and triangles, respectively; RT activity in uninfected PBLs from LR-1 is shown using the open circles ("LR-1-control"). The RT activity in PBLs from two HR individuals (HR-1 and HR-2) infected with HIV_{NLAD8} at 0.2 moi is shown using solid squares and triangles, respectively. Similar results were obtained with the T-cell tropic strain HIV-1_{NL4-3}. The immunostaining results confirmed the RT assay results.

To further examine whether the HR individuals' PBLs were somewhat resistant to HIV-1 infection, the tissue culture infection was performed using a series of diluted virus preparations (from moi of 10⁻¹ to 10⁻⁴). The results are summarized in Fig. 7b. In Fig. 7b, the RT activity (CPM/µl) is plotted against days post infection. The open circles show RT activity in uninfected PBLs from LR-1; the open squares and triangles show RT activity in PBLs from LR-1 infected with HIV-1_{NL4-3} at a moi of 0.001 and 0.0001, respectively. The solid circles show RT activity in uninfected PBLs from HR-1; the solid squares and triangles show RT activity in PBLs from HR-1 infected with HIV-1_{NL4-3} at a moi of 0.001 and 0.0001, respectively. These results showed that even with a moi of 10⁻⁴, the HR donors' PBLs were still infected. A comparable dose-dependent kinetics of infection with different concentrations of virus was observed between LR and HR individuals. Similar results have been reported for HR, HIV-1-seronegative hemophiliacs [Lederman et al. (1995) J. Inf. Dis.172:228]. However, little is known about the possible nature of the protective immunity in these individuals.

### Example 16 - HIV-1 Exposed-but-Uninfected Individuals Are Resistant to HIV-1 Infection In Vivo

To overcome the problem of the lack of an existing *in vivo* HIV-1 infection model, the human PBL-reconstituted severe combined immunodeficiency mouse model (hu-PBL-SCID) described in Exs. 3, 11 and 14 was used to investigate the nature of the protective immunity in the IHV-exposed but uninfected individuals. This *in vivo* model was used to test the susceptibility of the two HR, HIV-negative individuals to HIV-1 infection. PBLs were isolated and cells from each donor were used to reconstitute 6-7 SCID/bg mice each time as described in Ex. 3. Two weeks after reconstitution, the hu-PBL-SCID/bg mice were challenged with T cell or macrophage tropic HIV-1 using a TCID₅₀ of 100 by IP injection under metofane-induced anesthesia. Two weeks after HIV-1 challenge, mice were killed, and single cell suspensions were prepared from peritoneal lavage, spleen, and peripheral blood and processed for immununostaining, flow cytometry analysis, and PCR. All experiments were performed in a biosafety level 3 facility and protocols were approved by the Biosafety Committee, Research Ethics Board, and Health Sciences Animal Welfare Committee at the University of Alberta.

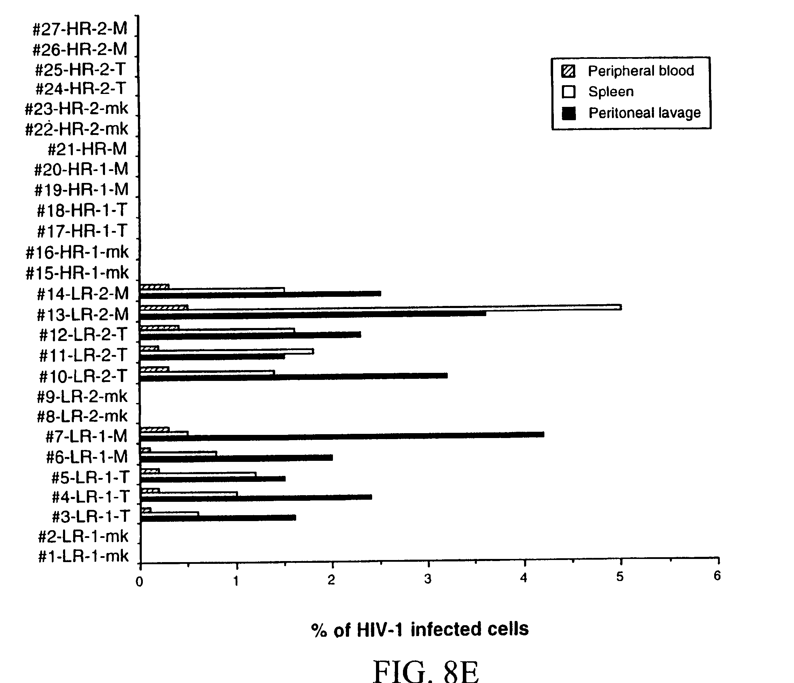

The cells harvested from the infected reconstituted mice were analyzed by flow cytometry using anti-mouse major histocompatibility complex class I antibody (H-2K^{d}) and anti-human CD45, CD3, CD4, and CD8 antibodies as described in Ex.11. At the same time, HIV-1 infection was examined by a sensitive single-cell immunohistochemical staining method using either HIV-positive patients' sera or a monoclonal anti-p24 antibody as described in Ex.15. Representative immunostaining results are shown in Fig. 8. For the results shown in Fig. 8, all mice were killed 4 weeks after reconstitution and single cell suspensions were prepared from the three different organ compartments for immunostaining. Figs. 8a and 8b show HIV-1-positive immunostaining of peritoneal lavage and splenocytes, respectively, of mice reconstituted from a LR individual. Figs. 8c and 8d show HIV-1-negative immunostaining of peritoneal lavage and splenocytes, respectively, of mice reconstituted from a HR individual.

The results of immunostaining demonstrated that all of the 10 hu-PBL-SCID/bg mice reconstituted with PBLs from control individuals were infected by either T cell or macrophage tropic HIV-1, at frequencies of about 1-4% of cells from the peritoneal lavage (Fig. 8a), 0.8-5% of splenocytes (Fig. 8b), and 0.1-0.5% of peripheral blood mononuclear cells. In contrast, all nine mice reconstituted with PBLs from the two HR, HIV-uninfected individuals were negative for the HIV immunostaining (Fig. 8c and 8d, peritoneal lavage and spleen, respectively), but one of the negative mice was positive in the spleen for HIV-1 DNA sequences by PCR analysis, suggesting that infection was established at the time of challenge. These results are summarized in Fig. 8e. In Fig. 8e, the solid, hatched and shaded bars represent cells from peritoneal lavage, spleen and peripheral blood, respectively. Reconstitution of mice with PBLs from the LR-1 donor (#1-7), the LR-2 donor (#8-14), the HR-1 donor (#15-21) and the HR-2 donor (#22-27) were confirmed by ELISA for human Ig and FACS analysis for the human CD45 leuckocyte marker. In Fig. 8e, "M-tropic" indicates infection with the macrophage tropic HIV_{NLAD8} and "T-tropic" indicates infection with the T-cell tropic HIV_{NL4-3}. The *in vivo* challenge experiments were repeated once with eight mice reconstituted for each donors' PBLs and similar results were obtained.

### Example 17 - Cell-Mediated Immune Responses In HR Individuals

To investigate if the *in vivo* protection is due at least in part to HIV-1-specific, cell-mediated immune responses in the HR, seronegative individuals, a convenient and very sensitive ELISPOT assay was modified to determine the fraction of IFN-γ producing cells after HIV antigen presentation. IFN-γ is produced by T cells and natural killer cells activated by antigens and is the key mediator of CD4⁺ Th1 cell development [Wenner et al. (1996) J. Immunol. 156:1442].

### (a) ELISPOT Analysis of IFN-γ Production

ELISPOT was performed as described [Miyahira et al. (1995) J. Immunol. Methods 181:45] with the following modifications. To quantify the IFN-γ producing cells, a 96-well nitrocellulose-bottomed plate (Multiscreen-HA, Millipore) was coated with 75 µl per well mouse anti-human IFN-γ (10 µg/ml), Pharmingen) at room temperature overnight. HIV-1-infected PBLs were used as stimulators in the ELISPOT assay. Infection was confirmed by RT assay and by immunohistochemical staining (ranged 3-5% of HIV-1-positive). No difference was observed between HR and LR individuals in either RT or HIV antigen expression in the infected PBLs. HIV-1-infected PBLs were treated with mitomycin C (5 µg/ml) for 2.5 hr, washed, and resuspended in RPMI medium 1640 containing 10% FBS and 20 units/ml interleukin 2 (Boehringer Mannheim), and seeded in the anti-IFN-γ-coated 96-well plate at 1 x 10⁵ per well as target cells (T). Frozen unstimulated autologous PBLs, which were thawed and used as effector cells (E), were added to the wells at E/T ratio of 0.4:1, 2:1, and 10:1 1 in triplicates, and incubated in RPMI medium 1640 containing 10% FBS and 20 units/ml IL-2 at 37°C, 95% O₂/5% CO₂ for 24 hr.

After overnight incubation, the wells were washed four times using PBS-Tween 20 (PBS-T, 0.05%), blocked with 20% FBS in PBS-T at room temperature for 15 min, and incubated with 100 µl of biotinylated-mouse-anti-human IFN-γ (Pharmingen, 2.5 µg/ml in PBS-T) at 4°C overnight Each well was then washed four times using PBS-T and incubated with peroxidase-labeled streptavidin (Caltag, South San Francisco, CA) in PBS/T at room for 1 hr. IFN-γ-producing cells were detected as purple brown spouts after DAB (Sigma) and 0.3% NiCl₂ staining. The wells washed four times with double distilled H₂O and air-dried, and were the number of IFN-γ producing cells was counted using a video-imaging (Appligene, Strasbourg, France), and computer analysis system. Control samples were prepared using the same effector cells plus autologous PBLs that were not infected with HIV-1.

### (b) PBLs From HR Donors, But Not LR Donors Contain HIV-Specific Responders

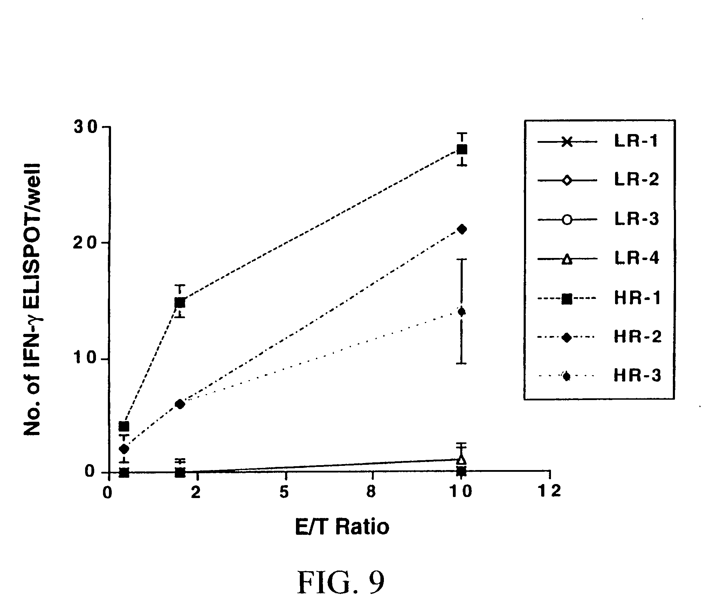

Four LR and three HR donors' PBLs, unstimulated, were incubated with mitomycin C-treated, HIV-infected autologous PBLs in a 96-well nitrocellulose-bottomed plate that had been coated with an anti-IFN-γ antibody. Production of IFN-γ was detected 24 hr later using a secondary horseradish peroxidase-conjugated anti-IFN-γ antibody. The results are summarized in Fig. 9. In Fig. 9, the number of IFN-γ ELISPOT/well is plotted against the E/T ratio. The Xs, open diamonds, open circles and open triangles show the results using PBLs from donors LR-1, LR-2, LR-3 and LR-4, respectively. The solid squares, solid diamonds and solid circles show the results using PBLs from donors HR-1, HR-2 and HR-3, respectively. HIV-1-specific-ELISPOT are shown as the mean ± SE in triplicate wells after subtracting the background from controls.

As shown in Fig. 9, no HIV-specific IFN-γ producing responders were observed in PBLs of four LR donors except for donor 4, who showed a frequency of IFN-γ producing cells at one in 1 x 10⁶ PBLs at the highest E/T ratio. In contrast, the existence of HIV-specific responders in the PBLs of HR seronegative donors were easily detected; at the E/T ratio of 10:1, the frequencies of HIV-specific IFN-γ producing cells were 14,21, and 28 per 10⁶ cells for the three HR donors, which was significantly different (P < 0.05, t test) from that of the LR donors. Even at an E/T ratio of 0.4:1, HIV-specific IFN-γ-producing cells were still detectable in all three HR donors.

The contribution of the individual subset of immune effector cells in this HIV-specific reaction was further studied by depleting CD4, CD8, or CD56 cells from PBLs of two of these HR donors prior to the *in vitro* ELISPOT assay. *In vitro* lymphocyte depletion was conducted as follows. For the depletion of CD4, CDB, and CD56 cells, thawed PBLs were incubated with mouse anti-human CD4-, CD8-, CD56-, or mouse IgG₁-labeled BioMag magnetic beads (PerSeptive Diagnostics, Cambridge, MA) at 50 beads per cell on ice for 30 min. The depletion was carried out by sorting the cells on a magnet for 5 min two times using mouse IgG₁ sorting as control. After depletion, cells were washed with culture medium and seeded in triplicates with the HIV-1-infected autologous PBLs as target cells in an anti-IFN-γ-coated 96-well plate for 24 has described above. To evaluate the contribution of individual subpopulation of lymphocytes in the IFN-γ production, comparison was based on the initial number of PBL used. Thus, the E/T ratio (10:1) was based on the starting PBL number before depletion. Results of this quantitative analysis indicated that CD8 T cells contributed significantly to the production of IFN-γ (48-71%).

### Example 18 - In Vivo Resistance to HIV-1 Infection Is CD8 T Cell-Dependent

The above information was used to further delineate the mechanism of *in vivo* protection from HIV-1 in the reconstituted SCID/bg mice. SCID/bg mice were reconstituted with PBLs from LR and HR donors which were either depleted of CD8 T cells or untreated. The reconstituted mice were then inoculated with the macrophage-tropic HIV-1_{NLAD8} and the ability to infect the human PBLs *in vivo* was examined. PBLs from HR donor 1 were employed in this study. As CD4 cells are the targets of HIV-1 infection, they were not depleted in this study.

### (a) Depletion of CD8 T Cells in hu-PBL-SCID/bg Mice

CD8 T cells were depleted as described [Baskar et al. (1993) Proc. Natl. Acad. Sci. USA 90:5687] with the following modification. Anti-human CD8 antibody was purified from ascites of OKT8 hybridoma (ATCC CRL 8014) using protein A affinity column (ImmunoPure Plus, Pierce). Mice were injected IP with 20 µg of the purified antibody five times in total: 2 days before PBL reconstitution, at the time of PBL injection, and at 1, 2, and 3 weeks after PBL injection. To confirm the depletion of CD8 T cells, hu-PBL-SCID/bg mice were killed, and the lymphocytes were analyzed by FACS analysis as described in Ex. 11. The cells were stained with PE-anti-mouse-H-2K^{d} (Pharmingen) and FITC-anti-human-CD45 (Anti-HLe-1, Becton Dickinson) or with PE-anti-human-CD4 and FTTC-anti-human CD8 (Becton Dickinson) for 30 min on ice followed by three washes with immunostaining buffer (PBS containing 0.1% FBS and 0.02% NaN₃). Isotype-matched PE-mouse-Ig and FITC-mouse Ig were used as controls.

### (b) In Vivo Resistance To HIV Infection is Dependent Upon CD8 T Cells

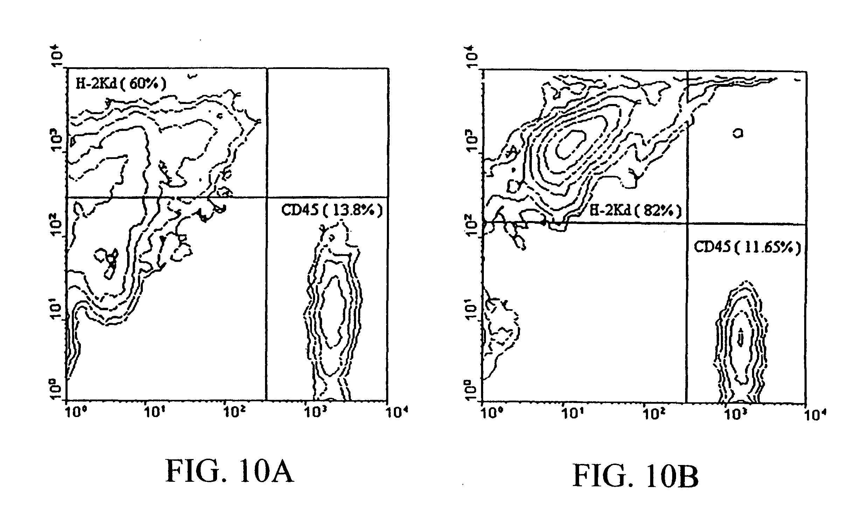

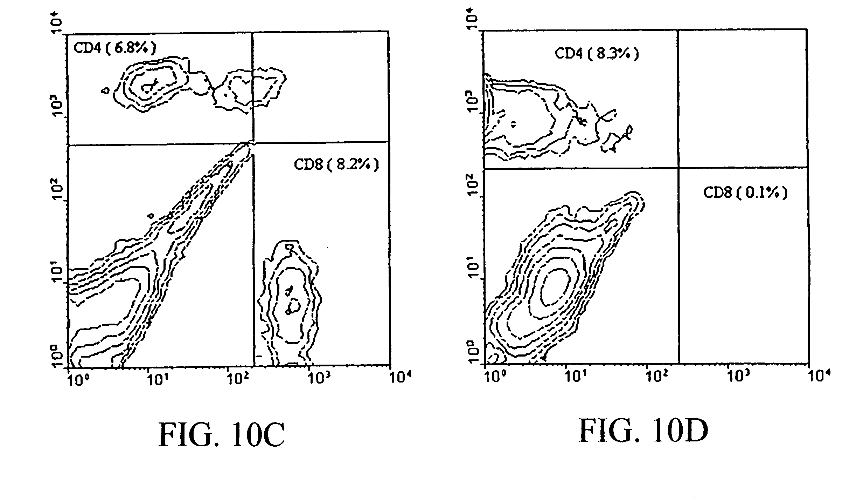

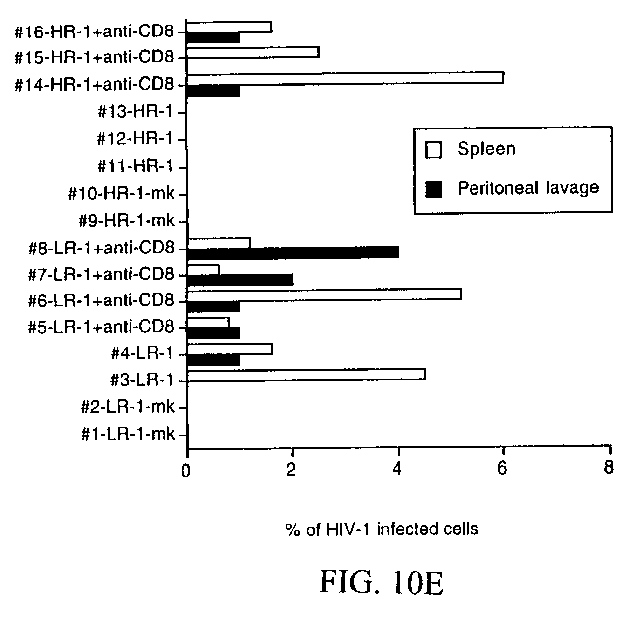

Reconstitution and CD8 depletion were confirmed by FACS analysis 4 weeks later. Representative FACS analysis are shown in Fig. 10. Figs. 10a and 10c show FACS analysis of splenocytes from the reconstituted hu-PBL-SCID/bg mouse #11 immunostained with anti-mouse H-2Kd, anti-human CD45, anti-human CD4 or anti-human CD8 as depicted. Figs. 10b and 10d show FACS analysis of splenocytes derived from the reconstituted hu-PBL-SCID/bg mouse #14, after anti-CD8 antibody treatment.

As shown in Fig. 10, in mice receiving no antibody, the following percentages of human lymphocyte subsets were observed: 14% human CD45 cells (Fig. 10a), 7% human CD4 cells, and 8% human CD8 cells (Fig. 10c) in spleens 4 weeks after reconstitution. In mice treated with anti-CD8 antibody, there were similar numbers of CD45 cells in the spleen (12%, Fig. 10b), but the CD8 cells were virtually absent (0.1%, Fig. 10d). These mice were inoculated with the macrophage-tropic HIV-1_{NLAD8} on day 14 after reconstitution and killed 14 days later for analysis as described above. Results of this study are summarized in Fig. 10e.

In Fig. 10e, the percentage of HIV-1 infected cells in the spleen (open bars) and in the peritoneal lavage (solid bars) 2 weeks after virus challenge is shown. The mock infected (mk) mice were not treated with anti-CD8 antibody.

The results shown in Fig 10e demonstrate that mice reconstituted with PBLs from HR donor 1 (HR-1) continued to be resistant to HIV-1 infection as long as CD8 cells were present. However, after CD8 cell depletion, similarly reconstituted mice became susceptible to HIV-1 infection (Fig.10e, hu-PBL-SC1D/bgmice #14, #15, and #16). These results demonstrate that *in vivo* protection from HIV-1 infection in the reconstituted mice is mediated by human CD8 lymphocytes.

The difference observed between the *in vivo* shown in this example and the *in vitro* studies (Ex. 16) indicates that anti-HIV-1 protective immunity cannot be properly assessed by tissue culture infection studies or by the *in vitro* assays for cell-mediated immunity alone. The hu-PBL-SCID/bg model of the present invention provides a suitable model for the assessment of anti-HIV-1 protective immunity. Results of the *in vivo* HIV-1 challenge suggest that the cell-mediated immunity developed in these HR subjects also protects against alternative strains of HIV-1 since the challenge strains used in this study, T cell and macrophage tropic HIV-1, are unrelated to the endogenous HIV-1 strains in the corresponding partners of these HR individuals under investigation.

Other studies have demonstrated the development of cell-mediated immune responses in HIV-1 exposed humans [Rowland-Jones et al. (1995) Nat. Med. 1:59; Taylor (1994), *supra;* Clerici et al. (1992) J. Infect. Dis. 165:1012; Rowland-Jones et al. (1993) Lancet 341:860; Pinto et al. J. Clin. Invest. 96:867; Clerici et al. (1994) J. Am. Med. Assoc. 271:42 and Shearer and Clerici (1996) Immunol. Today 17:21]. Studies of the simian AIDS models have also suggested that cell-mediated immune responses play an important role in protection against simian immunodeficiency virus infection [Salvato et al. (1994) J. Med. Primatol. 23:125]. It is possible that some genetic factors including HLA haplotype may also contribute to the development of resistance to HIV-1 infection [Haynes *et al., supra;* Kaslow et al. (1996) Nat. Med. 2:405 and Detels et al. (1996) AIDS 10:102]. However, the two HR subjects who participated in the above hu-PBL-SCID/bg mouse challenge study do not share common HLA haplotypes.

*In vivo* protective immunity against HIV-1 has also been hypothesized to involve the functions of CD8 T cells that secret anti-HIV chemokines [Cocchi et al. (1995) Science 270:1811]. In addition, CD4 T cells from 2 of 25 frequently exposed but uninfected individuals have recently been shown to have relative resistance to HIV-1 infection [Paxton et al. (1996) Nat. Med. 2:412]; subsequent genotype analysis of these two individuals by PCR and sequencing indicates that they have homozygous defects in the CCR-5 loci that encode the macrophage-tropic HIV-1 coreceptor [Liu et al. (1996) Cell 86:367]. Results of PCR analysis of six multiply-exposed, uninfected individuals including the two HR subjects in our *in vivo* study showed that one is heterozygous and five are wild type. Both of the HR subjects participating in the *in vivo* studies have homozygous wild-type CCR-5 loci. This is consistent with the result that PBLs of these two HR subjects were susceptible to macrophage-tropic HIV-1 infection *in vitro.*

While the *in vitro* ELISPOT analysis for the HIV-1-reactive effector functions showed that when exposed to autologous HIV-1-infected cells, the Th 1-associated, HIV-1 antigen-specific IFN-γ production was higher from CD8 cells than from CD4 or natural killer cells in the HR donors, the possible contribution of CD4 cells to resistance due to the loss of double positive cells (CD4⁺ CD8⁺) in the depletion assay cannot be excluded. Thus, it is possible that these two HR, HIV-seronegative individuals participating in the *in vivo* SCID/beige mouse challenge study may also have protective immunity developed in their CD4 T cell population. Further studies are necessary to characterize the possible mechanisms of CD4 or natural killer cell-mediated resistance.

The above results demonstrate the usefulness of the *in vivo* hu-PBL-SCID/bg mouse model for evaluating the protective immunity of individuals exposed to HIV-1. The SCID/bg mice can be reconstituted with human PBLs and infected with HIV-1 at a near 100% success rate. In addition, this model is useful for the assessment of the immune status of HIV-1 vaccinees.

### Example 19 - Attenuated Leishmania Cell Vaccines

The biggest frustration in the search for recombinant proteins which might serve as vaccine against a variety of diseases is the fact that recombinant proteins expressed in *Escherichia coli* trigger immune response which are not protective to mammals against the parent eukaryotic and viral pathogens. A major drawback of the bacterial expression system is the inability of bacteria to carry out post-translational modifications such as glycosylation and phosphorylation which eukaryotic proteins require in order to assume their natural configuration and become biologically active [Zhang et al. (1995) Nuc. Acids Res. 23:4073]. Immunization based on proteins expressed in *E. coli* fail to trigger protective immune response because such proteins do not assume the configuration of the wild type protein. In search for a new vaccine, one must always look at the history of vaccinology. The most successful vaccinations have been performed by the use of attenuated or inactivated pathogens. Although safety concerns make such an approach unsuitable for HIV vaccines, it is important to understand why vaccines comprising attenuated pathogens work: these vaccines work because they contain proteins whose configuration are identical with that of the expected pathogens.

Even if a protein has received proper post-translational modifications (*e.g*., glycosylation and phosphorylation), it will still fail to trigger a protective immune response if the mode of processing and presentation of the vaccine protein to the immune system is not appropriate. This is due to the fact that the mode of processing and presentation of a protein by antigen presenting cell (APC) plays a decisive role in the specific recruitment of distinct T Cell subsets into an immune response [Schirmbeck et al. (1994) J. Immunol. 152:1110]. Two alternative antigen processing pathways are now known [Schirmbeck *et al., supra* and McDonnell and Askari (1996) New Engl. J. Med. 334:42]. The first alternative is the exogenous pathway through which antigen-presenting cells take up extracellular proteins by either endocytosis or phagocytosis. MHC class II molecules in the endoplasmic reticulum pass through the Golgi apparatus and enter acidified endosomes in which the foreign protein has been fragmented into peptides. The MHC-peptide complex is then brought to the cell surface, where it can be recognized by helper T cells (CD4⁺). The second alternative is the endogenous pathway by which intracellular proteins in the cytosol are cleaved into short peptides (composing 8 to 10 amino acids). The peptides enter the endoplasmic reticulum with transport-associated proteins (TAP1 and TAP2) and bind there to MHC class I molecules. After binding, the complex is transported through the Golgi apparatus to the cell surface, where it can be recognized by cytotoxic T cells (CD8⁺). Hence, immunization with soluble protein antigens stimulates CD4⁺ T cells which are not protective in HIV. In fact, the development of HIV vaccines has been unsuccessful because most of the vaccines are composed of viral subunits expressed in *E. coli* and are presented through exogenous antigen presentation pathway. The route of immunization (oral, rectal, vaginal, etc.) and the pathway of antigen presentation (intracellular vs. extracellular) may hold the key to success in HIV vaccination [Miller and McGhee (1996) Nat. Med. 2:751 and Bloom (1996) Science 272:1888].

In Ex. 17-18 above, it was shown that *in vivo* protective immunity against HIV in multiple-exposed, seronegative individuals is cell-mediated rather than antibody-mediated. Therefore, the vaccination strategy for induction of immunity against HIV should be directed toward the production of cytotoxic T lymphocytes (CTL) vaccines which are capable of providing heterologous protection against different strains [McDonnell and Askari, *supra* and Ulmer (1993) Science 259:1745]. CD8⁺ CTLs which are protective in HIV infection can only be triggered if the immunogenic protein is generated within the antigen presenting cell like the macrophages (McDonnell and Askari, *supra*). The feasibility of using direct DNA injection technology that elicits immune responses similar to those induce by live attenuated vaccines has been demonstrated in animals models (Ulmer, *supra*), but clinical applications of this form of technology remain elusive (McDonnell and Askari, *supra*).

To provide a HIV vaccine capable of presenting HIV proteins in their native conformation and in the context of an APC, cysteine protease and HIV proteins were expressed in *Leishmania* cells to generate an attenuated *Leishmania* cell capable of acting a vector to transfer HIV proteins to macrophages. Biologically active cysteine protease was produced using this system. Other investigators have reported the production of biologically active human p53, murine INF-γ, *Leishmania* gp63 and *Plasmodium yoelii* circumsporozoite proteins in *Leishmania* [Zhang, *et al., supra;* Tobin et al. (1993) J. Immunol. 150:5059; Liu and Chang (1992) Proc. Natl. Acad. Sci. 89:4991; and Wang et al. (1995) Mol. Biochem. Parasitol. 69:139]. Therefore, it is possible to produce eukaryotic proteins which assume natural configuration in this system.

The impact of over-expressing cysteine proteases in *Leishmania* pathogenesis was examined. When *Leishmania* cells over-producing cysteine proteases were injected into susceptible BALB/c mice as a vaccine, the parasite failed to infect mice subsequently challenged with *Leishmania;* thus, the modified *Leishmania* cells functioned as an attenuated vaccine. This data suggests that cysteine proteases are powerful immunogens which provide protection against a variety of species of *Leishmania.* Since *Leishmania* infects macrophages only, this system provides a unique opportunity to generate proteins of interest (*i.e*., from pathogens) within the macrophages (APC) without causing disease. This system is used to provide a vector for delivering vaccines against HIV so that immunogenic proteins whose configuration is identical with that of the expected pathogens (*e.g*., wild type HIV strains) can be delivered and be delivered within the APC to humans. Such a protein would be processed and presented to the immune system through the endogenous pathway resulting in the production of cytotoxic T lymphocytes which are protective against HIV.

To provide *Leishmania* cell vaccines expressing HIV proteins, sequences of HIV *gag-pol*/*env, env* and/or *gag* genes are cloned downstream of the *Leishmania* cysteine protease gene as bi- or tri-cistronic constructs using pX or pALT-Neo vectors (kindly provided by Drs. Steven Beverely and Dyann Wirth, respectively; both located at Harvard Medical School, Boston, MA). pALT-Neo contains the *neo* gene inserted between two α-tubulin intergenic sequences and permits the stable expression of the neo gene in *Leishmania* cells. Expression of such bi- or tri-cistronic constructs have been successfully demonstrated (see Exs. 2, 9 and 10). Expression of gag-pol and env proteins from a replication defective gag-pol-env construct (*e.g*., pHP-1) has been successfully demonstrated (Ex. 20). Plasmids comprising the gene(s) of interest are used to transfect *Leishmania major* cells as described [Laban et al. (1990) Nature 343:572]. Briefly, plasmid DNA is linearized and introduced into the promastigote forms of *Leishmania* cells by electroporation. The electroporated cells are then grown in selective medium (*e.g*., in medium containing 200 µg/ml G418 when the plasmid employed contains the *neo* gene).

The transformed *Leishmania major* cells are examined for their ability to confer protection against HIV. Scid/bg mice reconstituted with human PBLs (*i.e*., hu-PBL-SCID/bg mice) are infected with *Leishmania* cells transformed with a cysteine protease-HIV-1 gag-pol/env construct. Two to four weeks later, mice are challenged with HIV-1. Evaluation of protection against infection is conducted two weeks after HIV-1 challenge. Splenocytes from the reconstituted mice are isolated and HIV-specific CMI (cell-mediated immunity) is analyzed using a modified ELISPOT method Ex.17). The cysteine protease gene will provide protection against *Leishmania* while the gag-pol/env gene will provide protection against HIV. Attenuated *Leishmania* vaccines which demonstrate the ability to reduce and/or prevent HIV infection and/or which induce CMI directed against HIV in the animal model of the present invention are then employed for the vaccination of humans.

### Example 20 - Construction of HIV-1 DNA Vaccines

Historically, the most effective vaccines have been live attenuated or inactivated pathogens. Attenuated viruses also have shown promise in AIDS research; the most potent protection of an AIDS-like virus (SIV) in a primate has been observed using attenuated SIV in macaques [Daniels et al. (1992) Science 258:1938]. Therefore, attenuated HIV vaccines may provide effective HIV immunity in humans. In this example, DNA constructs capable of generating viral particles containing an attenuated HIV genome are provided. In addition, DNA constructs comprising a replication-defective HIV provirus which are employed as DNA vaccines are provided.

### (a) Attenuated Replication-Competent Proviral DNA Vaccines

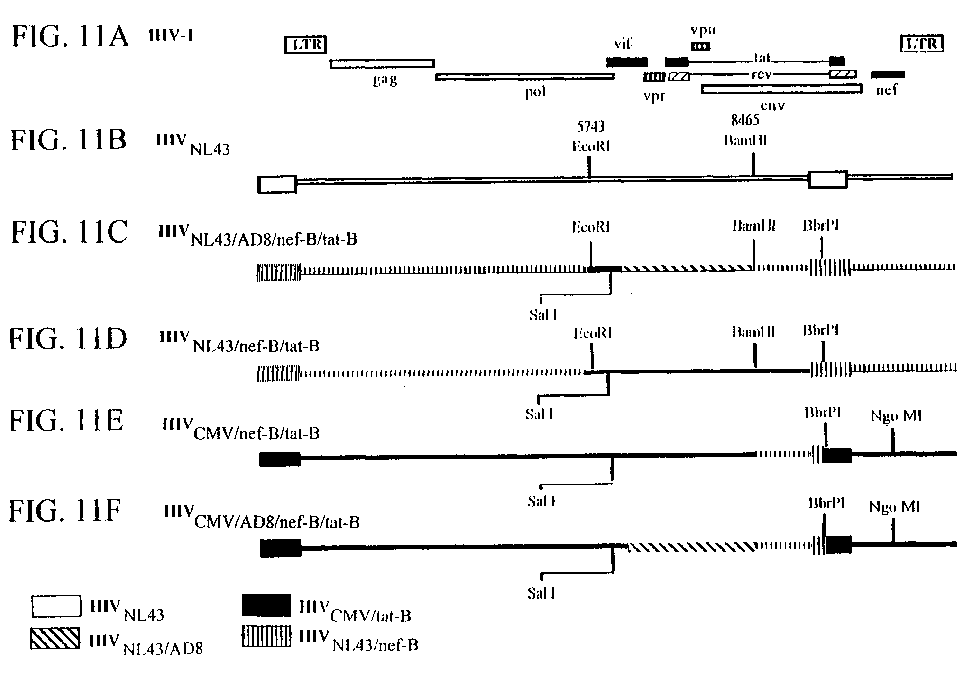

Several HIV clones containing mutations in the *tat* and nef genes were constructed to provide live attenuated HIV vaccines; these tat- nef- HIV clones are replication-competent. These HIV constructs may be employed as proviral DNA vaccines (*i.e*., naked DNA) or employed as a vaccine comprising viral particles. Figures 11A-E provide schematics showing the organization of the HIV-1 genome (11A) and the organization of the HIV_{NL4-3}, (11B) HIV_{NL43/AD8/tat-B/nef}-_{B} (11C), HIV_{NL43/nef-B/tat-B} (11D), HIV_{CMV/nef-B/tat-B} (11E) and HIV_{CMV/AD8/nef-B/tat-B} (11F) clones. In Figure 11, the open boxes indicate sequences derived from HIV_{NL4-3}, the solid boxes indicate sequences derived from HIV_{CMV/tat-B'} the shaded boxes indicate sequences derived from HIV_{NL43/AD8} and the striped boxes indicate sequences derived from HIV_{NL43/nef-B}.

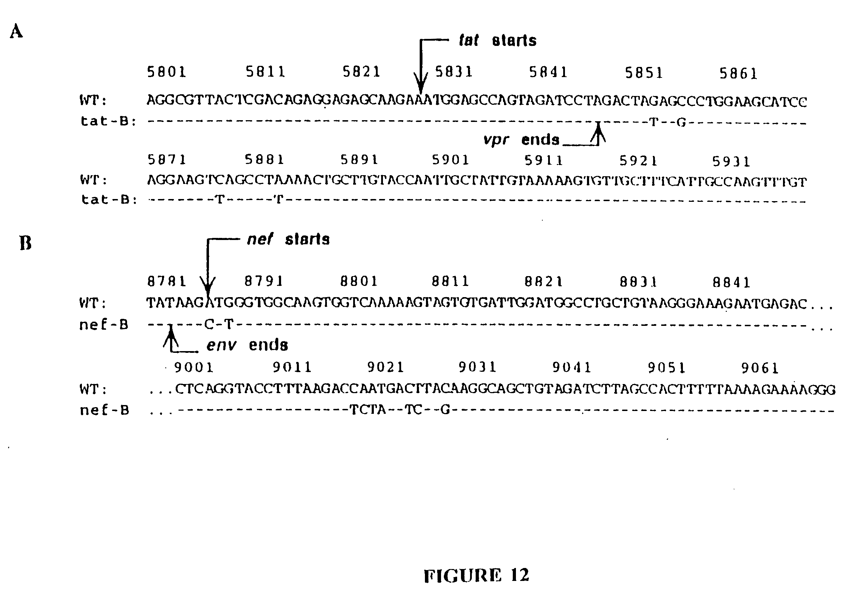

Before discussing the construction of specific tat- nef- HIV clones, a number of precursor HIV clones are described. The proviral HIV-1 clone HIV_{NL4-3} provides a full-length HIV-1 genome. HIV_{ADA} is a virus that shows macrophage tropism which localizes to the gp120 domain of the envelope protein. Sequences encoding the envelope protein of HIV_{ADA} were cloned into HIV_{NL4-3} to yield HIV_{AD8}, a virus which has demonstrated macrophage tropism (the sequence of the *env* gene from HIV_{ADA} is available from the GenBank database under accession number M60472). HIV_{CMV/tatB} is an HIV_{NL4-3} derivative containing two alterations (Chang and Zhang, *supra*). The first alteration consists of the insertion of the CMV-IE enhancer into the 3' and 5' LTR regions of HIV and a deletion of the Sp1 element from the HIV LTR. The second mutation consists of the integration of three stop codons between nt 5851 and 5851 of the *tat* gene [numbered according to the system of Myers et al. (1992) Human Retroviruses and AIDS, Los Alamos National Laboratory, Los Alamos, NM]. This tat- HIV clone has been shown to be efficiently expressed in several cell lines (Chang and Zhang, *supra*). HIV_{NL43/nef-B} is an HIV_{NL4-3} derivative in which three stop codons were introduced in the nef gene between nt 8781 and 9031. In addition to the introduction of the three stop codons, this clone contains an engineered *Hind*III site at nt 8784 and two engineered *Xba*I sites at nt 9019 and nt 9025. Figures 12A-B provide schematics showing a portion of the wild type HIV-1 sequence as well as the tat-B (Fig. 12A; wild-type sequence provided in SEQ ID NO:26) and nef-B mutations (Fig. 12B; wild-type sequence provided in SEQ ID NOS:27 and 28). HIV_{NL43/AD8/tat-B}, a Tat-virus, was constructed by ligation of the following three fragments: 1) a ~5.7 kb *Bam*HI*-Eco*RI fragment isolated from HIV_{NL43} which provides the *env*-vector backbone-gag-pol region (nt 8465 to 5743) and 2) a ~0.6 kb *Eco*RI-*Asp*718 fragment isolated from HIV_{CMV/tat-B} which provides the *tat* gene (nt 5743 to 6343) and 3) a~2.1 kb *Asp*718-*Bam*HI fragment isolated from HIV_{ADS} which provides the *env* gene (nt 6343 to 8465).

### (i) Construction of HIV_{NL43/AD8/tat-B/nef-B}

HIV_{NL43/AD8/tat-B/nef-B} was constructed by ligation of the following two fragments: 1) a -5.7 kb *Bam*HI*-Eco*RI fragment isolated from HIV_{NL43/nef-B} which provides the *env*-vector backbone-gag-pol region (nt 8465 to 5743) and 2) a ~2.7 kb *Eco*RI*-Bam*HI fragment isolated from HIV_{NL43/AD8/tat-B} which provides the *tat-env* region. HIV_{NL43/AD8/tat-B/nef-B} does not produce functional Tat or Nef proteins and is macrophage tropic (AD8 *env* gene).

### (ii) Construction of HIV_{NL43/nef-B/tat-B}

HIVNL43/nef-B/tat-B was constructed by ligation of the following two fragments: 1) a ~5.7 kb BamHI EcoRI fragment isolated from HIV_{NLA3ref-B} which provides the *env*-vector backbone-*gag-pol* region (nt 8465 to 5743) and 2) a ~2.7 kb *Eco*RI*-Bam*HI fragment isolated from HIV_{CMV/tat-B} which provides the *tat-env* region. HIV_{NL43/nef}-_{B/tat-B} does not produce functional Tat or Nef proteins and is T cell tropic (NL4-3 *env* gene).

### (iii) Construction of HIV_{CMV/nef-B/tat-B}

HIV_{CMV/nef-B/tat-D} was constructed by ligation of the following three fragments: 1) a ~5.7kb*Ngo*MI-SalI fragment isolated from HIV_{CMV/tat-B} which provides the vector backbone-*U3-gag-pol-tat* region (nt 10349 to 5785), 2) a ~3.6 kb *Sal*I*-Bbr*PI fragment isolated from HIV_{NL43/nef-B/tat-B} which provides the *tat-env-nef* region (nt 5785 to 9365) and 3) a ~0.98 kb *Bbr*PI*-Ngo*MI fragment isolated from HIV_{CMV/tat-B} which provides U5 (nt 9365 to 10349). HIV_{CMV/nef-B/tat-B} does not produce functional Tat or Nef proteins and is T cell tropic (NL4-3 *env* gene).

### (iv) Construction of HIV_{CMV/AD8/nef-B/tat-B}

HIV_{CMV/AD8/nef-B/tat-B} was constructed by ligation of the following three fragments: 1) a~5.7kd *Ngo*MI-*Sal*I fragment isolated from HIV_{CMV/tat-B} wich provides the vector backbone-*U3-gag-pol-tat* region (nt 10349 to 5785), 2) a ~3.6 kb *Sal*I*-Bbr*PI fragment isolated from HIV_{CMV/tat-B} which provides the *tat-env-nef* region (nt 5785 to 9365) and 3) a ~0.98 kb *Bbr*PI*-Ngo*MI fragment isolated from HIV_{CMV/tat-B} which provides U5 (nt 9365 to 10349). HIV_{CMV/AD8/nef-B/tat-B}does not produce functional Tat or Nef proteins and is macrophage tropic (AD8 *env* gene).

The tat- nef- HIV clones described in sections i-iv above may be employed as naked DNA vaccines or alternatively, these DNA constructs may be transfected into either PBLs (HIV_{NL43/nef-B/tat-B} and HIV_{CMV/nef-B/tat-B}) or macrophages (HIV_{NL43/AD8/tat-B/nef-B} and HIV_{CMV/AD8/nef-B/tat-B}) and virus may be isolated as described in Ex. 15 and the attenuated viruses may be employed as vaccines. The tat- nef- HIV clones described above will establish an infection in the host and induce both humoral and cell-mediated immune responses which will then control or limit the infection induced by these viruses. Ex. 21 below provide protocols for the evaluation of these attenuated viruses and proviral DNA vaccines in hu-PBL-SCID/bg mice.

### (b) Replication-Defective HIV-1 Packaging Vector Vaccines

Expression vectors useful for the packaging of HIV-derived vectors are provide useful HIV-1 DNA vaccines as these constructs express viral structural genes but lack the ability to generate viral RNA capable of being packaged into viral particles.

### (i) Construction of Expression Vectors Capable of Synthesis of All Viral Structural Genes But Lacking Packaging Signals

pHP-1 lacks the primer binding site (PBS), polypurine tract (PPT), 3' LTR and most of the untranslated 5' leader sequences including the conventional retroviral packaging signal (Ψ) and the major HIV-1 splice donor (SD) site. pHP-1 contains all HIV structural genes except for the *nef* gene and thus is capable of expressing the vast majority of the viral proteins. pHP-1 provides a provirus capable of mimicking HIV-1 infection in term of the viral proteins expressed yet this virus cannot be packaged into viral particles. Further the mutations introduced into the pHP-1 provirus greatly reduce the possibility that wild-type HIV virus will be produced by recombination. Thus, pHP-1 provides an excellent HIV DNA vaccine.

pHP-1 was constructed as follows. First, the Tat-responsive enhancer promoter CMV-TATA-TAR fragment (approximately 400 bp) was isolated from dl.kB/Spl-CMV-TATA-TAR HIV [Chang et al. (1993) J. Virol. 67:743] by *Brp*I-*Hind*III digestion, and cloned into *Eco*RV-*Bam*HI digested pSP72 (Promega) via a linker providing HindIII and *Bam*HI cohesive sites which contains a modified gag AUG with Kozak translation initiation context and a major splice donor site of Rous sarcoma virus. This linker was formed by annealing the following oligonucleotides: 5'AGCTTGGTCGCCCGGTGGATCAAGACOGGTAGCCGTCATAAAGGTGATTTCGTCG-3' (SEQ ID NO:29) and 5'-GATCCGACGA AATCACCTTT ATGACGGCT ACCGGTCTTG ATCCACCGGGCGACCA-3' (SEQ ID NO:30). This first subclone was called pSP-CMV-TAR-SD.

Secondly, the gag coding sequence was obtained by PCR from pNL4-3 (a full-length HIV-1 plasmid) using a 5' primer [5'-CGGGATCCAC CATGGGTGCG AGAGCGTC-3' (SEQ ID NO:31)] and a 3' primer downstream of the *Sph*I site in the gag gene [5'-ATCCTATTTG TTCCTGAAGG-3' (SEQ ID NO:32)]. The PCR product was digested with *Bam*HI-*Sph*I (∼660 bp) and this fragment was ligated with *Bam*HI-*Sph*I digested pSP-CMV-TAR-SD to obtain pSP-CMV-TAR-SD-dl.gag.

Next the poly-A minus subclone pHP-dl.pA was constructed by ligating the following three fragments: a 1112 bp *Hpa*I-*Sph*I fragment isolated from pSP-CMV-TAR-SD-dl.gag (contains the promoter-TAR-SD-dl.gag), a 7922 bp *Sph*I-*Xho*I fragment (dl.gag-pol-env-gpt) of pNLgpt, and a plasmid vector backbone provided by *Eco*RV-*Xho*I digested pBS-KS(-) (Stratagene).

Lastly, pHP-1 was made by the following ligation: *Not*I-*Xho*I (9059 bp) of pHP-dl.pA containing dl.CMV-TATA-TAR-SD-gag-pol-env-gpt, a 422 bp poly-A site from *Xho*I-*Pst*I digested pREP9 (Invitrogen), and *Not*I-*Pst*I digested pBS-KS(-). The sequence of pHP-1 (12.494 kb) is provided in SEQ ID NO:33; this sequence begins at the promoter at the half-*Bbr*PI site.

To see whether an replication-competent HIV (RC-HIV) recombinant could be generated, human TE671 cells (ATCC CRL 8805) were transfected with pHP-1 DNA and the transfected cells were cocultured with the human lymphoma cell line MT4 for 7 days. MT4 cells (NIH AIDS Research and Reference Reagent Program of National Institutes of Health, Bethesda, MD) are an HTLV-1 transformed human CD4+ lymphoma cell line, which are very sensitive to HIV-1 infection. No infectious HIV-1 was detected after a 2 month coculture indicating the absence of RC-HIV production in cells containing pHP-1. To detect the synthesis of HIV-1 proteins, cell lysates were prepared and analyzed by Western blotting in comparison with a wild type HIV-1 construct, pNL4-3. Results of this study showed that the level of viral proteins synthesized by pHP-1 was similar to that of the wild type pNL4-3.

Analysis of reverse transcriptase (RT) activity in the transfected culture supernatants indicated that the level of active RT production was reduced 40% for pHP-1 compared with the wild type construct pNL4-3. The expression of Gag-Pol function indicates that tat and rev are functional. Thus, the artificially engineered splice donor (SD) site in the pHP-1 construct, which is unrelated to HIV sequences, works like the wild type SD site (*i.e*., allowing partition of spliced and unspliced mRNAs into the cytoplasm).

pHP-1 contains the bacterial gpt gene and lacks the HIV *nef* gene. The *gpt* gene is removed from pHP-1 to create pHP-2 as follows. pHP-1 is digested with *Bam*HI and *Sph*I and the~7 kb *Bam*HI (nt 8870 of pHP-1)-*Sph*I (nt 1842 of pHP-1) fragment is isolated. pHP-1 is digested with *BamHI* and *Hind*III and the 322 bp *Bam*HI (nt 8870 of pHP-1)-*Hind*III (nt 9192 of pHP-1) fragment is isolated. pHP-1 is digested with *Xho*I and *Eco*RI and the 431 bpXhoI (nt 9764 of pHP-1)-EcoRI (nt 10195 of pHP-1) fragment is isolated. pHP-1 is digested with *Eco*RI and *Sph*I and the ∼1.8 kb *Eco*RI (nt 10195 of pHP-1)*-Sph*I (nt 1842 of pHP-1) fragment is isolated. These four fragments are ligated together along with an *Hind*III*-Xho*I adapter to generate pHP-2. pHP-2 lacks the gpt gene of pHP-1 and also lacks the HIV *nef* gene.

The *nef* gene is inserted into pHP-2 to generate pHP-3. pHP-3 contains all HIV structural genes and like pHP-1 and pHP-2 is replication-defective. The *nef* gene is isolated by PCR amplification using primers having a *Hind*III (5' primer) or a *Xho*I site (3' primer) in addition to sequences complementary to the *nef* gene. The amplified PCR product is digested with *Hind*III and *Xho*I and ligated with the four fragments derived from pHP-1 used to generate pHP-2 (described above) to generate pHP-3. The *nef* PCR product is thus used in place of the *Hind*III-*Xho*I adapter used to generate pHP-2 in the construction of pHP-3.

The proviruses contained on pHP-1, pHP-2 and pHP-3 can be placed on vector backbone having a kanamycin-resistance gene rather than an ampicillin-resistance gene using standard techniques in order to satisfy requirements for FDA approval of DNA vaccines.

The ability of pHP-1, pHP-2 and/orpHP-3 DNA (or kanamycin-resistant derivatives) to function as effective HIV vaccines is evaluated in the hu-PBL-SCID/bg mice as described in Ex. 21 below.

### Example 21 - Evaluation of Candidate Vaccines In Hu-PBL-SCID/beige Mice

This example describes the use of hu-PBL-SCID/bg mice for the evaluation of vaccines designed to induce immunity against human pathogens. When working with mice exposed to attenuated or wild type HIV, extreme caution is taken to avoid exposure to HIV. Double gloves are worn and mice are handled (prior to anaesthesia) using long forceps. All procedures related to HIV are conducted in a Bio-safety level 3 facility; all blood and tissue samples transferred out of the level 3 facility are kept in a bio-safety container.

### (a) Reconstitution Of Hu-PBL-SCID/bg Mice

Hu-PBL-SCID/bg mice are prepared as described in Ex. 3 or as follows. Human PBLs are isolated by density gradient centrifugation on Ficoll-Hypaque (Ex. 15). The separated PBL are resuspended in PBS at a concentration of 4 x 10⁷/ml. Freshly isolated PBLs (*i.e*., within 2 hr of isolation) are preferred for the reconstitution of SCID/bg mice. Frozen PBLs and buffy coat preparations (as provided by blood banks) permitted to stand at room temperature for ∼24 hr demonstrate a reduced efficiency of reconstitution compared to freshly isolated PBLs.

SCID/bg mice are injected i.p. with 2 x 10⁷ human PBLs in 0.5 ml PBS. The injected animals are placed in clean cages on a folded piece of paper towel (on the cage bottom) to avoid inhalation of the bedding and are checked daily.

### (b) Confirmation Of Reconstitution By ELISA

Three to four weeks after the injection of human PBLs, blood is collected from the mouse tail using a microtainer (Beckton Dickinson). The blood sample is tested for human immunoglobulin (hu-Ig) by ELISA. Animals with hu-Ig levels > 100 µg/ml are considered successfully reconstituted. Animals with hu-Ig levels > 100 µg/ml at the time they are to be vaccinated (*i.e*., injected with a preparation comprising a candidate vaccine such as an attenuated HIV or a HIV DNA preparation) may be employed for the evaluation of vaccines. Typically, animals receive the first dose of vaccine 1-2 weeks after injection of human PBLs.

### (c) Immunization of Hu-PBL-SCID/bg Mice With Heterologous DNA Vaccines

Hu-PBL-SCID/beige mice are generated as described in section a) above. One week after injection of the human PBLs, the reconstituted mice are anesthetized with metofane. Once the surgical plane of anesthesia is reached, the animal is placed in a restraint device and immunized with either 1) 0.5 ml of attenuated HIV (*e.g*., HIV _{NLA3/AD8/tat-B/nef-B}) (TCID50 = 50 to 100, in AIM-V serum-free medium) by i.p. injection, or 2) 0.2 ml (containing approximately 30 µg DNA in saline) of naked proviral DNA (*e.g*., pHP-1) via intramuscular injection. After the first injection, the same immunization process is be repeated twice, with three days between immunizations.

One week after the last injection of the attenuated live or DNA HIV vaccine, the animal is challenged with wild type HIV isolates by i.p. injection (0.5 ml, TCID50 = 100) under anesthesia with metofane. Following immunization and challenge with wild type HIV-1, blood samples (0.1 ml) are collected weekly from the mouse tail to monitor HIV-1 infection by immunohistochemical staining using an anti-p24 monoclonal antibody (prepared from hybridoma 183-H12-5C; available from the NIH AIDS Research and Reference Reagent Program of National Institutes of Health); the staining is carried out as described in Ex. 15. At various time points, the animals are sacrificed by cervical dislocation after induction of anesthesia. At the time of sacrifice, the blood, peritoneal lavage and spleen are collected for examination of HIV-1 infection. The splenocytes are also used to investigate HIV-1-specific CMI responses by ELISPOT (Ex. 17).

### (d) Evaluation of Attenuated HIV Vaccines and Anti-HIV Gene Therapy Vectors in Humanized SCID/beige Mice

Human immune function in established in SCID/beige mice using human fetal hematopoietic tissues (liver, thymus, lymph node or bone marrow). These humanized animals provide models for the evaluation of HIV vaccines and anti-HIV gene therapy strategies.

### (i) Establishment of Humanized SCID/beige Mice

Human fetal tissue is prepared for transplantation as follows. Human fetal tissue (HIV-negative), 17-24 gestational weeks, is obtained from Advanced Bioscience Resources, Inc. (Alameda, CA), and placed in a sterile 50 ml tube containing RPMI 1640 medium supplemented with 10% fetal calf serum (FCS), 50 units/ml penicillin and 50 µg/ml streptomycin. The samples are shipped on wet ice and received within 36 hours. Tissue and blood is tested for the presence of HIV using a PCR assay. In addition, for future use, the organ fragments or dissociated cells are frozen in 10% DMSO and 40% FCS.

SCID/bg mice are anesthetized with metofane. A 1.5 cm² area on the back of each mouse is shaved using a # 22 blade and this area is swabbed with povidone iodine. A 1-cm flank incision is made to expose the right or/and left kidneys.

Human fetal liver and thymus are cut with a sharp blade into small pieces (1 mm³). About 10 pieces of each tissue are implanted under the kidney capsule using a 16 or 18-gauge trocar. The wound is sutured and metal clips are secured over the wound to ensure the healing

Blood samples (0.1 ml) are collected from mouse tails monthly to confirm the presence of human immune cells (as described in Ex. 11; additional markers such as CD25 may also be employed) and human Ig in the peripheral blood (by ELISA) in the transplanted SCID/bg mice. Once the immune system of the SCID/beige mice is successfully humanized, they are used for vaccine evaluation. Alternatively, transduced human hematopoietic stem cells (CD34+ cells) carrying anti-HIV genes can be injected i.p. into the humanized SCID/beige mice. Hematopoietic stem cells are transduced (*i.e*., infected) using Moloney murine leukemia virus (MoMLV)-based vectors or HIV-based vectors comprising anti-HIV genes (*e.g*., anti-HIV ribozymes and intracellular single chain antibody genes). Examples of MoMLV-based vectors comprising anti-HIV genes are provided in co-pending application Serial No. 08/336,132 the disclosure of which is herein incorporated by reference.

### (ii) Evaluation of Attenuated HIV Vaccine and Anti-HIV Gene Therapy Vectors in the Humanized SCID/beige Mice

The humanized (*i.e*., transplanted) SCID/bg mice are anesthetized with metofane and placed in a restraint device and injected with attenuated HIV vaccines as described above (virus in AIM-V medium or DNA in saline). After three injections of the attenuated HIV vaccines (spaced 3-7 days apart), the animals are challenged with wild type HIV-1 by i.p. injection (0.5 ml, TCID50 = 100) under the anesthesia with metofane. Following wild type HIV injection, blood samples are collected weekly from the tail under the anesthesia with metofane to check for HIV infection.

At various time points (before death), the animals are sacrificed by cervical dislocation post-anesthesia. At the same time, the blood, peritoneal lavage and spleen are collected for the examination of HIV infection and *in vitro* CMI by ELISPOT as described in Ex. 17.

It is clear from the above that the present invention provides animal models suitable for the evaluation of HIV vaccines and anti-HIV immunity. In addition, the present invention provides attenuated live HIV vaccines and DNA vaccines for the induction of anti-HIV immunity.

### Example 22 - Computer-Assisted ELISPOT Assay

### (a) Materials and Methods

### (i) Cells

AA-2 cell line is derived from the WIL-2 human splenic EBV+ B-lymphoblastoid line which expresses high levels of CD4 [Chaffee et al. (1988) J. Exp. Med. 168:605-621]. AA-2 was obtained from AIDS Research and Reference Reagent Program (NIH, Bethesda, Maryland) and maintained at 37°C, 5% CO₂ in 1640 RPMI supplemented with 10% fetal calf serum (FCS), 0.1 mg/ml Penicillin-Streptomycin, 2 mM L-glutamine, 1 mM non-essential amino acids, and sodium pyruvate. Human peripheral blood lymphocytes (PBLs) were obtained from healthy or repeatedly HIV-1-exposed seronegative donors and separated by FICOLL gradient as previously reported [Chang et al. (1995) Virology 211:157-169] and frozen in liquid nitrogen until use.

### (ii) Separation of Subsets of Human Lymphocytes

PBLs were thawed at 37°C and used only if the viability was higher than 90%. They were incubated with mouse anti-human CD4-, CD8-, CD56- or mouse IgG₁-labeled BIOMEG magnetic beads (PerSeptive Biosystems, Framingham, MA) at 50 beads per cell on ice for 30 min. Cell depletion was carried out by sorting the cells on a magnet for 5 min twice using mouse IgG, sorting as a control. After sorting, cells were washed with medium and seeded with either stimulator or target cells in each experiment as described below.

### (iii) Preparation of Stimulators and Target Cells

AA-2 cells were used as stimulator or target cells in the allogeneic reaction. For stimulation, AA-2 was treated with 5 µg/ml of mitomycin for 2.5 h , washed with phosphate-buffered saline (PBS) three times, and seeded at 5 X 10⁴ cells per well in a round-bottomed 96-well plate together with various subsets of T cells prepared as described above from 2 X 10² to 5 X 10³ per well in triplicates in RPMI 1640 containing 10% FCS and 20 U/ml of IL-2 (Boehringer Mannheim Canada) in a total volume of 200 µl. The cells were incubated at 37°C, 5% CO₂ for 3 days before being used in the ELISPOT assay. To prepare target cells for the detection of HIV-1-specific effector function, PBLs from either healthy or HIV-1-exposed but uninfected donors were treated with PHA (5 µg/ml) for 2 days and infected with wild-type HIV-1 at a multiplicity of infection doses of 0.2 for 3 h. After infection, PBLs were incubated in RPMI 1640 containing 10% FCS in the presence of IL-2 (20 U/ml) for 5 days. HIV-1 infection was confirmed by reverse transcriptase assay as reported previously [Chang et al. (1995) Virology 211:157-169]. The infected PBLs were frozen in liquid nitrogen for later use. PHA-stimulated but uninfected PBLs of the same individual were used as a negative control.

### (iv) ELISPOT Assay

The ELISPOT assay several steps which were carried out over four consecutive days as shown in Figure 13: coating the plates with antibody, capturing the cells of interest, binding of secondary antibody, enzyme conjugation, and color development [Czerkinsky et al. (1988) J. Immunol. Methods 110:29-36] with the following modifications. For determining the allogeneic response, 1X10⁵ mitomycin C-treated AA-2 cells in 100 µl of RPMI 1640 containing 10% FCS and IL-2 (20 U/ml) were added to each well. Meanwhile, PBLs which had been incubated for 3 days with stimulators were also transferred from the round-bottomed 96-well plate to the nitrocellulose-well plate in 100 µl and incubated at 37°C, 5% CO₂ for 24 h, using AA-2 or PBLs alone as controls.

HIV-1-specific immune responses were studied using unstimulated, frozen PBLs (4 X 10⁴ to 1 X 10⁶) which were obtained from seronegative HTV-1-exposed, high-risk or un-exposed, low-risk donors. In each well, the thawed effector PBLs were incubated with 1 X 10⁵ mitomycin C-treated autologous HIV-infected PBLs in a total volume of 200 µl for 24 h. The uninfected, autologous PBLs were used as controls.

### (v) Enumeration by Computerized Video-Imaging

The dried nitrocellulose 96-well plates were placed on top of an illumination box and images were captured using a Cohu 4910 series monochrome CCD camera which was connected to an Appligene computer using program "The Imager" version 2.03 (Appligene, Plesonton, Georgia). The image was optimized by adjusting contrast, greyscale, and exposure time and saved in a picture file format (TIFF) before it was analyzed using the shareware "NIH Image Version 1.55." The readings of 6-24 wells can be captured and saved in one file and processed together. When retrieved, the image was sharpened and/or smoothed by adjusting the greyscale to obtain the best possible resolution. The image was then converted to a binary (black and white) format by setting up a proper threshold. The individual well was selected and the spots were counted using the "analysis particles" option in the program. The size of particles can be pre-defined so that background spots of different size will be automatically excluded.

### (b) Results of Computer-Assisted ELISPOT assay

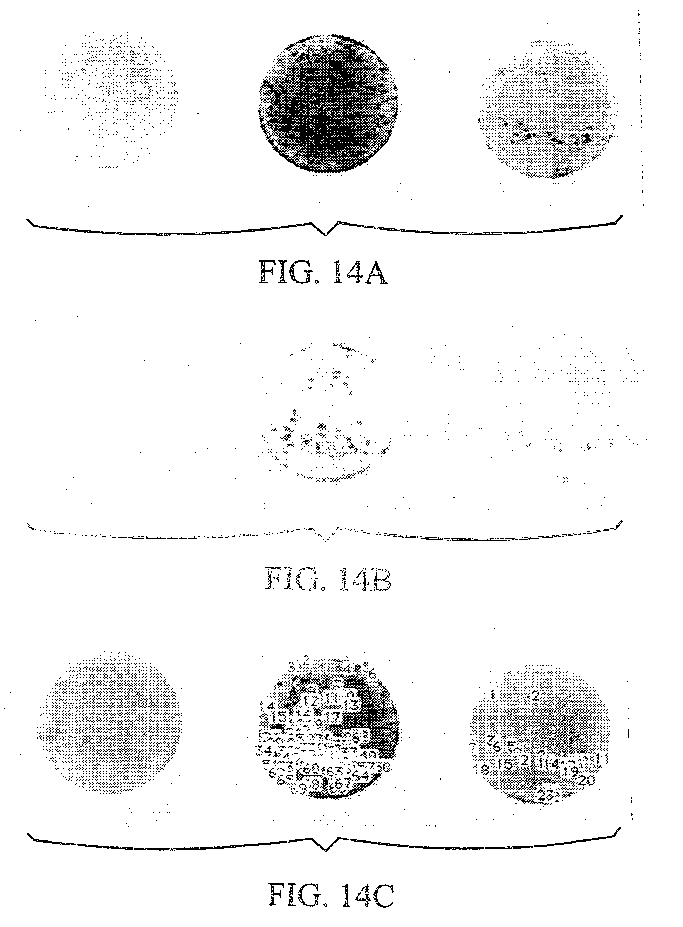

In a normal immunocompetent individual, the allogeneic antigen stimulation induces a strong cell-mediated immune response, accompanied with marked IFN-γ production. This reaction serves as a good model for the ELISPOT assay. To induce an allogeneic response, unmatched donors' PBLs were mixed with irradiated AA-2 as target cells for three days, and the frequency of the IFN-γ producing cells in the PBL population was determined by ELISPOT assay as described in the Materials and Methods. The spots formed by IFN-γ secreting cells in the nitrocellulose-bottomed 96-wells when viewed under the stereomicroscope were dark purple in the center and a little lighter in the surrounding region, similar to previous observation [Czerkinsky et al. (1988) J. Immunol. Methods 110:29-36]. These ELISPOTs were distinct and easily distinguished from the artifact spots which were smaller, denser and mostly uniformed. When the computer video-image system was employed, the smaller artifacts did not appear from the background at the magnification used. Figure 14A shows a print-out of a fine-tuned computer ELISPOT image processed by the free shareware "NIH Image." The well on the left had the target AA-2 alone. The wells in the center and on the right contained 1,000 and 200 PBLs respectively, along with the target AA-2. Using a binary format (Figure 14B), the image of ELISPOTs is easily viewed as black spots and can be counted automatically (Figure 14C). The number of IFN-γ producing cells (IPC) counted using this system is similar to that determined with the aid of a stereomicroscope. This processing system avoids the tedious and unpleasant enumeration experience under the microscope. Most importantly, as both the darkness and the size of the ELISPOT can be pre-defined using the analysis program, the result uniformly eliminates unwanted artifacts. When compared with manual microscopic counting, the computerized analysis is accurate and objectively distinguishes the smaller artificial spots from those real ones. In addition to the "NIH Image" program, any picture-oriented program can be used to retrieve and print the captured image.

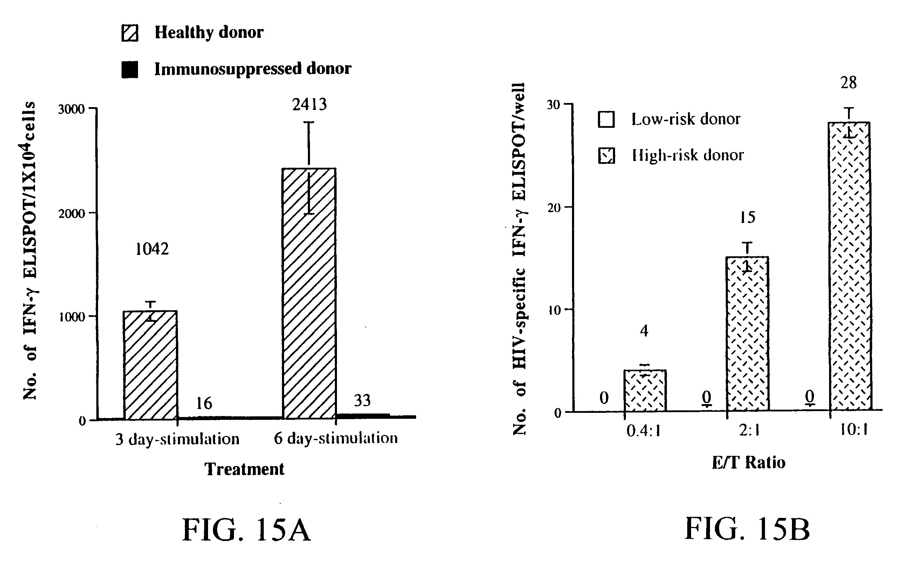

A representative ELISPOT assay for non-specific allogeneic response using mixed lymphocytes is shown in Figure 15A. The PBLs of a healthy donor reacted to AA-2 showing over 1,000 IPCs in 1X10⁴ PBLs after a 3 day-stimulation and the number of IPCs doubled after a 6-day-stimulation (Fig. 15A). In contrast, the allogeneic immune response of a cancer patient, who had been under chemotherapy two weeks before the test, was 50 times less than that of the healthy donor. This result demonstrates that ELISPOT is a simple, fast and yet sensitive protocol for assessing the frequency of a strong, non-specific immune response.

To demonstrate the specificity and sensitivity of the ELISPOT assay, the frequency of HIV-1 specific IPCs in an HIV-1 repeatedly exposed (high-risk), but uninfected individual, compared to that in a control, low-risk individual, using the autologous HIV-1 -antigen presenting cells as the target cells was studied. The secretion of IFN-γ upon encountering HIV-1 antigen presenting cells would be an indication of prior exposure to HIV-1 [Jassoy et al. (1993) J. Virology 67:2844-2852]. The target cells were prepared by infecting autologous PBLs from the corresponding individuals as described in the Materials and Methods section above. The results of this study indicate that the PBLs from the low risk donor do not contain any HIV-1-specific responders in up to 1X10⁶ T cells (10:1 ratio, Figure 15B). In contrast, the PBLs from the high-risk donor have a precursor frequency of 28 HIV-1-specific responders per 1X10⁶ lymphocytes without *in vitro* stimulation (Figure 15B). The existence of HIV-1-specific IPCs in the high-risk donor is in concordance with previous reports that high-risk seronegative individuals have developed HIV-1-specific CTL responses [Clerici et al. (1992) J. Inf. Dis. 165:1012-1019 and Rowland-Jones et al. (1995) Nature Medicine 1:59-64]. While HIV-1 specific CTLs can be detected in HIV-1-infected individuals without *in vitro* stimulation [Walker et al. (1987) Nature 328:345-348], HIV-1-specific CTLs and the precursor frequencies in high-risk individuals were demonstrated only after *in vitro* stimulation [Aldhous et al. (1994) Clin. Exp. Immunol. 97-61-67, Clerici et al. (1992) J. Inf Dis. 165:1012-1019 and Rowland-Jones et al. (1995) Nature Medicine 1:59-64]. This result thus demonstrates that the ELISPOT assay is sensitive enough to detect a weak antigen-specific immune response and to determine the precursor frequency without an additional step of *in vitro* stimulation.

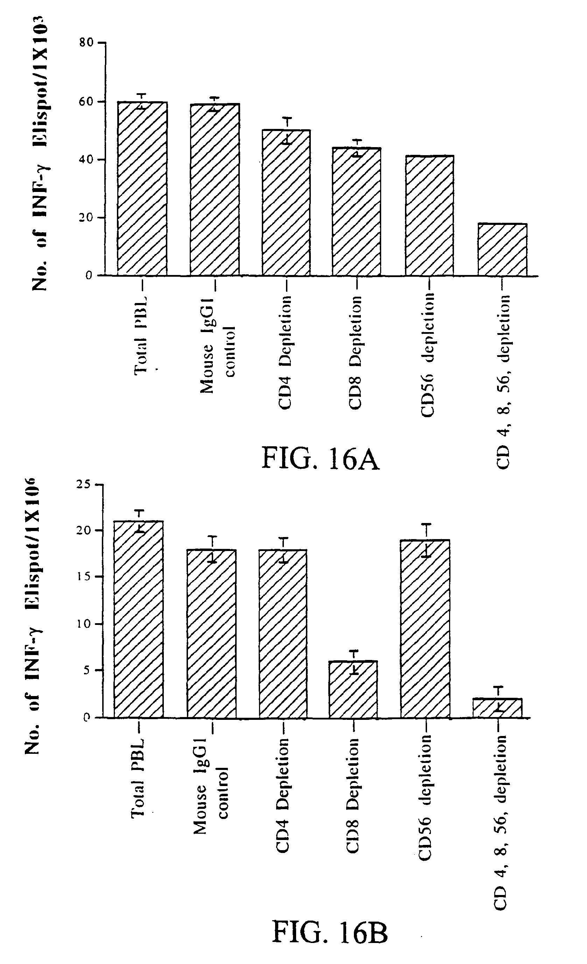

To study the roles of individual T cell subsets in the allogeneic and HIV-1-specific immune reactions, CD4, CD8, CD56 (NK), or all of the three subsets of lymphocytes were depleted in separate experiments and an ELISPOT assay was performed as described. As shown in Figures 16A and 16B, all three cell types contributed, albeit to different extents, to IFN-γ production in both the allogeneic and HIV-1-specific reactions. In the allogeneic reaction, NK cells accounted for about 30% , CD8 26%, and CD416% of the IFN-γ production. In the HIV-1 exposed but uninfected individual, however, CD8 cells contributed the most, up to 71%, to the HIV-1 specific IFN-γ secretion and CD4 and NK cells only accounted for 14% and 10% of total IFN-γ production respectively. Together these studies demonstrated a quick analysis for the quantitation of IFN-γ producing cells in antigen-specific and non-specific immune reactions.

The combination of an immuospot assay with a computer-assisted data acquisition and processing system simplifies the experimental procedures and can be used for the clinical or experimental assessment of general and specific immune responses, especially when dealing with a large sample size. Immunospot assays according to the present invention can also be used more conveniently in many immunological studies, including new applications such as establishing bioassays for miscellaneous interleukin functions [Chang and Cui, in preparation]. The methods of the present invention can also be used with two-color ELISPOT methods [Czerkinsky et al. (1988) J. Immunol. Methods 115:31-37] using a color image capture system or a pair of appropriate filters with the monochrome image system.

### SEQUENCE LISTING

(1) GENERAL INFORMATION:
(i) APPLICANT INFORMATION:

| | |
|---|---|
| Applicant Name(s): | Chang, Lung-Ji |
| Street Address: | 3102 N.W. 57^{th} Terrace |
| City: | Gainesville |
| State/Province: | Florida |
| Country: | US |
| Postal Code/Zip: | 32606-6939 |
| Phone number: | (352) 392-3315 |

(ii) TITLE OF INVENTION: Animal Model For Evaluation Of Vaccines
(iii) NUMBER OF SEQUENCES: 33
(iv) CORRESPONDENCE ADDRESS:
(A) ADDRESSEE: Saliwanchik, Lloyd & Saliwanchik
(B) STREET: 2421 N.W. 41^{st} Street, Suite A-1
(C) CITY: Gainesville
(D) STATE: FL
(E) COUNTRY: USA
(F) ZIP: 32606-6669

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US (B) FILING DATE: (C) CLASSIFICATION:

(vii) PRIOR APPLICATION DATA:
(A) APPLICATION NUMBER: US 08/848,760
(B) FILING DATE: 01-MAY-1997

(vii) PRIOR APPLICATION DATA:
(A) APPLICATION NUMBER: US 08/838,702
(B) FILING DATE: 09-APR-1997

(viii) ATTORNEY/AGENT INFORMATION:
(A) NAME: Pace, Doran R.
(B) REGISTRATION NUMBER: 38,261
(C) REFERENCE/DOCKET NUMBER: CHG-101

(ix) TELECOMMUNICATION INFORMATION:
(A) TELEPHONE: (352) 375-8100
(B) TELEFAX: (352) 372-5800

(2) INFORMATION FOR SEQ ID NO:1:
(i) SEQUENCE CHARACTERISTICS:
   (A) LENGTH: 6145 base pairs
   (B) TYPE: nucleic acid
   (C) STRANDEDNESS: double
   (D) TOPOLOGY: linear
(ii) MOLECULE TYPE: DNA (genomic)
(xi) SEQUENCE DESCRIPTION: SEQ ID NO:1:
   GAATTCATAC CAGATCACCG AAAACTGTCC TCCAAATGTG TCCCCCTCAC ACTCCCAAAT     60
   TCGCGGGCTT CTGCCTCTTA GACCACTCTA CCCTATTCCC CACACTCACC GGAGCCAAAG    120
   CCGCGGCCCT TCCGTTTCTT TGCTTTTGAA AGACCCCACC CGTAGGTGGC AAGCTAGCTT    180
   AAGTAACGCC ACTTTGCAAG GCATGGAAAA ATACATAACT GAGAATAGAA AAGTTCAGAT    240
   CAAGGTCAGG AACAAAGAAA CAGCTGAATA CCAAACAGGA TATCTGTGGT AAGCGGTTCC    300
   TGCCCCGGCT CAGGGCCAAG AACAGATGAG ACAGCTGAGT GATGGGCCAA ACAGGATATC    360
   TGTGGTAAGC AGTTCCTGCC CCGGCTCGGG GCCAAGAACA GATGGTCCCC AGATGCGGTC    420
   CAGCCCTCAG CAGTTTCTAG TGAATCATCA GATGTTTCCA GGGTGCCCCA AGGACCTGAA    480
   AATGACCCTG TACCTTATTT GAACTAACCA ATCAGTTCGC TTCTCGCTTC TGTTCGCGCG    540
   CTTCCGCTCT CCGAGCTCAA TAAAAGAGCC CACAACCCCT CACTCGGCGC GCCAGTCTTC    600
   CGATAGACTG CGTCGCCCGG GTACCCGTAT TCCCAATAAA GCCTCTTGCT GTTTGCATCC    660
   GAATCGTGGT CTCGCTGTTC CTTGGGAGGG TCTCCTCTGA GTGATTGACT ACCCACGACG    720
   GGGGTCTTTC ATTTGGGGGC TCGTCCGGGA TTTGGAGACC CCTGCCCAGG GACCACCGAC    780
   CCACCACCGG GAGGTAAGCT GGCCAGCAAC TTATCTGTGT CTGTCCGATT GTCTAGTGTC    840
   TATGTTTGAT GTTATGCGCC TGCGTCTGTA CTAGTTAGCT AACTAGCTCT GTATCTGGCG    900
   GACCCGTGGT GGAACTGACG AGTTCTGAAC ACCCGGCCGC AACCCTGGGA GACGTCCCAG    960
   GGACTTTGGG GGCCGTTTTT GTGGCCCGAC CTGAGGAAGG GAGTCGATGT GGAATCCGAC   1020
   CCCGTCAGGA TATGTGGTTC TGGTAGGAGA CGAGAACCTA AAACAGTTCC CGCCTCCGTC   1080
   TGAATTTTTG CTTTCGGTTT GGAACCGAAG CCGCGCGTCT TGTCTGCTGC AGCGCTGCAG   1140
   CATCGTTCTG TGTTGTCTCT GTCTGACTGT GTTTCTGTAT TTGTCTGAAA ATTAGGGCCA   1200
   GACTGTTACC ACTCCCTTAA GTTTGACCTT AGGTCACTGG AAAGATGTCG AGCGGATCGC   1260
   TCACAACCAG TCGGTAGATG TCAAGAAGAG ACGTTGGGTT ACCTTCTGCT CTGCAGAATG   1320
   GCCAACCTTT AACGTCGGAT GGCCGCGAGA CGGCACCTTT AACCGAGACC TCATCACCCA   1380
   GGTTAAGATC AAGGTCTTTT CACCTGGCCC GCATGGACAC CCAGACCAGG TCCCCTACAT 1440
   CGTGACCTGG GAAGCCTTGG CTTTTGACCC CCCTCCCTGG GTCAAGCCCT TTGTACACCC 1500
   TAAGCCTCCG CCTCCTCTTC CTCCATCCGC CCCGTCTCTC CCCCTTGAAC CTCCTCGTTC 1560
   GACCCCGCCT CGATCCTCCC TTTATCCAGC CCTCACTCCT TCTCTAGGCG CCGGAATTCC 1620
   GATCTGATCA AGAGACAGGA TGAGGATCGT TTCGCATGAT TGAACAAGAT GGATTGCACG 1680
   CAGGTTCTCC GGCCGCTTGG GTGGAGAGGC TATTCGGCTA TGACTGGGCA CAACAGACAA 1740
   TCGGCTGCTC TGATGCCGCC GTGTTCCGGC TGTCAGCGCA GGGGCGCCCG GTTCTTTTTG 1800
   TCAAGACCGA CCTGTCCGGT GCCCTGAATG AACTGCAGGA CGAGGCAGCG CGGCTATCGT 1860
   GGCTGGCCAC GACGGGCGTT CCTTGCGCAG CTGTGCTCGA CGTTGTCACT GAAGCGGGAA 1920
   GGGACTGGCT GCTATTGGGC GAAGTGCCGG GGCAGGATCT CCTGTCATCT CACCTTGCTC 1980
   CTGCCGAGAA AGTATCCATC ATGGCTGATG CAATGCGGCG GCTGCATACG CTTGATCCGG 2040
   CTACCTGCCC ATTCGACCAC CAAGCGAAAC ATCGCATCGA GCGAGCACGT ACTCGGATGG 2100
   AAGCCGGTCT TGTCGATCAG GATGATCTGG ACGAAGAGCA TCAGGGGCTC GCGCCAGCCG 2160
   AACTGTTCGC CAGGCTCAAG GCGCGCATGC CCGACGGCGA GGATCTCGTC GTGACCCATG 2220
   GCGATGCCTG CTTGCCGAAT ATCATGGTGG AAAATGGCCG CTTTTCTGGA TTCATCGACT 2280
   GTGGCCGGCT GGGTGTGGCG GACCGCTATC AGGACATAGC GTTGGCTACC CGTGATATTG 2340
   CTGAAGAGCT TGGCGGCGAA TGGGCTGACC GCTTCCTCGT GCTTTACGGT ATCGCCGCTC 2400
   CCGATTCGCA GCGCATCGCC TTCTATCGCC TTCTTGACGA GTTCTTCTGA GCGGGACTCT 2460
   GGGGTTCGAA ATGACCGACC AAGCGACGCC CAACCTGCCA TCACGAGATT TCGATTCCAC 2520
   CGCCGCCTTC TATGAAAGGT TGGGCTTCGG AATCGTTTTC CGGGACGCCG GCTGGATGAT 2580
   CCTCCAGCGC GGGGATCTCA TGCTGGAGTT CTTCGCCCAC CCCGGGCTCG ATCCCCTCGC 2640
   GAGTTGGTTC AGCTGCTGCC TGAGGCTGGA CGACCTCGCG GAGTTCTACC GGCAGTGCAA 2700
   ATCCGTCGGC ATCCAGGAAA CCAGCAGCGG CTATCCGCGC ATCCATGCCC CCGAACTGCA 2760
   GGAGTGGGGA GGCACGATGG CCGCTTTGGT CGACCCGGAC GGGACGCTCC TGCGCCTGAT 2820
   ACAGAACGAA TTGCTTGCAG GCATCTCATG AGTGTGTCTT CCCGTTTTCC GCCTGAGGTC 2880
   ACTGCGTGGA TGGAGCGCTG GCGCCTGCTG CGCGACGGCG AGCTGCTCAC CACCCACTCG 2940
   AGGGCGTGCA GCGCTGCAGA GGCCGAGTGC AGAACTGCTC CAAAGGGACC TCAAGGCTTT 3000
   CCGAGGGACA CTAGGCTGAC TCCATCGAGC CAGTGTAGAG ATAAGCTTAT CGATTAGTCC 3060
   AATTTGTTAA AGACAGGATA TCAGTGGTCC AGGCTCTAGT TTTGACTCAA CAATATCACC 3120
   AGCTGAAGCC TATAGAGTAC GAGCCATAGA TAAAATAAAA GATTTTATTT AGTCTCCAGA 3180
   AAAAGGGGGG AATGAAAGAC CCCACCTGTA GGTTTGGCAA GCTAGCTTAA GTAACGCCAT 3240
   TTTGCAAGGC ATGGAAAAAT ACATAACTGA GAATAGAGAA GTTCAGATCA AGGTCAGGAA 3300
   CAGATGGAAC AGCTGAATAT GGGCCAAACA GGATATCTGT GGTAAGCAGT TCCTGCCCCG 3360
   GCTCAGGGCC AAGAACAGAT GGAACAGCTG AATATGGGCC AAACAGGATA TCTGTGGTAA 3420
   GCAGTTCCTG CCCCGGCTCA GGGCCAAGAA CAGATGGTCC CCAGATGCGG TCCAGCCCTC 3480
   AGCAGTTTCT AGAGAACCAT CAGATGTTTC CAGGGTGCCC CAAGGACCTG AAATGACCCT 3540
   GTGCCTTATT TGAACTAACC AATCAGTTCG CTTCTCGCTT CTGTTCGCGC GCTTCTGCTC 3600
   CCCGAGCTCA ATAAAAGAGC CCACAACCCC TCACTCGGGG CGCCAGTCCT CCGATTGACT 3660
   GAGTCGCCCG GGTACCCGTG TATCCAATAA ACCCTCTTGC AGTTGCATCC GACTTGTGGT 3720
   CTCGCTGTTC CTTGGGAGGG TCTCCTCTGA GTGATTGACT ACCCGTCAGC GGGGGTCTTT 3780
   CATTTGGGGG CTCGTCCGGG ATCGGGAGAC CCCTGCCCAG GGACCACCGA CCCACCACCG 3840
   GGAGGTAAGC TGGCTGCCTC GCGCGTTTCG GTGATGACGG TGAAAACCTC TGACACATGC 3900
   AGCTCCCGGA GACGGTCACA GCTTGTCTGT AAGCGGATGC CGGGAGCAGA CAAGCCCGTC 3960
   AGGGCGCGTC AGCGGGTGTT GGCGGGTGTC GGGGCGCAGC CATGACCCAG TCACGTAGCG 4020
   ATAGCGGAGT GTATACTGGC TTAACTATGC GGCATCAGAG CAGATTGTAC TGAGAGTGCA 4080
   CCATATGCGG TGTGAAATAC CGCACAGATG CGTAAGGAGA AAATACCGCA TCAGGCGCTC 4140
   TTCCGCTTCC TCGCTCACTG ACTCGCTGCG CTCGGTCGTT CGGCTGCGGC GAGCGGTATC 4200
   AGCTCACTCA AAGGCGGTAA TACGGTTATC CACAGAATCA GGGGATAACG CAGGAAAGAA 4260
   CATGTGAGCA AAAGGCCAGC AAAAGGCCAG GAACCGTAAA AAGGCCGCGT TGCTGGCGTT 4320
   TTTCCATAGG CTCCGCCCCC CTGACGAGCA TCACAAAAAT CGACGCTCAA GTCAGAGGTG 4380
   GCGAAACCCG ACAGGACTAT AAAGATACCA GGCGTTTCCC CCTGGAAGCT CCCTCGTGCG 4440
   CTCTCCTGTT CCGACCCTGC CGCTTACCGG ATACCTGTCC GCCTTTCTCC CTTCGGGAAG 4500
   CGTGGCGCTT TCTCATAGCT CACGCTGTAG GTATCTCAGT TCGGTGTAGG TCGTTCGCTC 4560
   CAAGCTGGGC TGTGTGCACG AACCCCCCGT TCAGCCCGAC CGCTGCGCCT TATCCGGTAA 4620
   CTATCGTCTT GAGTCCAACC CGGTAAGACA CGACTTATCG CCACTGGCAG CAGCCACTGG 4680
   TAACAGGATT AGCAGAGCGA GGTATGTAGG CGGTGCTACA GAGTTCTTGA AGTGGTGGCC 4740
   TAACTACGGC TACACTAGAA GGACAGTATT TGGTATCTGC GCTCTGCTGA AGCCAGTTAC 4800
   CTTCGGAAAA AGAGTTGGTA GCTCTTGATC CGGCAAACAA ACCACCGCTG GTAGCGGTGG 4860
   TTTTTTTGTT TGCAAGCAGC AGATTACGCG CAGAAAAAAA GGATCTCAAG AAGATCCTTT 4920
   GATCTTTTCT ACGGGGTCTG ACGCTCAGTG GAACGAAAAC TCACGTTAAG GGATTTTGGT 4980
   CATGAGATTA TCAAAAAGGA TCTTCACCTA GATCCTTTTA AATTAAAAAT GAAGTTTTAA 5040
   ATCAATCTAA AGTATATATG AGTAAACTTG GTCTGACAGT TACCAATGCT TAATCAGTGA 5100
   GGCACCTATC TCAGCGATCT GTCTATTTCG TTCATCCATA GTTGCCTGAC TCCCCGTCGT 5160
   GTAGATAACT ACGATACGGG AGGGCTTACC ATCTGGCCCC AGTGCTGCAA TGATACCGCG 5220
   AGACCCACGC TCACCGGCTC CAGATTTATC AGCAATAAAC CAGCCAGCCG GAAGGGCCGA 5280
   GCGCAGAAGT GGTCCTGCAA CTTTATCCGC CTCCATCCAG TCTATTAATT GTTGCCGGGA 5340
   AGCTAGAGTA AGTAGTTCGC CAGTTAATAG TTTGCGCAAC GTTGTTGCCA TTGCTGCAGG 5400
   CATCGTGGTG TCACGCTCGT CGTTTGGTAT GGCTTCATTC AGCTCCGGTT CCCAACGATC 5460
   AAGGCGAGTT ACATGATCCC CCATGTTGTG CAAAAAAGCG GTTAGCTCCT TCGGTCCTCC 5520
   GATCGTTGTC AGAAGTAAGT TGGCCGCAGT GTTATCACTC ATGGTTATGG CAGCACTGCA 5580
   TAATTCTCTT ACTGTCATGC CATCCGTAAG ATGCTTTTCT GTGACTGGTG AGTACTCAAC 5640
   CAAGTCATTC TGAGAATAGT GTATGCGGCG ACCGAGTTGC TCTTGCCCGG CGTCAACACG 5700
   GGATAATACC GCGCCACATA GCAGAACTTT AAAAGTGCTC ATCATTGGAA AACGTTCTTC 5760
   GGGGCGAAAA CTCTCAAGGA TCTTACCGCT GTTGAGATCC AGTTCGATGT AACCCACTCG 5820
   TGCACCCAAC TGATCTTCAG CATCTTTTAC TTTCACCAGC GTTTCTGGGT GAGCAAAAAC 5880
   AGGAAGGCAA AATGCCGCAA AAAAGGGAAT AAGGGCGACA CGGAAATGTT GAATACTCAT 5940
   ACTCTTCCTT TTTCAATATT ATTGAAGCAT TTATCAGGGT TATTGTCTCA TGAGCGGATA 6000
   CATATTTGAA TGTATTTAGA AAAATAAACA AATAGGGGTT CCGCGCACAT TTCCCCGAAA 6060
   AGTGCCACCT GACGTCTAAG AAACCATTAT TATCATGACA TTAACCTATA AAAATAGGCG 6120
   TATCACGAGG CCCTTTCGTC TTCAA 6145

(2) INFORMATION FOR SEQ ID NO:2:
(i) SEQUENCE CHARACTERISTICS:
   (A) LENGTH: 67 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:2:
   GATCTAAGCT TGCGGCCGCA GATCTCGAGC CATGGATCCT AGGCCTGATC ACGCGTCGAC 60
   TCGCGAT 67

(2) INFORMATION FOR SEQ ID NO:3:
(i) SEQUENCE CHARACTERISTICS:
   (A) LENGTH: 65 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:3:
   CGATCGCGAG TCGACGCGTG ATCAGGCCTA GGATCCATGG CTCGAGATCT GCGGCCGCAA 60
   GCTTA 65

(2) INFORMATION FOR SEQ ID NO:4:
(i) SEQUENCE CHARACTERISTICS:
   (A) LENGTH: 33 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:4:
   AAGCTTGATC ACCACCATGA TTGAACAAGA TGG 33

(2) INFORMATION FOR SEQ ID NO:5:
(i) SEQUENCE CHARACTERISTICS:
   (A) LENGTH: 34 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:5:
   CCGGATCCGT CGACCCCAGA GTCCCGCTCA GAAG 34

(2) INFORMATION FOR SEQ ID NO:6:
(i) SEQUENCE CHARACTERISTICS:
   (A) LENGTH: 35 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:6:
   CCCGGGAAGC TTCCACCATG TGGCTGCAGA GCCTG 35

(2) INFORMATION FOR SEQ ID NO:7:
(i) SEQUENCE CHARACTERISTICS:
   (A) LENGTH: 29 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:7:
   AATGGATCCT ATCACTCCTG GACTGGCTC 29

(2) INFORMATION FOR SEQ ID NO:8:
(i) SEQUENCE CHARACTERISTICS:
   (A) LENGTH: 435 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:8:
   ATGTGGCTGC AGAGCCTGCT GCTCTTGGGC ACTGTGGCCT GCAGCATCTC TGCACCCGCC     60
   CGCTCGCCCA GCCCCAGCAC GCAGCCCTGG GAGCATGTGA ATGCCATCCA GGAGGCCCGG    120
   CGTCTCCTGA ACCTGAGTAG AGACACTGCT GCTGAGATGA ATGAAACAGT AGAAGTCATC    180
   TCAGAAATGT TTGACCTCCA GGAGCCGACC TGCCTACAGA CCCGCCTGGA GCTGTACAAG    240
   CAGGGCCTGC GGGGCAGCCT CACCAAGCTC AAGGGCCCCT TGACCATGAT GGCCAGCCAC    300
   TACAAGCAGC ACTGCCCTCC AACCCCGGAA ACTTCCTGTG CAACCCAGAT TATCACCTTT    360
   GAAAGTTTCA AAGAGAACCT GAAGGACTTT CTGCTTGTCA TCCCCTTTGA CTGCTGGGAG    420
   CCAGTCCAGG AGTGA                                                     435

(2) INFORMATION FOR SEQ ID NO:9:
(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:9:
   TGTGGATCCA CCATGGGACT GAGTAACATT 30

(2) INFORMATION FOR SEQ ID NO:10:
(i) SEQUENCE CHARACTERISTICS:
   (A) LENGTH: 35 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:10 :
   TTTGGATCCT TAAAAACATG TATCACTTTT GTCGC 35

(2) INFORMATION FOR SEQ ID NO:11:
(i) SEQUENCE CHARACTERISTICS:
   (A) LENGTH: 972 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:11:
   ATGGGACTGA GTAACATTCT CTTTGTGATG GCCTTCCTGC TCTCTGGTGC TGCTCCTCTG 60
   AAGATTCAAG CTTATTTCAA TGAGACTGCA GACCTGCCAT GCCAATTTGC AAACTCTCAA 120
   AACCAAAGCC TGAGTGAGCT AGTAGTATTT TGGCAGGACC AGGAAAACTT GGTTCTGAAT 180
   GAGGTATACT TAGGCAAAGA GAAATTTGAC AGTGTTCATT CCAAGTATAT GGGCCGCACA 240
   AGTTTTGATT CGGACAGTTG GACCCTGAGA CTTCACAATC TTCAGATCAA GGACAAGGGC 300
   TTGTATCAAT GTATCATCCA TCACAAAAAG CCCACAGGAA TGATTCGCAT CCACCAGATG 360
   AATTCTGAAC TGTCAGTGCT TGCTAACTTC AGTCAACCTG AAATAGTACC AATTTCTAAT 420
   ATAACAGAAA ATGTGTACAT AAATTTGACC TGCTCATCTA TACACGGTTA CCCAGAACCT 480
   AAGAAGATGA GTGTTTTGCT AAGAACCAAG AATTCAACTA TCGAGTATGA TGGTATTATG 540
   CAGAAATCTC AAGATAATGT CACAGAACTG TACGACGTTT CCATCAGCTT GTCTGTTTCA 600
   TTCCCTGATG TTACGAGCAA TATGACCATC TTCTGTATTC TGGAAACTGA CAAGACGCGG 660
   CTTTTATCTT CACCTTTCTC TATAGAGCTT GAGGACCCTC AGCCTCCCCC AGACCACATT 720
   CCTTGGATTA CAGCTGTACT TCCAACAGTT ATTATATGTG TGATGGTTTT CTGTCTAATT 780
   CTATGGAAAT GGAAGAAGAA GAAGCGGCCT CGCAACTCTT ATAAATGTGG AACCAACACA 840
   ATGGAGAGGG AAGAGAGTGA ACAGACCAAG AAAAGAGAAA AAATCCATAT ACCTGAAAGA 900
   TCTGATGAAG CCCAGCGTGT TTTTAAAAGT TCGAAGACAT CTTCATGCGA CAAAAGTGAT 960
   ACATGTTTTT AA 972

(2) INFORMATION FOR SEQ ID NO:12:
(i) SEQUENCE CHARACTERISTICS:
   (A) LENGTH: 29 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:12:
   AAAAGCTTGG ATCCACCATG AGTAAAGGA 29

(2) INFORMATION FOR SEQ ID NO:13:
(i) SEQUENCE CHARACTERISTICS:
   (A) LENGTH: 30 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:13:
   AATCTAGATT ACTATTTGTA TAGTTCATCC 30

(2) INFORMATION FOR SEQ ID NO:14:
(i) SEQUENCE CHARACTERISTICS:
   (A) LENGTH: 1451 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO:14:
   AAGCTTTGGA GCTAAGCCAG CAATGGTAGA GGGAAGATTC TGCACGTCCC TTCCAGGCGG 60
   CCTCCCCGTC ACCACCCCCC CCAACCCGCC CCGACCGGAG CTGAGAGTAA TTCATACAAA 120
   AGGACTCGCC CCTGCCTTGG GGAATCCCAG GGACCGTCGT TAAACTCCCA CTAACGTAGA 180
   ACCCAGAGAT CGCTGCGTTC CCGCCCCCTC ACCCGCCCGC TCTCGTCATC ACTGAGGTGG 240
   AGAAGAGCCA TGCGTGAGGC TCCGGTGCCC GTCAGTGGGC AGAGCGCACA TCGCCCACAG 300
   TCCCCGAGAA GTTGGGGGGA GGGGTCGGCA ATTGAACCGG TGCCTAGAGA AGGTGGCGCG 360
   GGGTAAACTG GGAAAGTGAT GTCGTGTACT GGCTCCGCCT TTTTCCCGAG GGTGGGGGAG 420
   AACCCGTATA TAAGTGCAGT AGTCGCCGTG AACGTTCTTT TTCGCAACGG GTTTGCCGCC 480
   AGAACACAGG TAAGTGCCGT GTGTGGTTCC CGCGGGCCTG GCCTCTTTAC GGGTTATGGC 540
   CCTTGCGTGC CTTGAATTAC TTCCACGCCC CTGGCTGCAG TACGTGATTC TTGATCCCGA 600
   GCTTCGGGTT GGAAGTGGGT GGGAGAGTTC GAGGCCTTGC GCTTAAGGAG CCCCTTCGCC 660
   TCGTGCTTGA GTTGAGGCCT GGCCTGGGCG CTGGGGCCCC CGCGTGCGAA TCTGGTGGCA 720
   CCTTCGCGCC TGTCTCGCTG CTTTCGATAA GTCTCTAGCC ATTTAAAATT TTTGATGACC 780
   TGCTGCGACG CTTTTTTTCT GGCAAGATAG TCTTGTAAAT GCGGGCCAAG ATCTGCACAC 840
   TGGTATTTCG GTTTTTGGGG CCGCGGGCGG CGACGGGGCC CGTGCGTCCC AGCGCACATG 900
   TTCGGCGAGG CGGGGCCTGC GAGCGCGGCC ACCGAGAATC GGACGGGGGT AGTCTCAAGC 960
   TGGCCGGCCT GCTCTGGTGC CTGGCCTCGC GCCGCCGTGT ATCGCCCCGC CCTGGGCGGC 1020
   AAGGCTGGCC CGGTCGGCAC CAGTTGCGTG AGCGGAAAGA TGGCCGCTTC CCGGCCCTGC 1080
   TGCAGGGAGC TCAAAATGGA GGACGCGGCG CTCGGGAGAG CGGGCGGGTG AGTCACCCAC 1140
   ACAAAGGAAA AGGGCCTTTC CGTCCTCAGC CGTCGCTTCA TGTGACTCCA CGGAGTACCG 1200
   GGCGCCGTCC AGGCACCTCG ATTAGTTCTC GAGCTTTTGG AGTACGTCGT CTTTAGGTTG 1260
   GGGGGAGGGG TTTTATGCGA TGGAGTTTCC CCACACTGAG TGGGTGGAGA CTGAAGTTAG 1320
   GCCAGCTTGG CACTTGATGT AATTCTCCTT GGAATTTGCC CTTTTTGAGT TTGGATCTTG 1380
   GTTCATTCTC AAGCCTCAGA CAGTGGTTCA AAGTTTTTTT CTTCCATTTC AGGTGTCGTG 1440
   AAAACTCTAG A 1451

(2) INFORMATION FOR SEQ ID NO:15:
(i) SEQUENCE CHARACTERISTICS:
   (A) LENGTH: 24 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:15:
   AAGCTTTGGA GCTAAGCCAG CAAT 24

(2) INFORMATION FOR SEQ ID NO:16:
(i) SEQUENCE CHARACTERISTICS:
   (A) LENGTH: 23 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:16:
   TCTAGAGTTT TCACGACACC TGA 23

(2) INFORMATION FOR SEQ ID NO:17:
(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:17:
   TCTAGAGCGG CCGCGGAGGC CGAATTCG 28

(2) INFORMATION FOR SEQ ID NO:18:
(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:18:
   GATCCGAATT CGGCCTCCGC GGCCGCTCTA GATGCA 36

(2) INFORMATION FOR SEQ ID NO:19:
(i) SEQUENCE CHARACTERISTICS:
   (A) LENGTH: 40 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:19:
   GAAGATCTGC GGCCGCCACC ATGTGGCCCC CTGGGTCAGC 40

(2) INFORMATION FOR SEQ ID NO:20:
(i) SEQUENCE CHARACTERISTICS:
   (A) LENGTH: 29 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:20:
   CCTCTCGAGT TAGGAAGCAT TCAGATAGC 29

(2) INFORMATION FOR SEQ ID NO:21:
(i) SEQUENCE CHARACTERISTICS:
   (A) LENGTH: 762 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:21:
   ATGTGGCCCC CTGGGTCAGC CTCCCAGCCA CCGCCCTCAC CTGCCGCGGC CACAGGTCTG 60

(2) INFORMATION FOR SEQ ID NO:22:
(i) SEQUENCE CHARACTERISTICS:
   (A) LENGTH: 34 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:22:
   AAAGAGCTCC ACCATGTGTC ACCAGCAGTT GGTC 34

(2) INFORMATION FOR SEQ ID NO:23:
(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:23:
   AAGGATCCTA ACTGCAGGGC ACAGATGC 28

(2) INFORMATION FOR SEQ ID NO:24:
(i) SEQUENCE CHARACTERISTICS:
   (A) LENGTH: 987 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:24:
   ATGTGTCACC AGCAGTTGGT CATCTCTTGG TTTTCCCTGG TTTTTCTGGC ATCTCCCCTC     60
   GTGGCCATAT GGGAACTGAA GAAAGATGTT TATGTCGTAG AATTGGATTG GTATCCGGAT    120
   GCCCCTGGAG AAATGGTGGT CCTCACCTGT GACACCCCTG AAGAAGATGG TATCACCTGG    180
   ACCTTGGACC AGAGCAGTGA GGTCTTAGGC TCTGGCAAAA CCCTGACCAT CCAAGTCAAA    240
   GAGTTTGGAG ATGCTGGCCA GTACACCTGT CACAAAGGAG GCGAGGTTCT AAGCCATTCG    300
   CTCCTGCTGC TTCACAAAAA GGAAGATGGA ATTTGGTCCA CTGATATTTT AAAGGACCAG    360
   AAAGAACCCA AAAATAAGAC CTTTCTAAGA TGCGAGGCCA AGAATTATTC TGGACGTTTC    420
   ACCTGCTGGT GGCTGACGAC AATCAGTACT GATTTGACAT TCAGTGTCAA AAGCAGCAGA    480
   GGCTCTTCTG ACCCCCAAGG GGTGACGTGC GGAGCTGCTA CACTCTCTGC AGAGAGAGTC    540
   AGAGGGGACA ACAAGGAGTA TGAGTACTCA GTGGAGTGCC AGGAGGACAG TGCCTGCCCA    600
   GCTGCTGAGG AGAGTCTGCC CATTGAGGTC ATGGTGGATG CCGTTCACAA GCTCAAGTAT    660
   GAAAACTACA CCAGCAGCTT CTTCATCAGG GACATCATCA AACCTGACCC ACCCAACAAC    720
   TTGCAGCTGA AGCCATTAAA GAATTCTCGG CAGGTGGAGG TCAGCTGGGA GTACCCTGAC    780
   ACCTGGAGTA CTCCACATTC CTACTTCTCC CTGACATTCT GCGTTCAGGT CCAGGGCAAG    840
   AGCAAGAGAG AAAAGAAAGA TAGAGTCTTC ACCGACAAGA CCTCAGCCAC GGTCATCTGC    900
   CGCAAAAATG CCAGCATTAG CGTGCGGGCC CAGGACCGCT ACTATAGCTC ATCTTGGAGC    960
   GAATGGGCAT CTGTGCCCTG CAGTTAG                                        987

(2) INFORMATION FOR SEQ ID NO:25:
(i) SEQUENCE CHARACTERISTICS:
   (A) LENGTH: 2097 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:25:
   ATGAGGCTCG CCGTGGGAGC CCTGCTGGTC TGCGCCGTCC TGGGGCTGTG TCTGGCTGTC 60
   CCTGATAAAA CTGTGAGATG GTGTGCAGTG TCGGAGCATG AGGCCACTAA GTGCCAGAGT 120
   TTCCGCGACC ATATGAAAAG CGTCATTCCA TCCGATGGTC CCAGTGTTGC TTGTGTGAAG 180
   AAAGCCTCCT ACCTTGATTG CATCAGGGCC ATTGCGGCAA ACGAAGCGGA TGCTGTGACA 240
   CTGGATGCAG GTTTGGTGTA TGATGCTTAC TTGGCTCCCA ATAACCTGAA GCCTGTGGTG 300
   GCAGAGTTCT ATGGGTCAAA AGAGGATCCA CAGACTTTCT ATTATGCTGT TGCTGTGGTG 360
   AAGAAGGATA GTGGCTTCCA GATGAACCAG CTTCGAGGCA AGAAGTCCTG CCACACGGGT 420
   CTAGGCAGGT CCGCTGGGTG GAACATCCCC ATAGGCTTAC TTTACTGTGA CTTACCTGAG 480
   CCACGTAAAC CTCTTGAGAA AGCAGTGGCC AATTTCTTCT CGGGCAGCTG TGCCCCTTGT 540
   GCGGATGGGA CGGACTTCCC CCAGCTGTGT CAACTGTGTC CAGGGTGTGG CTGCTCCACC 600
   CTTAACCAAT ACTTCGGCTA CTCGGGAGCC TTCAAGTGTC TGAAGGATGG TGCTGGGGAT 660
   GTGGCCTTTG TCAAGCACTC GACTATATTT GAGAACTTGG CAAACAAGGC TGACAGGGAC 720
   CAGTATGAGC TGCTTTGCCT AGACAACACC CGGAAGCCGG TAGATGAATA CAAGGACTGC 780
   CACTTGGCCC AGGTCCCTTC TCATACCGTC GTGGCCCGAA GTATGGGCGG CAAGGAGGAC 840
   TTGATCTGGG AGCTTCTCAA CCAGGCCCAG GAACATTTTG GCAAAGACAA ATCAAAAGAA 900
   TTCCAACTAT TCAGCTCTCC TCATGGGAAG GACCTGCTGT TTAAGGACTC TGCCCACGGG 960
   TTTTTAAAAG TCCCCCCAAG GATGGATGCC AAGATGTACC TGGGCTATGA GTATGTCACT 1020
   GCCATCCGGA ATCTACGGGA AGGCACATGC CCAGAAGCCC CAACAGATGA ATGCAAGCCT 1080
   GTGAAGTGGT GTGCGCTGAG CCACCACGAG AGGCTCAAGT GTGATGAGTG GAGTGTTAAC 1140
   AGTGTAGGGA AAATAGAGTG TGTATCAGCA GAGACCACCG AAGACTGCAT CGCCAAGATC 1200
   ATGAATGGAG AAGCTGATGC CATGAGCTTG GATGGAGGGT TTGTCTACAT AGCGGGCAAG 1260
   TGTGGTCTGG TGCCTGTCTT GGCAGAAAAC TACAATAAGA GCGATAATTG TGAGGATACA 1320
   CCAGAGGCAG GGTATTTTGC TGTAGCAGTG GTGAAGAAAT CAGCTTCTGA CCTCACCTGG 1380
   GACAATCTGA AAGGCAAGAA GTCCTGCCAT ACGGCAGTTG GCAGAACCGC TGGCTGGAAC 1440
   ATCCCCATGG GCCTGCTCTA CAATAAGATC AACCACTGCA GATTTGATGA ATTTTTCAGT 1500
   GAAGGTTGTG CCCCTGGGTC TAAGAAAGAC TCCAGTCTCT GTAAGCTGTG TATGGGCTCA 1560
   GGCCTAAACC TGTGTGAACC CAACAACAAA GAGGGATACT ACGGCTACAC AGGCGCTTTC 1620
   AGGTGTCTGG TTGAGAAGGG AGATGTGGCC TTTGTGAAAC ACCAGACTGT CCCACAGAAC     1680
   ACTGGGGGAA AAAACCCTGA TCCATGGGCT AAGAATCTGA ATGAAAAAGA CTATGAGTTG     1740
   CTGTGCCTTG ATGGTACCAG GAAACCTGTG GAGGAGTATG CGAACTGCCA CCTGGCCAGA     1800
   GCCCCGAATC ACGCTGTGGT CACACGGAAA GATAAGGAAG CTTGCGTCCA CAAGATATTA     1860
   CGTCAACAGC AGCACCTATT TGGAAGCAAC GTAACTGACT GCTCGGGCAA CTTTTGTTTG     1920
   TTCCGGTCGG AAACCAAGGA CCTTCTGTTC AGAGATGACA CAGTATGTTT GGCCAAACTT     1980
   CATGACAGAA ACACATATGA AAAATACTTA GGAGAAGAAT ATGTCAAGGC TGTTGGTAAC     2040
   CTGAGAAAAT GCTCCACCTC ATCACTCCTG GAAGCCTGCA CTTTCCGTAG ACCTTAA       2097

(2) INFORMATION FOR SEQ ID NO:26:
(i) SEQUENCE CHARACTERISTICS:
   (A) LENGTH: 140 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:26:
   AGGCGTTACT CGACAGAGGA GAGCAAGAAA TGGAGCCAGT AGATCCTAGA CTAGAGCCCT     60
   GGAAGCATCC AGGAAGTCAG CCTAAAACTG CTTGTACCAA TTGCTATTGT AAAAAGTGTT     120
   GCTTTCATTG CCAAGTTTGT                                                 140

(2) INFORMATION FOR SEQ ID NO:27:
(i) SEQUENCE CHARACTERISTICS:
   (A) LENGTH: 70 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:27:
   TATAAGATGG GTGGCAAGTG GTCAAAAAGT AGTGTGATTG GATGGCCTGC TGTAAGGGAA 60
   AGAATGAGAC 70

(2) INFORMATION FOR SEQ ID NO:28:
(i) SEQUENCE CHARACTERISTICS:
   (A) LENGTH: 70 base pairs
   (B) TYPE: nucleic acid
   (C) STRANDEDNESS: double
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:28:
   CTCAGGTACC TTTAAGACCA ATGACTTACA AGGCAGCTGT AGATCTTAGC CACTTTTTAA 60
   AAGAAAAGGG 70

(2) INFORMATION FOR SEQ ID NO:29:
(i) SEQUENCE CHARACTERISTICS:
   (A) LENGTH: 55 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:29:
   AGCTTGGTCG CCCGGTGGAT CAAGACCGGT AGCCGTCATA AAGGTGATTT CGTCG 55

(2) INFORMATION FOR SEQ ID NO:30:
(i) SEQUENCE CHARACTERISTICS:
   (A) LENGTH: 55 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:30:
   GATCCGACGA AATCACCTTT ATGACGGCTA CCGGTCTTGA TCCACCGGGC GACCA 55

(2) INFORMATION FOR SEQ ID NO:31:
(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:31:
   CGGGATCCAC CATGGGTGCG AGAGCGTC 28

(2) INFORMATION FOR SEQ ID NO:32:
(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(v) FRAGMENT TYPE: linear
(xi) SEQUENCE DESCRIPTION: SEQ ID NO:32:
   ATCCTATTTG TTCCTGAAGG 20

(2) INFORMATION FOR SEQ ID NO:33:
(i) SEQUENCE CHARACTERISTICS:
   (A) LENGTH: 12494 base pairs
   (B) TYPE: nucleic acid
   (C) STRANDEDNESS: double
   (D) TOPOLOGY: circular
(ii) MOLECULE TYPE: other nucleic acid
   (A) DESCRIPTION: /desc = "DNA"
(xi) SEQUENCE DESCRIPTION: SEQ ID NO:33:

### References

Aldhous, M.C., K.C. Watret, J.Y. Mok, A.G. Bird and K.S. Froebel (1994) "Cytotoxic T lymphocyte activity and CD8 subpopulations of children at risk of HIV infection," Clin. Exp. Immunol. 97:61-67.
Chaffee, S., J.M. Leeds, T.J. Matthews, K.J. Weinhold, M. Skinner, D.P. Bolognesi, and M.S. Hershfield (1988) "Phenotypic variation in the response to the human immunodeficiency virus among derivatives of the CEM T and WIL-2 B cell lines" J. Exp. Med. 168:605-621.
Chang, L.-J., and Y. Cui "A rapid and reliable bioassay for functional human interleukin-12 by ELISPOT" (in preparation).
Chang, L.-J., and C. Zhang (1995) "Infection and replication of Tat-minus human immunodeficiency viruses: genetic analyses of LTR and tat mutants in primary and long-term human lymphoid cells" Virology 211:157-169.
Clerici, M., J.V. Giorgi, C.C. Chou, V.K. Gudeman, J.A. Zack, P. Gupta, H.N. Ho, P.G. Nishanian, J.A. Berzofsky, and G.M. Shearer (1992) "Cell-mediated immune response to human immunodeficiency virus (HIV) type 1 in seronegative homosexual men with recent sexual exposure to HIV-1" J. Inf. Dis. 165:1012-1019.
Czerkinsky, C.C., G. Anderson, H. Ekre, L. Nilsson, L. Klareskog and O. Ouchterlony (1988) "Reverse ELISPOT assay for clonal analysis of cytokine production. I. Enumeration of gamma-interferon-secreting cells" J. Immunol. Methods 110:29-36.
Czerkinsky, C.C., Z. Moldoveanu, J. Mestecky, L. Nilsson and O. Ouchterlony (1988) "A novel two colour ELISPOT assay. I. Simultaneous detection of distinct types of antibody-secreting cells" J. Immunol. Methods 115:31-37*.*
Jassoy, C., T. Harrer, T. Rosenthal, B.A. Navia, J. Worth, R.P. Johnson and B.D. Walker (1993) "Human immunodeficiency virus type 1-specific cytotoxic T lymphocytes release gamma interferon, tumor necrosis factor alpha (TNF-α), and TNF-β when they encounter their target antigens" J. Virology 67:2844-2852.
Rowland-Jones, S., J. Sutton, K. Ariyoshi, T. Dong, F. Gotch, S. McAdam, D. Whitby, S. Sabally, A. Gallimore, T. Corrah, M. Takiguchi, T. Schultz, A. McMichael and H. Whittle (1995) "HIV-specific cytotoxic T-cells in HIV-exposed but uninfected Gambian women" Nature Medicine 1:59-64.
Walker, B.D., S. Chakrabarti, B. Moss, T.J. Paradis, T. Flynn, A.G. Durno, R.S. Blumberg, J.C. Kaplan, M.S. Hirsch and R.T. Schooley (1987) "HIV-specific cytotoxic T lymphocytes in seropositive individuals" Nature 328:345-348.

## Claims

1. A method for evaluating a response of human immune cells to a humans immunodeficiency virus, said method comprising:
(a) providing:
i) a SCID/beige mouse reconstituted with human peripheral blood lymphocytes wherein said peripheral blood lymphocytes are used for reconstitution within 2 hours of isolation,
said mouse the having been injected with an injectable preparation comprising one or more components from said human immunodeficiency virus;
ii) a composition comprising non-attenuated human immunodeficiency virus;
(b) exposing said injected mouse to said composition; and
(c) monitoring said exposed mouse for an immune response of said human immune cells to said non-attenuated human immunodeficiency virus;
wherein said component of said human immunodeficiency virus comprises DNA, said DNA comprising proviral DNA encoding a human immunodeficiency virus genome capable of expressing viral structural genes but incapable of being packaged into viral particles.

2. The method of Claim 1, wherein the injecting referred to in step (a) has been repeated at least once prior to exposing said mouse to said composition.

3. The method of Claim 1, wherein said injectable preparation comprises an attenuated replication-competent human immunodeficiency virus.

4. The method of Claim 3, wherein the genome of said attenuated virus comprises a tat gene comprising a mutation wherein functional Tat protein is not expressed.

5. The method of Claim 4, wherein said genome of said attenuated virus further comprises a nef gene comprising a mutation wherein functional Nef protein is not expressed.

## Patentansprüche

1. Methode zum Beurteilen einer Reaktion humaner Immunzellen auf ein humanes Immundefizienz-Virus, wobei die Methode Folgendes umfasst:
(a) das Bereitstellen
i) einer SCID/beige Maus, die mit humanen peripheren Blutlymphozyten rekonstituiert worden ist, wobei die peripheren Blutlymphozyten zum Rekonstituieren innerhalb von 2 Stunden nach der Isolation verwendet werden, wobei die Maus dann mit einem injizierbaren Präparat injiziert worden ist, das eine oder mehrere Komponenten aus dem humanen Immundefizienz-Virus umfasst;
ii) einer Zusammensetzung umfassend ein nichtattenuiertes humanes Immundefizienz-Virus;
(b) das Aussetzen der injizierten Maus der Zusammensetzung gegenüber; und
(c) das Überwachen der ausgesetzten Maus auf eine Immunantwort der humanen Immunzellen auf das nichtattenuierte humane Immundefizienz-Virus;
wobei die Komponente des humanen Immundefizienz-Virus DNA umfasst, wobei die DNA provirale DNA umfasst, die ein humanes Immundefizienz-Virusgenom kodiert, das dazu fähig ist, Virusstrukturgene zu exprimieren, jedoch unfähig ist, in Virusteilchen gepackt zu werden.

2. Methode nach Anspruch 1, wobei das in Schritt (a) erwähnte Injizieren vor dem Aussetzen der Maus der Zusammensetzung gegenüber mindestens einmal wiederholt worden ist.

3. Methode nach Anspruch 1, wobei das injizierbare Präparat ein attenuiertes, replikationskompetentes humanes Immundefizienz-Virus umfasst.

4. Methode nach Anspruch 3, wobei das Genom des attenuierten Virus ein tat-Gen umfasst, das eine Mutation umfasst, wobei funktionelles Tat-Protein nicht exprimiert wird.

5. Methode nach Anspruch 4, wobei das Genom des attenuierten Virus des Weiteren ein nef-Gen umfasst, das eine Mutation umfasst, wobei funktionelles Nef-Protein nicht exprimiert wird.

## Revendications

1. Méthode d'évaluation de la réponse de cellules immunitaires humaines à un virus de l'immunodéficience humaine, ladite méthode comprenant :
(a) l'utilisation :
i) d'une souris SCID/beige qui a reçu des lymphocytes du sang périphérique humain, où lesdits lymphocytes du sang périphérique sont utilisés pour la reconstitution dans les 2 heures qui suivent l'isolation, ladite souris ayant été injectée avec une préparation injectable comprenant un ou plusieurs composants dudit virus de l'immunodéficience humaine ;
ii) d'une composition comprenant un virus de l'immunodéficience humaine non atténué ;
(b) l'exposition de ladite souris injectée à ladite composition ; et
(c) la surveillance de ladite souris exposée pour détecter une réponse immunitaire desdites cellules immunitaires humaines au dit virus de l'immunodéficience humaine non atténué ;
où ledit composant dudit virus de l'immunodéficience humaine comprend un ADN, ledit ADN comprenant un ADN proviral qui code pour un génome du virus de l'immunodéficience humaine capable d'exprimer les gènes viraux structuraux mais qui ne peut pas être empaqueté dans des particules virales.

2. Méthode de la revendication 1, où l'injection à laquelle il est fait référence à l'étape (a) a été répétée au moins une fois avant l'exposition de ladite souris à ladite composition.

3. Méthode de la revendication 1, où ladite préparation injectable comprend un virus de l'immunodéficience humaine atténué compétent pour la réplication.

4. Méthode de la revendication 3, où le génome dudit virus atténué comprend un gène tat portant une mutation telle qu'une protéine Tat fonctionnelle n'est pas exprimée.

5. Méthode de la revendication 4, où le génome dudit virus atténué comprend également un gène nef portant une mutation telle qu'une protéine Nef fonctionnelle n'est pas exprimée.
